# EUROPEAN PATENT SPECIFICATION

(11) **EP 3 880 304 B1**
(45) Date of publication and mention of the grant of the patent: **16.10.2024**
(21) Application number: 19800907.8
(22) Date of filing: 15.11.2019
(51) Int. Cl.: A61P 17/00, A61P 17/18, A61K 35/74, A61K 38/16, A61K 8/66, A61K 8/99, G01N 33/50

(54) **COMPOSITIONS FOR TREATING OXIDATIVE STRESS-ASSOCIATED SKIN DISEASES AND SKIN AGEING**
ZUSAMMENSETZUNGEN ZUR BEHANDLUNG VON OXIDATIVEN STRESS-ASSOZIIERTEN HAUTERKRANKUNGEN UND HAUTALTERUNG
COMPOSITIONS POUR LE TRAITEMENT DE MALADIES CUTANÉES ASSOCIÉES AU STRESS OXYDATIF ET DU VIEILLISSEMENT DE LA PEAU

(30) Priority: 16.11.2018 EP 18206646
(43) Date of publication of application: 22.09.2021
(73) Proprietor: S-Biomedic NV, 2340 Beerse (BE)
(72) Inventor: LOOD, Rolf, 224 74 Lund (SE)
(74) Representative: De Clercq & Partners
(86) International application number: PCT/EP2019/081546
(87) International publication number: WO 2020/099663

(56) References cited:
- ELSTON MARLEE J ET AL: "Cutibacterium acnes (formerly Proprionibacterium acnes ) and Shoulder Surgery", HAWAI'I JOURNAL OF HEALTH & SOCIAL WELFARE, 1 November 2019 (2019-11-01), United States, pages 3 - 5, XP093019657, Retrieved from the Internet <URL:https://www.ncbi.nlm.nih.gov/pmc/articles/PMC6874694/pdf/hjhsw7811_S2_0003.pdf> [retrieved on 20230201]
- TILDE ANDERSSON ET AL: "Common skin bacteria protect their host from oxidative stress through secreted antioxidant RoxP", SCIENTIFIC REPORTS, vol. 9, no. 1, 5 March 2019 (2019-03-05), XP055659361, DOI: 10.1038/s41598-019-40471-3
- GIZEM ERTÜ RK ET AL: "Highly sensitive detection and quantification of the secreted bacterial benevolence factor RoxP using a capacitive biosensor: A possible early detection system for oxidative skin diseases", 1 March 2018 (2018-03-01), XP055659372, Retrieved from the Internet <URL:https://journals.plos.org/plosone/article/file?id=10.1371/journal.pone.0193754&type=printable> [retrieved on 20200120]
- MARIA ALLHORN ET AL: "A novel enzyme with antioxidant capacity produced by the ubiquitous skin colonizer Propionibacterium acnes", SCIENTIFIC REPORTS, vol. 6, no. 1, 2 November 2016 (2016-11-02), XP055659407, DOI: 10.1038/srep36412

## Description

### TECHNICAL FIELD

Aspects of the invention are broadly in the medical and cosmetic fields and more specifically concern methods, uses and compositions for the prophylactic or therapeutic treatment of oxidative stress-associated skin diseases or for prevention or reduction of skin ageing.

### BACKGROUND

The human skin is constantly exposed to oxidative stress and free radicals, such as to high quantities of reactive oxygen species (ROS), derived among others from ordinary metabolic reactions and continuous exposure to air, solar or artificial UV radiation, environmental pollutants, as well as physical and/or chemical agents (e.g., cosmetics).

Under physiological conditions, an endogenous production of reducing agents such as glutathione S-transferase and glutathione reductase protects the human skin from excessive oxidative stress. Under pathological conditions, however, the production of ROS may become too great and consequently contribute to the pathogenesis of, for example, psoriasis (Kadam et al. 2010 Indian J Clin Biochem 25:388-392) or skin cancer (Sander et al. 2003 Br J Dermatol 148:913-922). Non-melanoma skin cancers, including squamous cell carcinoma and basal cell carcinoma, display not only increased evidence of oxidative stress, but also of impaired endogenous antioxidant function (Sander et al. 2003).

Oxidative damage caused by free radicals such as ROS is also a main cause of physical ageing in general, and of the skin in particular. While skin is equipped with an elaborate antioxidant system that protects it from oxidative damage, the natural antioxidant pool can be compromised with age, or overwhelmed by oxidative stress, e.g., due to excess UV exposure. Free radicals can attack collagen and elastin causing the structure to break down. With age, the accumulated free radical effects begin to slow down the cell function, therefore reducing the body's self-repair capabilities. Free radical attacks eventually lead to wrinkles, sagging skin, and "age spots."

Andersson *et al.* (Scientific Reports, 9(1), 2019) disclose that RoxP positively influences the viability of monocytes and keratinocytes in cell cultures exposed to oxidative stress, and that a congruent concentration decline of RoxP can be observed in skin affected by oxidative disease. Andersson *et al.* was only published after the priority date of the present patent application. Ertürk et al., (Plos One, 13(3), 2018) identify RoxP for maintaining the redox homeostasis on the skin and provide a capacitative biosensor used to detect RoxP on skin *in vivo.*

Allhorn *et al.* (Scientific Reports, 6(1), 2016) indicate that RoxP has antioxidant activity.

There exists an ongoing need to provide additional or improved avenues to prevent or reduce oxidative stress and its harmful effects on the skin in both therapeutic and cosmetic contexts.

The present inventors previously characterised the protein RoxP (radical oxygenase of *Propionibacterium acnes)* abundantly expressed by C. *acnes* isolates, and showed that RoxP exhibits antioxidant activity in certain *in vitro* conditions, and can support C. *acnes* growth in aerobic settings *in vitro* and the colonization of human skin explants *ex vivo* (Allhorn et al. 2016 Sci Rep 6:36412).

### SUMMARY OF THE INVENTION

The present invention is defined in the claims and is at least in part based on the unexpected discovery that RoxP protects human skin from oxidative damage in therapeutically and cosmetically relevant model systems, and that reduced skin quantity of RoxP correlates with oxidative stress-associated skin diseases in humans. These observations for the first time identify RoxP or *C*. *acnes* bacteria secreting RoxP as dependable avenues for therapeutic or cosmetic interventions aimed at addressing pathological or physiological oxidative stress processes in the skin.

Accordingly, an aspect provides a cosmetic use of a composition comprising a live *Cutibacterium acnes* strain secreting RoxP (radical oxygenase of *Propionibacterium acnes)* for preventing or reducing skin ageing in a subject.

A related aspect provides a cosmetic method for preventing or reducing skin ageing in a subject, the method comprising administering to the skin of the subject a cosmetically effective amount of a composition comprising a live *C*. *acnes* strain secreting RoxP.

Another aspect provides a composition comprising a live *C*. *acnes* strain secreting RoxP for use in a method of preventing or treating an oxidative stress-associated skin disease, wherein the oxidative stress-associated skin disease is selected from the group comprising actinic keratosis (AK), and basal cell carcinoma (BCC).

A further aspect provides a cosmetic use of a composition comprising RoxP for preventing or reducing skin ageing in a subject.

A related aspect provides a cosmetic method for preventing or reducing skin ageing in a subject, the method comprising administering to the skin of the subject a cosmetically effective amount of a composition comprising RoxP.

Another aspect provides a composition comprising RoxP for use in a method of preventing or treating an oxidative stress-associated skin disease; wherein the oxidative stress-associated disease is selected from the group comprising actinic keratosis (AK) and basal cell carcinoma (BCC).

These and further aspects and preferred embodiments of the invention are described in the following sections and in the appended claims.

### BRIEF DESCRIPTION OF THE FIGURES

The teaching of the application is illustrated by the following Figures which are to be considered as illustrative only and do not in any way limit the scope of the claims.

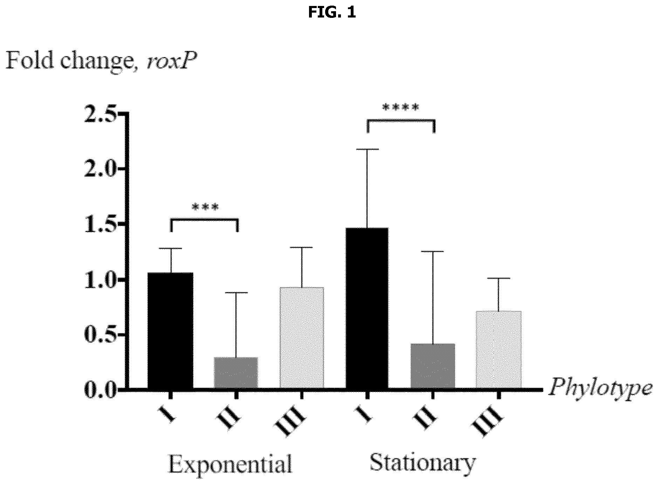

**Figure 1****:** *roxP* expression in different *C. acnes* phylotypes. RNA was extracted from exponential and/or stationary phase *C. acnes* cultures, representative of type I (n = 6), type II (n = 5) and type III (n = 2) strains. qPCR quantifications of *roxP* mRNA were run in biological and technological duplicates, normalized against *gapdh* transcripts and evaluated using double delta Cq analysis. Grouped with their respective phylotypes, statistical significance was subsequently determined through a student's t-test. *** p<0.001, **** p<0.0001.

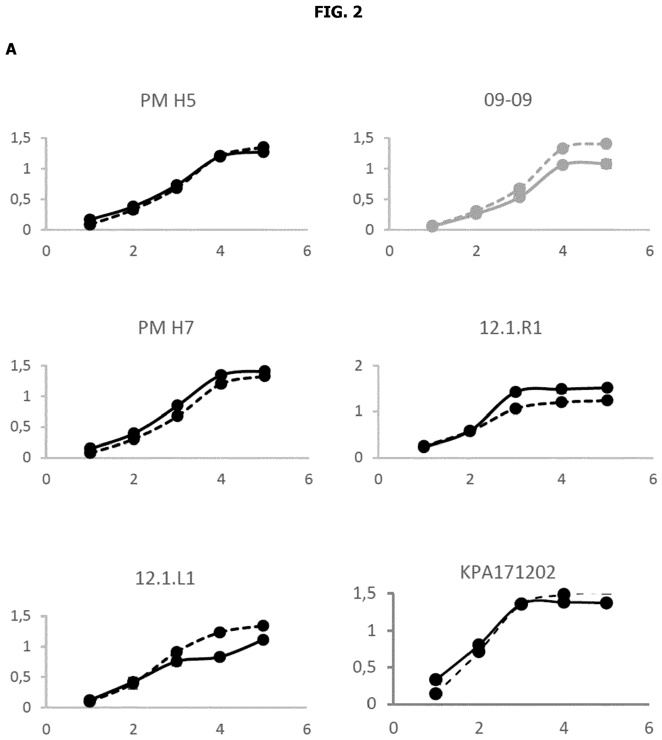

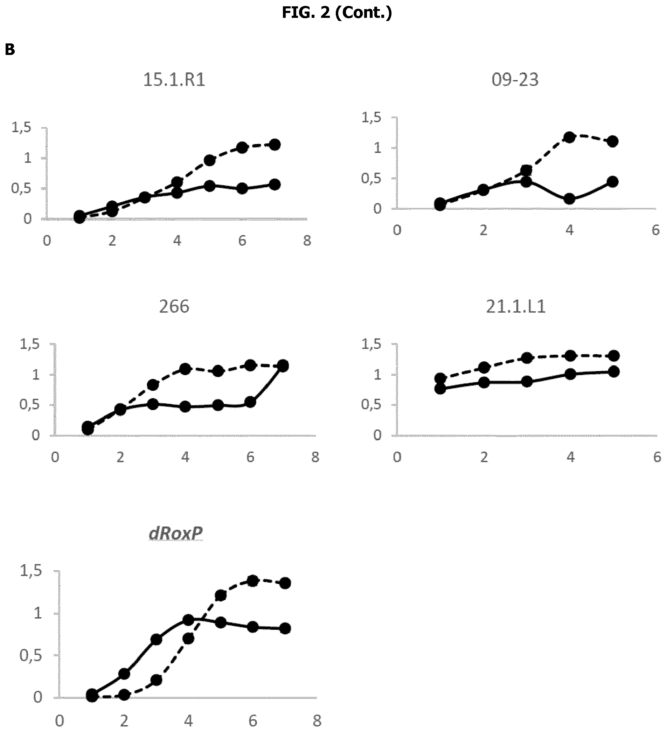

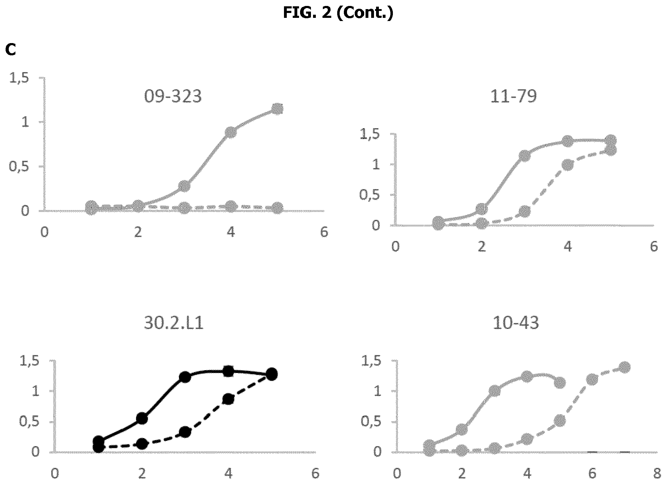

**Figure 2****:** Fifteen different strains of *C*. *acnes* were cultured in TSB under anaerobic (solid line) or aerobic (dashed line) conditions for 5-7 days. Certain strains **(A)** showed no significant difference between oxic and anoxic growth, **(B)** were favouring aerobic growth mainly in the late exponential phase, or **(C)** had an increased lag time in aerobic conditions. Lines coloured in black represent strains expressing the most common RoxP homologue, and grey lines represent strains expressing the less common protein variant. The Y-axis describes OD₆₀₀ and the X-axis represents time (days).

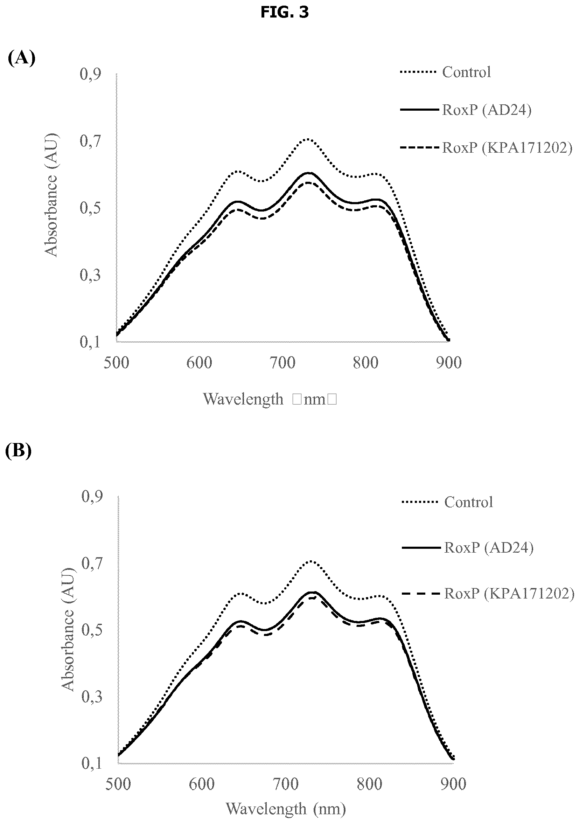

**Figure 3****:** Activity of RoxP.

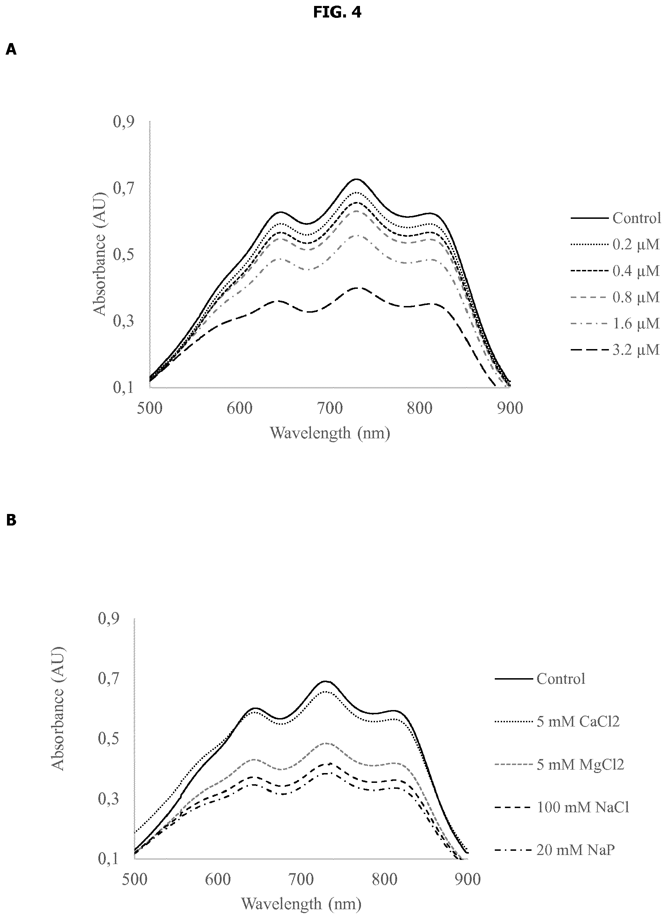

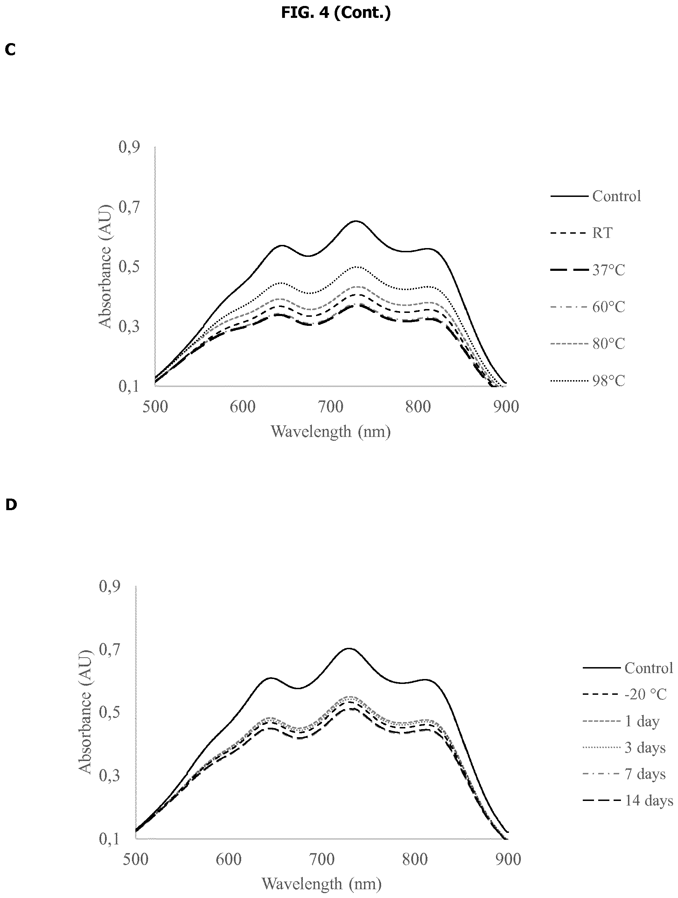

**Figure 4****:** RoxP antioxidant activity under adverse conditions. The ability of RoxP to reduce ABTS radical cations, denoted by a decrease in absorbance at 734 nm, was tested in multiple settings. Different concentrations of recombinant RoxP (rRoxP) (0.2-3.2 µM) in sodium phosphate buffer (20 mM, pH 7.4) (A), rRoxP (2.7 µM) incubated with salt solutions **(B),** preheated rRoxP (2.7 µM) incubated for 20 min in temperatures ranging from RT to 98°C and allowed to cool down to ambient temperature prior to activity measurements **(C),** or rRoxP (2 µM) stored at room temperature for up to 2 weeks **(D)** were added to a solution of pre-formed ABTS⁺ radicals and the absorbance recorded after 30 sec.

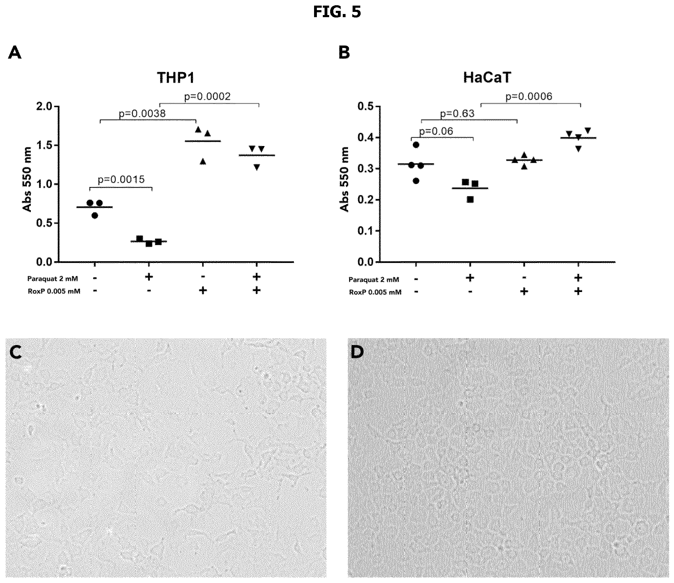

**Figure 5****:** Human monocyte **(A)** or keratinocyte **(B)** cell lines were exposed to oxidative stress through addition of paraquat, while adding RoxP momentarily afterwards. Cells were incubated for 24 h (keratinocytes) or 48 h (monocytes) before cell viability was analysed through MTT assays. Representative microscopy visualizations of monocytes after paraquat **(C)** or paraquat + RoxP **(D)** are demonstrated. Statistics were calculated using a student's t-test.

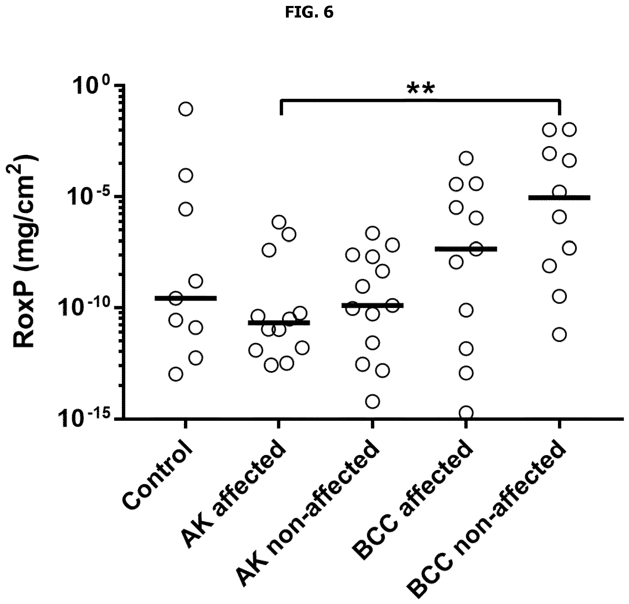

**Figure 6****:** Individuals with either healthy skin (control, n=18), actinic keratosis (AK, n=18) or basal cell carcinoma (BCC, n=18) were swabbed at diseased (affected) and healthy (non-affected) regions. Absolute quantity of RoxP was determined by RoxP-MIP capacitive biosensor measurements. All individual data points were included in the analysis.

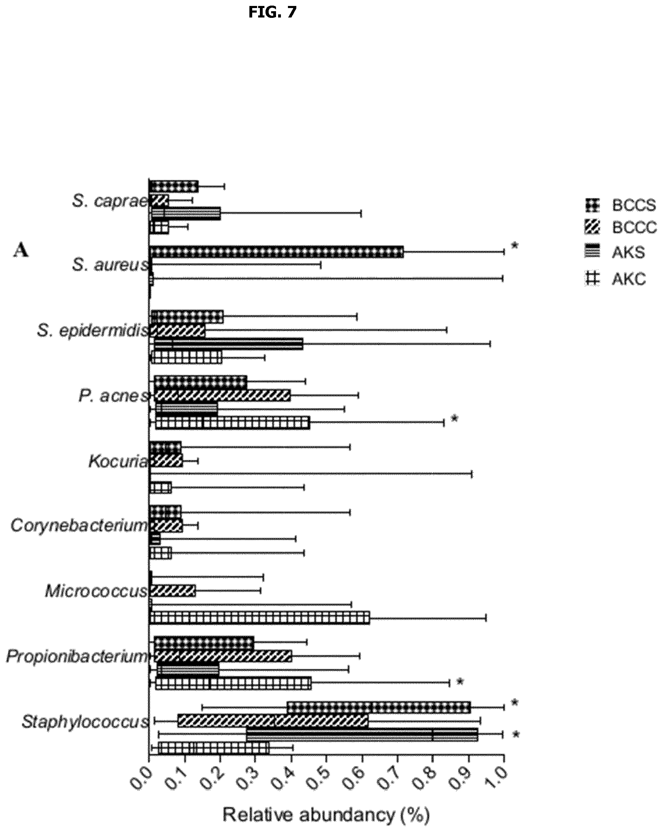

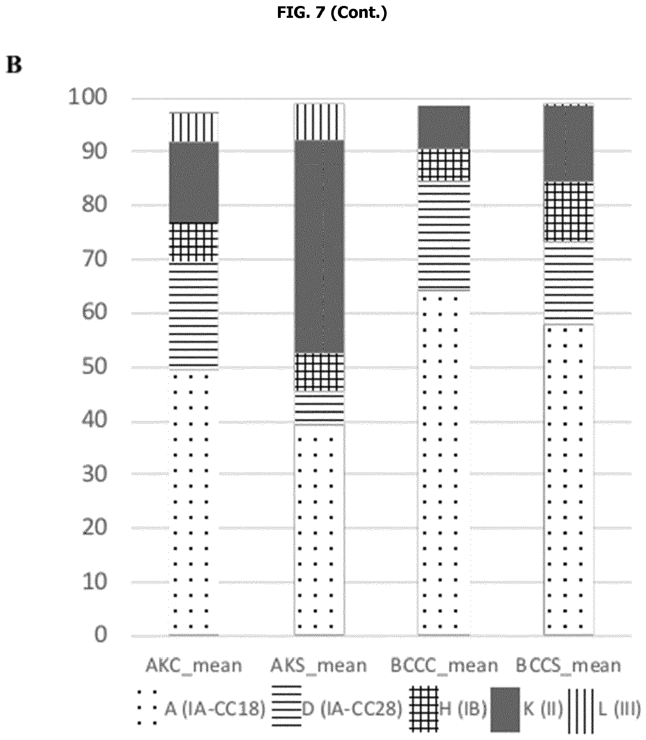

**Figure 7****:** Box plot of the relative abundances of the five most abundant genera and four most abundant species in the four groups **(A).** Whiskers shows the 5-95% percentile and median values are shown as vertical lines. Differentially abundant taxa based of LefSe analysis are marked with an *. **(B)** Determination of relative abundancies of *C*. *acnes* phylotypes in AK and BCC. *C*. *acnes* type II abundances increase in AK-affected skin sites compared to controls (increase from 15% to 39%), whereas type IA strains decline (from 70% to 45%).

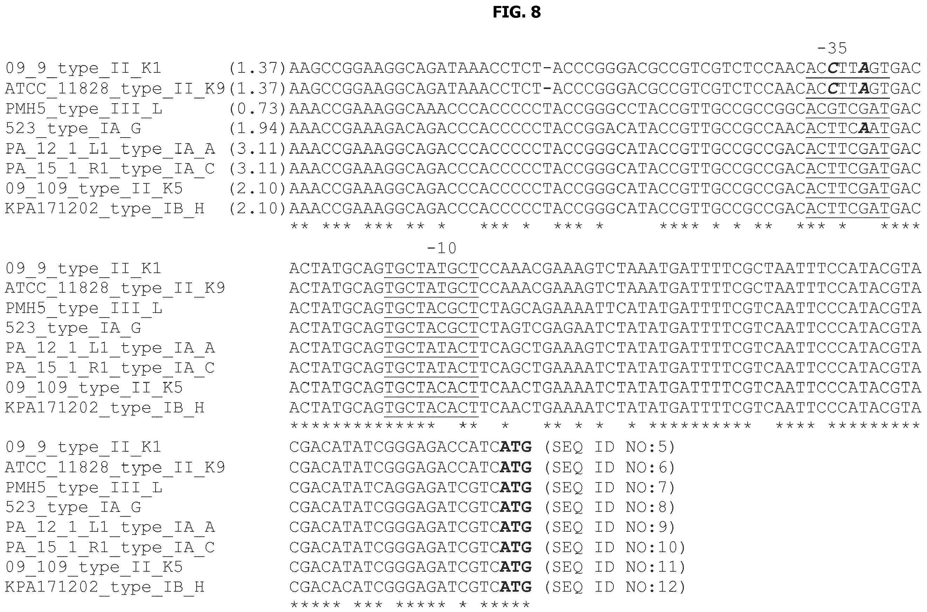

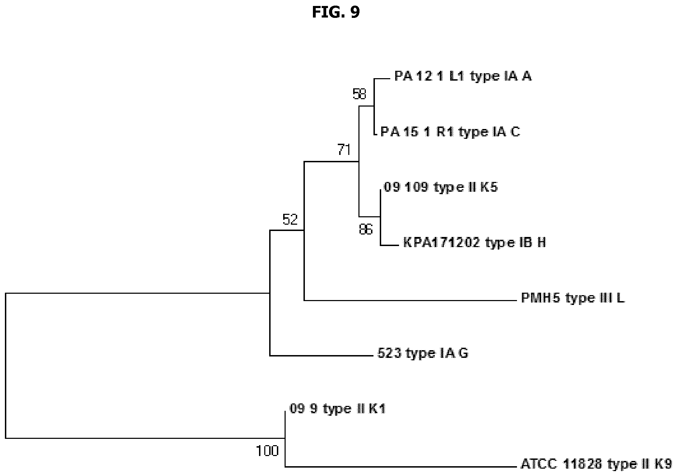

**Figure 8****:** Alignment of the *roxP* upstream region of eight *C*. *acnes* strains. The strains represent the eight dominant *roxP* upstream sequences, covering the entire *C*. *acnes* population (see Figure 9). The upstream sequences contain the putative *roxP* promoter, underlined. This promoter was found using the tool BPROM (Solovyev and Salamov 2011, Automatic Annotation of Microbial Genomes and Metagenomic Sequences. In Metagenomics and its Applications in Agriculture, Biomedicine and Environmental Studies (Ed. R.W. Li), Nova Science Publishers, p. 61-78) (the confidence values (LDF (Linear discriminant function) scores are given in brackets after the strain name). In addition, the C. *acnes* consensus promoter sequence (-10 region: T.A.n.n.n.T and -35 region: G/A.n.T/G.T/G.n.G), identified by Lin et al. (2013 BMC Genomics 14:620) was compared: all mismatched bases are bold italics. Type IA strains with the SLST type A and C carry a *roxP* upstream sequence with a perfect match to the consensus promoter. Type II strains represented by strains 09-9 and ATCC 11828 carry mismatches in the -35 region.
**Figure 9****:** Analysis of the *roxP* upstream region containing the putative promoter. 155 sequenced *C*. *acnes* genomes were screened for the *roxP* upstream region (200 nt). All *C*. *acnes* genomes carried this region. Eight dominant sequences were found; representative strains carrying each an individual *roxP* upstream sequence are shown. The respective upstream regions were phylogenetically compared. Most sequenced genomes carried the version represented by strain PA_12.1.L1: 81 genomes; the other strains represented: PA_15.1.R1, 24 genomes; 09-109, 8 genomes; KPA171202, 11 genomes; PMH5, 4 genomes; 523, 3 genomes; 09-9, 2 genomes; ATCC_11828, 20 genomes. The respective type names IA, IB, II, III as well as the SLST types A, C, G, H, K, L of the strains are given in the figure. Type II strains can be found in different clusters.

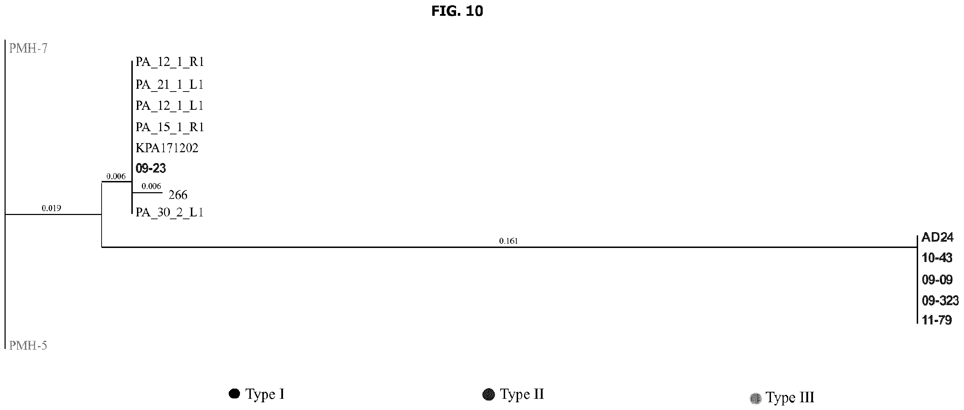

**Figure 10****. Phylogenetic tree of RoxP sequences from *C*. *acnes.*** Amino acid sequences of RoxP from the investigated *C*. *acnes* strains were aligned using a ClustalW algorithm, which separated RoxP into three main types. The first type contained RoxP from strains PMH-7 and PMH-5 (both belonging to phylotype III), the second type contained RoxP from strains PA_12_1_R1, PA_21_1_L1, PA_12_1_L1, PA_15_1_R1, KPA171202, 09-23, and PA_30_2_L1 (all but one belonging to phylotype I; strain 09-23 belongs to phylotype II), the third type contained RoxP from strains AD24, 10-43, 09-09, 09-323, and 11-79 (all belonging to phylotype II).

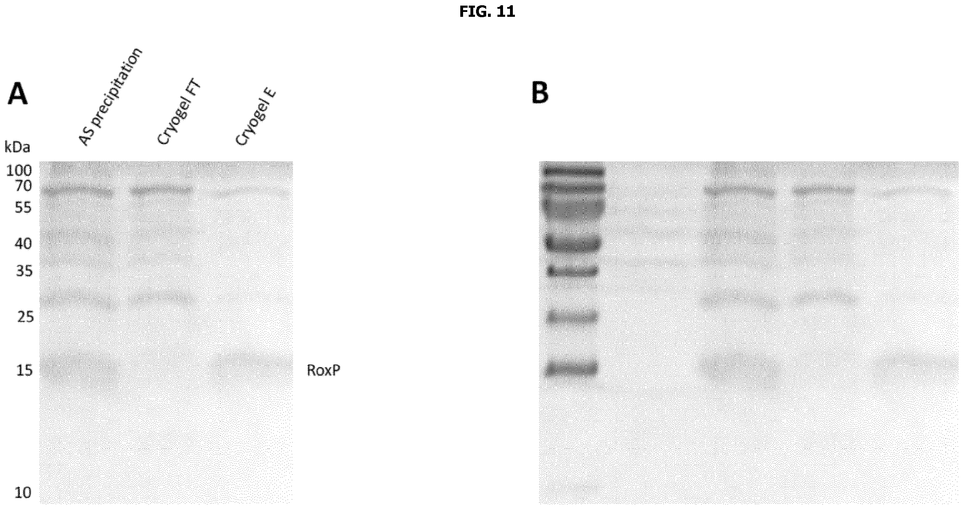

**Figure 11****.** Affinity purification of RoxP from bacterial supernatant to high purity using RoxP-based molecular imprint cryogel matrix. **(A)** Ammonium sulphate precipitated (80%) and re-buffered (100 mM sodium phosphate pH 7.0) supernatant from *C*. *acnes* type II strain AD24 were run over a RoxP-based molecular imprint cryogel matrix. The flow-through was collected and bound proteins eluted with 500 mM imidazole. PageRuler^{™} Prestained Protein Ladder (Thermo Scientific) was used as a molecular weight determinant. **(B)** Original gel.

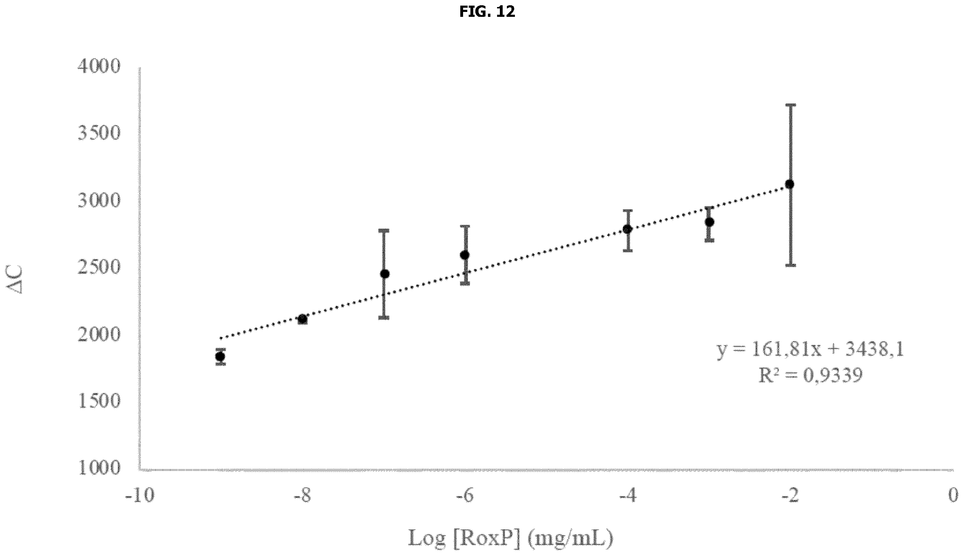

**Figure 12****:** Calibration curve for determination of RoxP concentrations in skin swab samples using a capacitive biosensor. The contents of aerated skin swabs, not applied to skin or other solid surfaces, was diluted in a 10 mM sodium phosphate buffer pH 7.0 supplemented with ranging concentrations of RoxP. Samples were injected into the system and the resulting change in capacitance plotted against logarithmic protein concentrations.

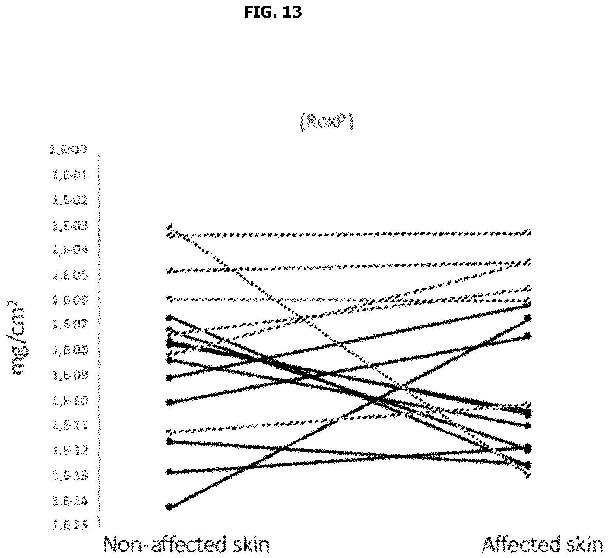

**Figure 13****: Prevalence of RoxP in patients with BCC.** Individuals with either basal cell carcinoma (grey) or actinic keratosis (black) were swabbed at diseased (affected) and healthy (non-affected) regions of the skin. Absolute quantity of RoxP was determined by RoxP-MIP capacitive biosensor measurements. The analysis includes only those individuals from whom two matching data points could be successfully computed.

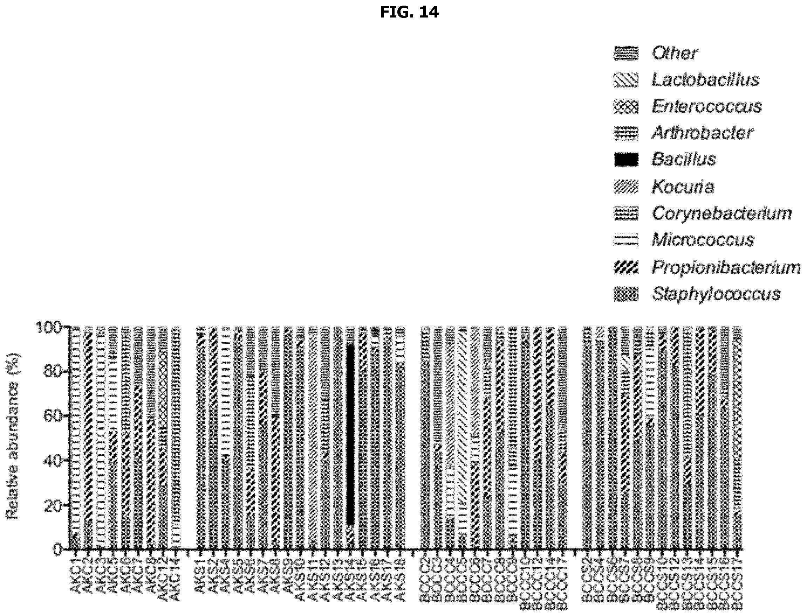

**Figure 14****:** Relative abundance of the most abundant genera (all other genera clustered in the "Other" group) in the four groups: AKC (actinic keratosis: healthy skin site), AKS (actinic keratosis: affected skin site), BCCC (basal cell carcinoma: healthy skin site), and BCCS (basal cell carcinoma: affected skin site).

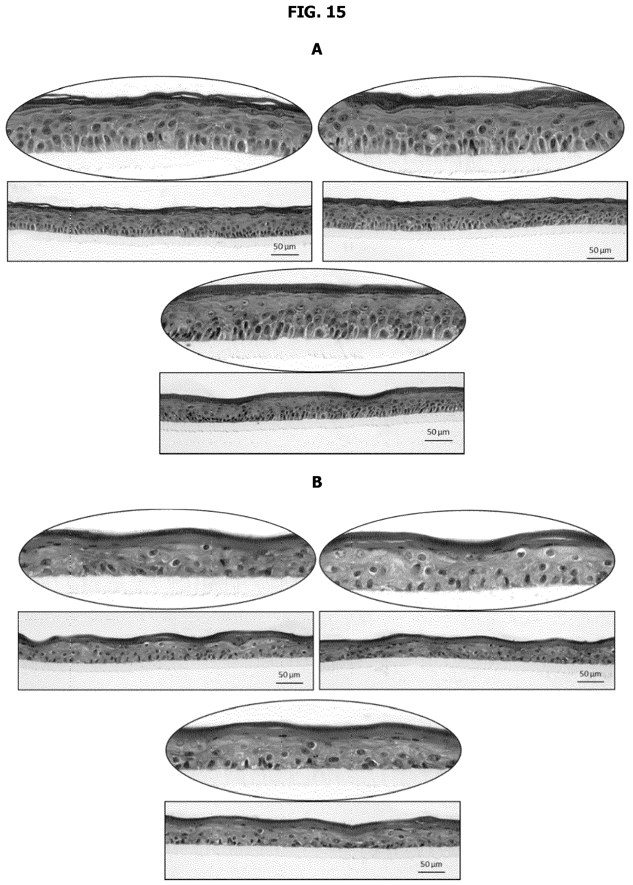

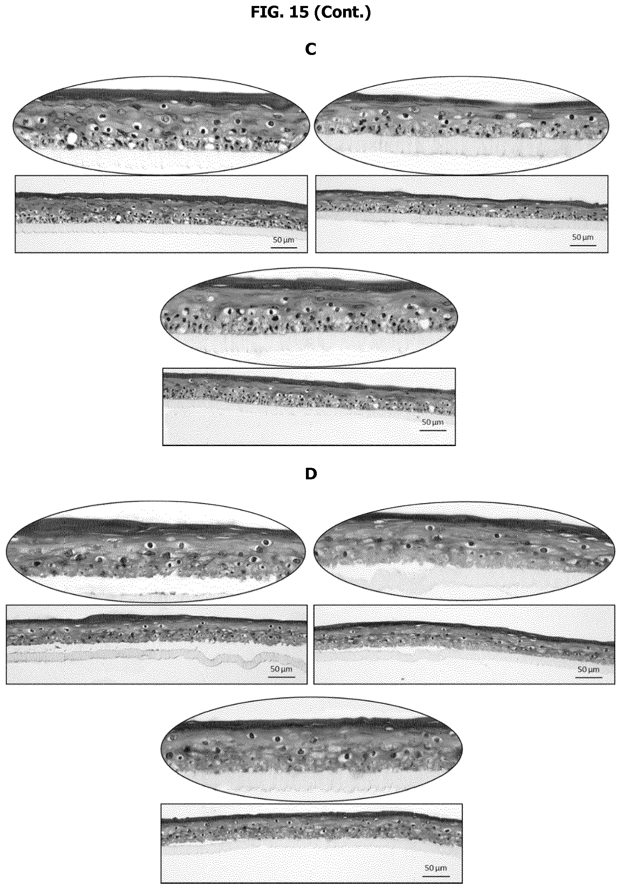

**Figure 15****:** Morphology evaluation of reconstituted human epidermis (RHEs) exposed to UV stress. **(A)** Negative control 1 - unstressed RHEs without RoxP-enriched *C. acnes* supernatant, **(B)** Negative control 2 - unstressed RHEs with *C*. *acnes* supernatant at 10% v/v, **(C)** Positive control 1 - RHEs stressed with UV, **(D)** Test 1 - RHEs stressed with UV in the presence of RoxP-enriched *C*. *acnes* supernatant at 10% v/v.

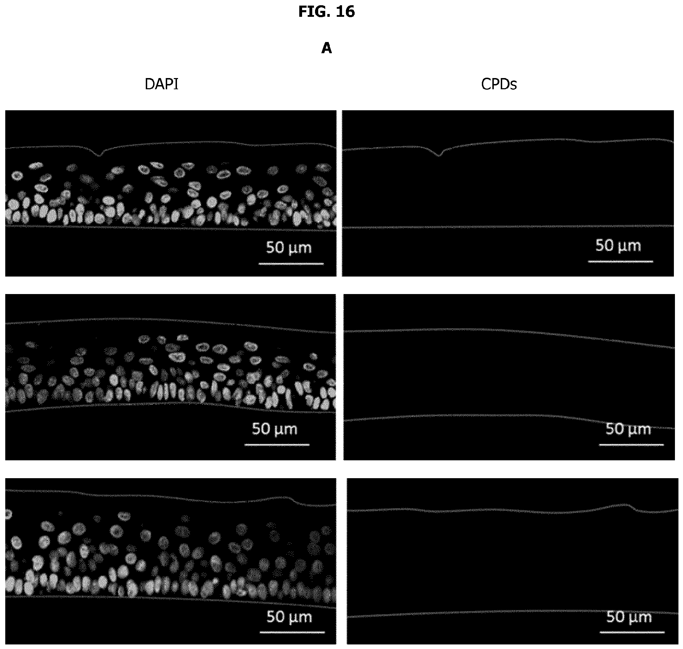

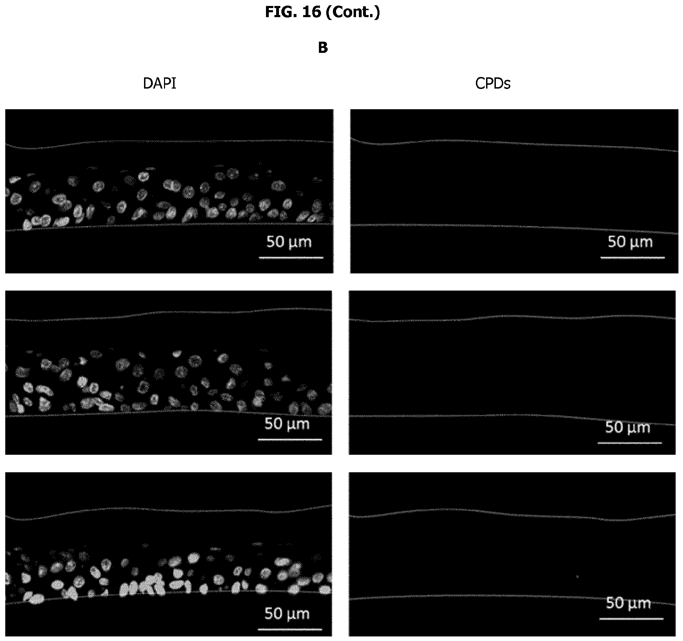

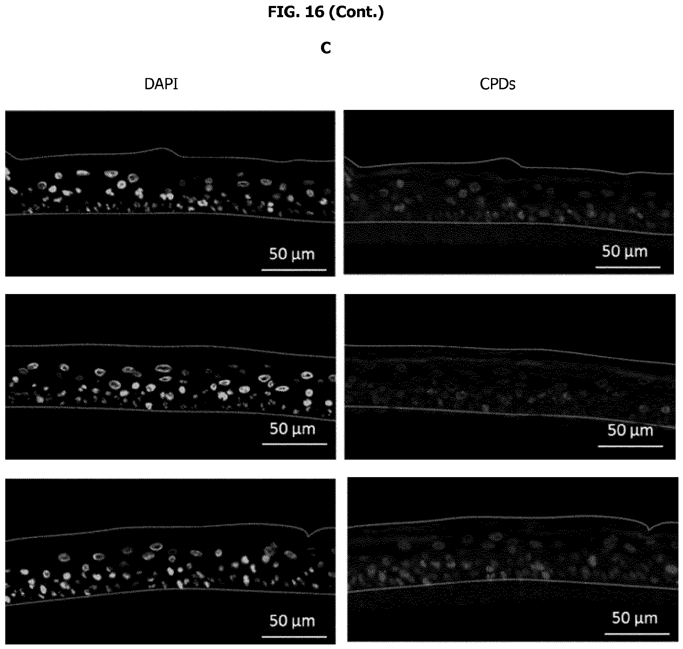

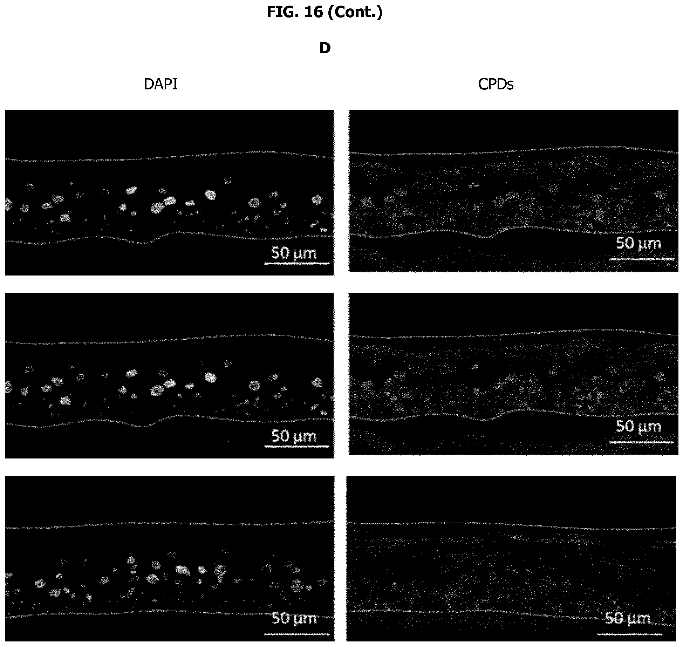

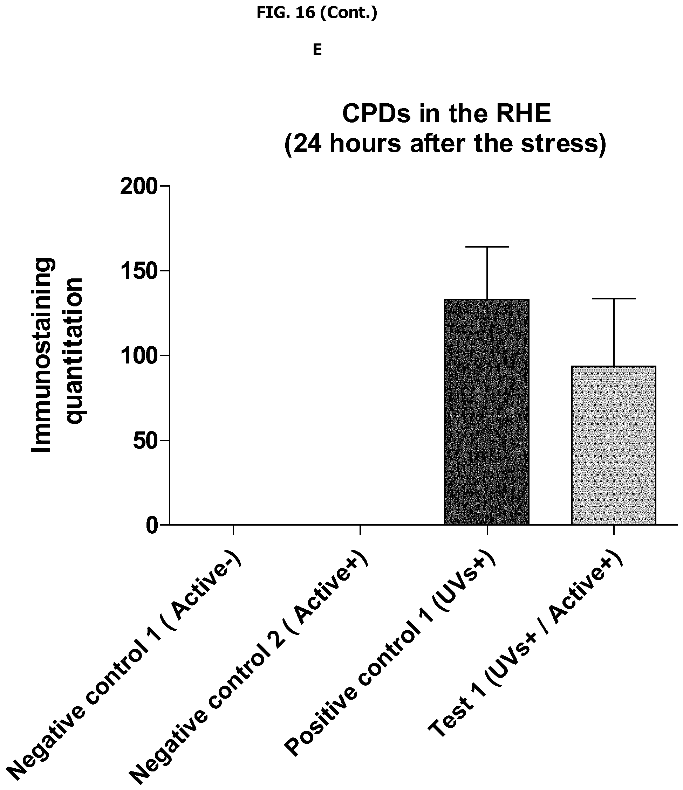

**Figure 16****:** DNA damage / CPDs evaluation of RHEs exposed to UV stress. **(A)** Negative control 1 - unstressed RHEs without RoxP-enriched *C*. *acnes* supernatant, **(B)** Negative control 2 - unstressed RHEs with RoxP-enriched *C*. *acnes* supernatant at 10% v/v, **(C)** Positive control 1 - RHEs stressed with UV, **(D)** Test 1 - RHEs stressed with UV in the presence of RoxP-enriched *C*. *acnes* supernatant at 10% v/v, **(E)** Quantification of the results shown in (A)-(D).

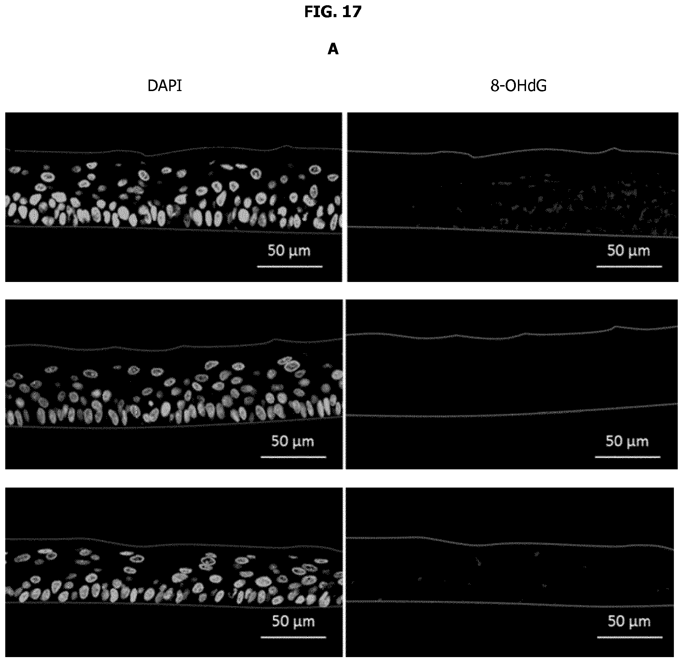

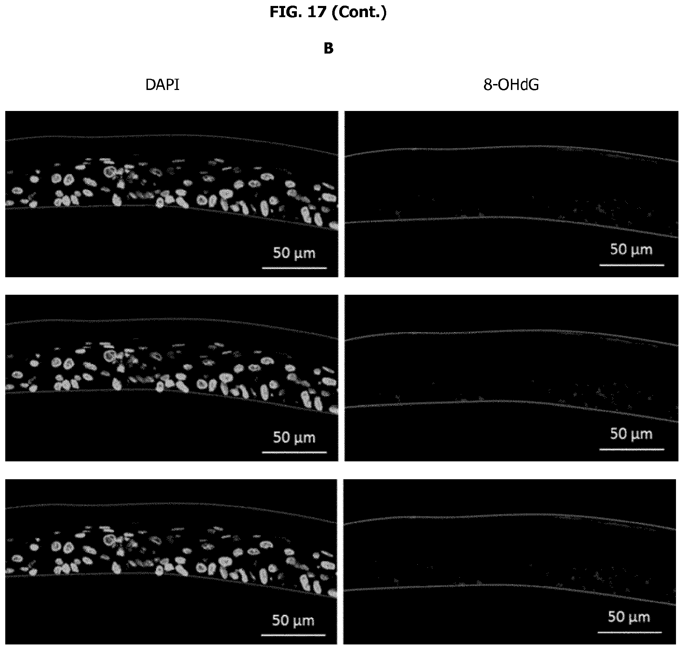

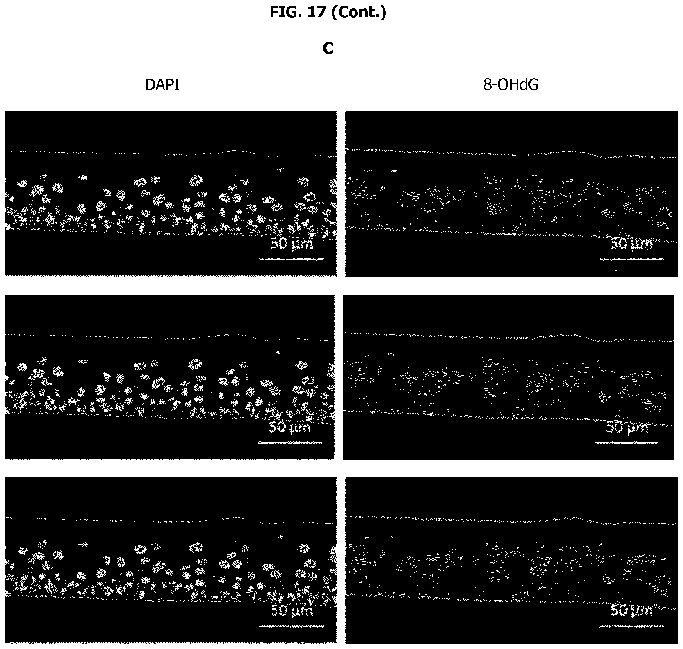

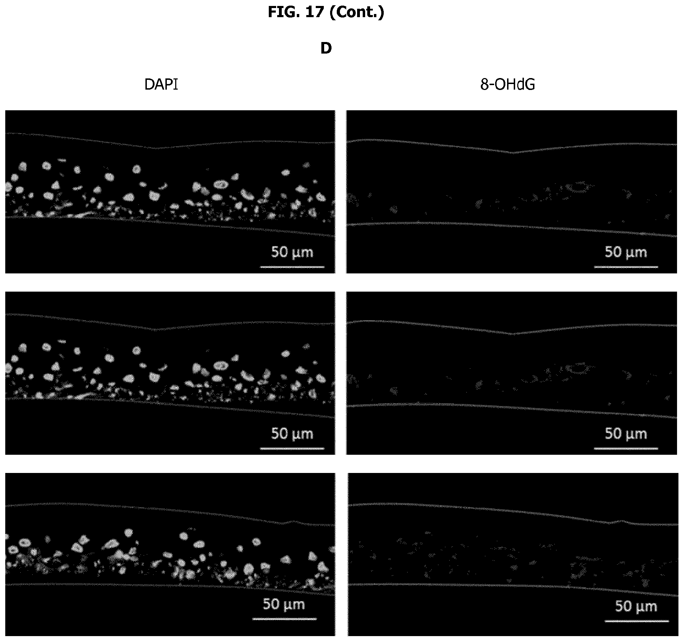

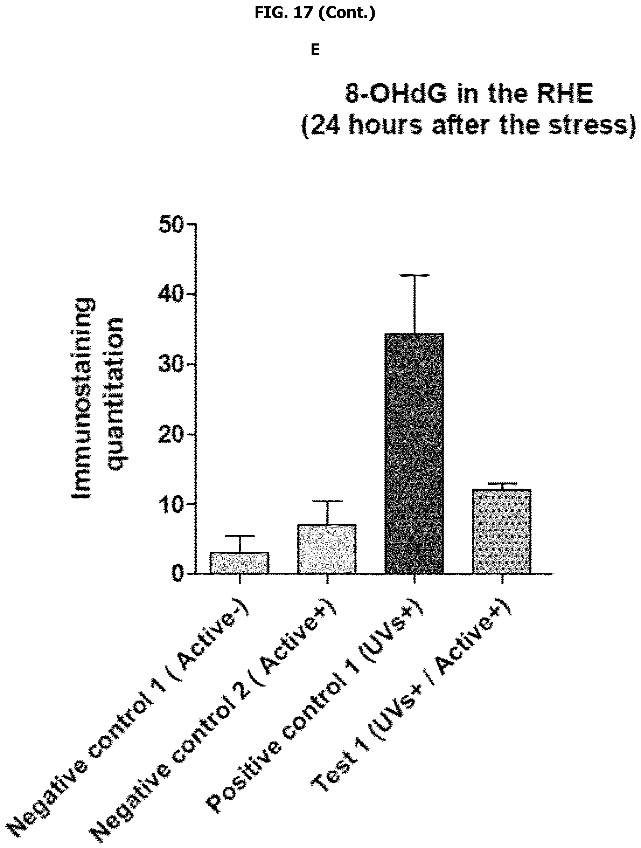

**Figure 17****:** DNA damage / 8-OHdG evaluation of RHEs exposed to UV stress. **(A)** Negative control 1 - unstressed RHEs without RoxP-enriched *C*. *acnes* supernatant, **(B)** Negative control 2 - unstressed RHEs with RoxP-enriched *C*. *acnes* supernatant at 10% v/v, **(C)** Positive control 1 - RHEs stressed with UV, **(D)** Test 1 - RHEs stressed with UV in the presence of RoxP-enriched *C*. *acnes* supernatant at 10% v/v, **(E)** Quantification of the results shown in (A)-(D).

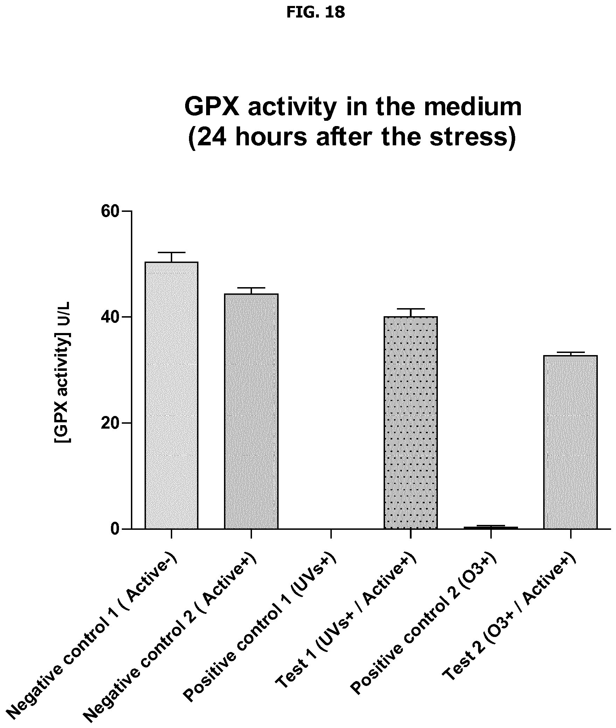

**Figure 18****:** System detoxification evaluation (GPx analysis) of RHEs exposed to UV or ozone stress. Negative control 1 - unstressed RHEs without RoxP-enriched *C*. *acnes* supernatant, Negative control 2 - unstressed RHEs with RoxP-enriched *C*. *acnes* supernatant at 10% v/v, Positive control 1 - RHEs stressed with UV, Test 1 - RHEs stressed with UV in the presence of RoxP-enriched C. *acnes* supernatant at 10% v/v, Positive control 2 - RHEs stressed with ozone, Test 2 - RHEs stressed with ozone in the presence of RoxP-enriched *C*. *acnes* supernatant at 10% v/v.

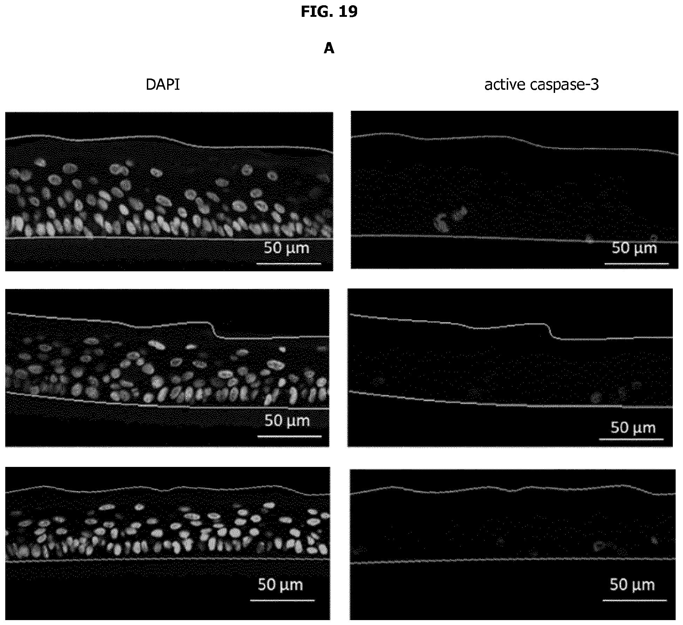

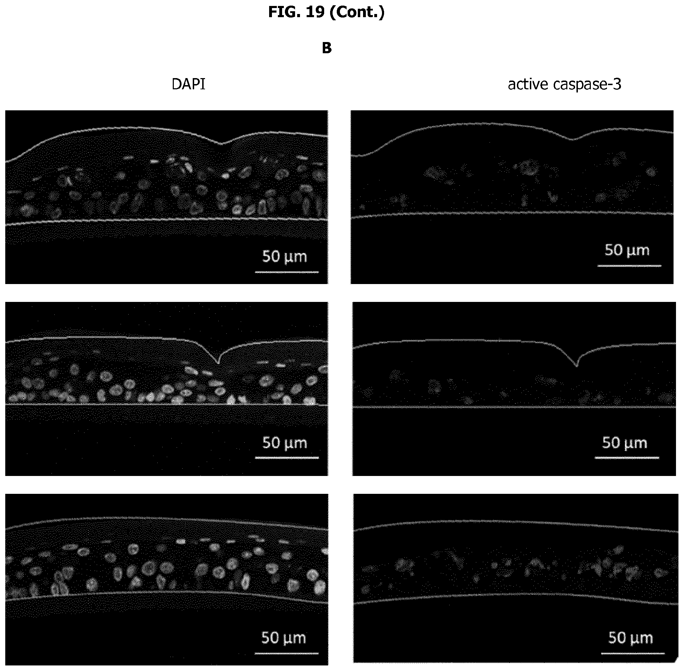

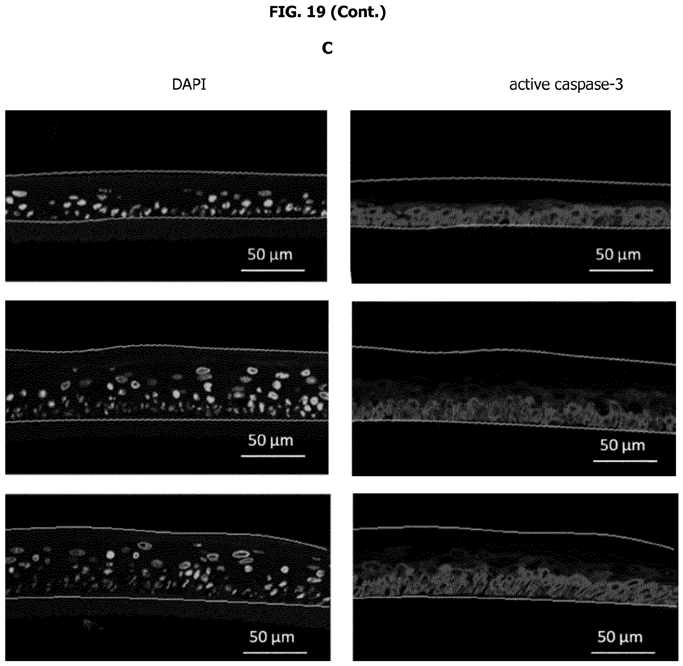

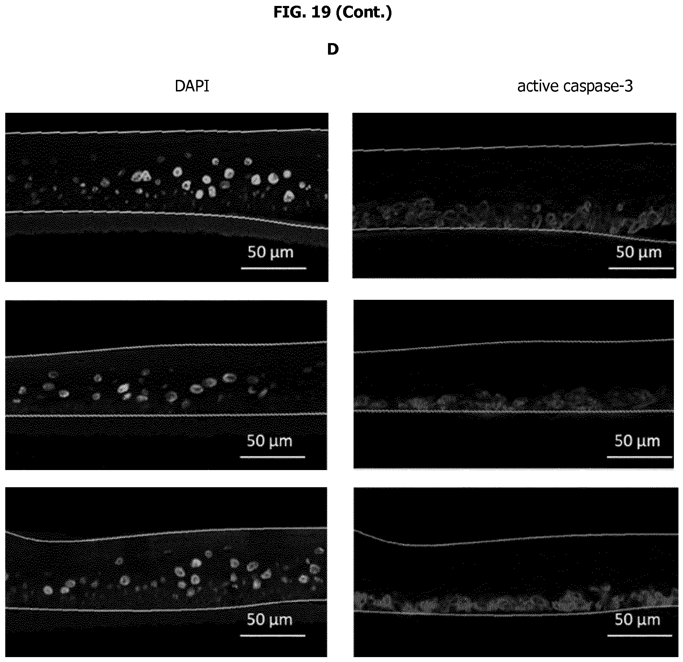

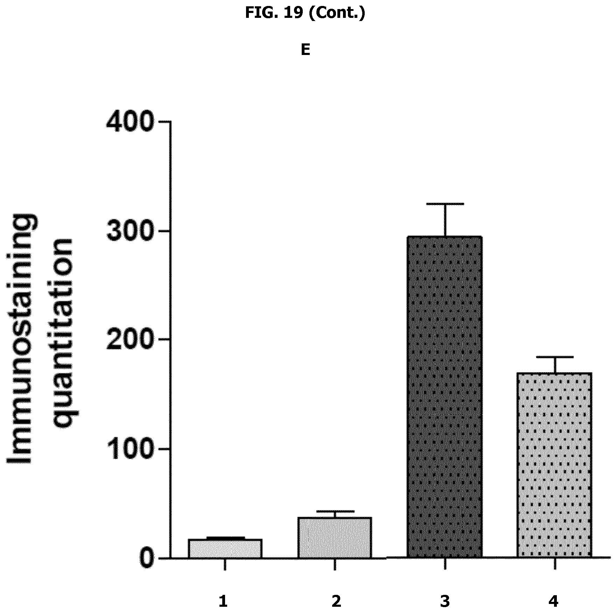

**Figure 19****:** Apoptosis evaluation (active caspase-3) evaluation of RHEs exposed to UV stress. **(A)** Negative control 1 - unstressed RHEs without RoxP-enriched *C*. *acnes* supernatant, **(B)** Negative control 2 - unstressed RHEs with RoxP-enriched *C*. *acnes* supernatant at 10% v/v, **(C)** Positive control 1 - RHEs stressed with UV, **(D)** Test 1 - RHEs stressed with UV in the presence of RoxP-enriched *C*. *acnes* supernatant at 10% v/v, **(E)** Quantification of the results shown in (A)-(D), bar #1 Negative control 1, bar #2 Negative control 2, bar #3 Positive control 1, bar #4 Test 1.

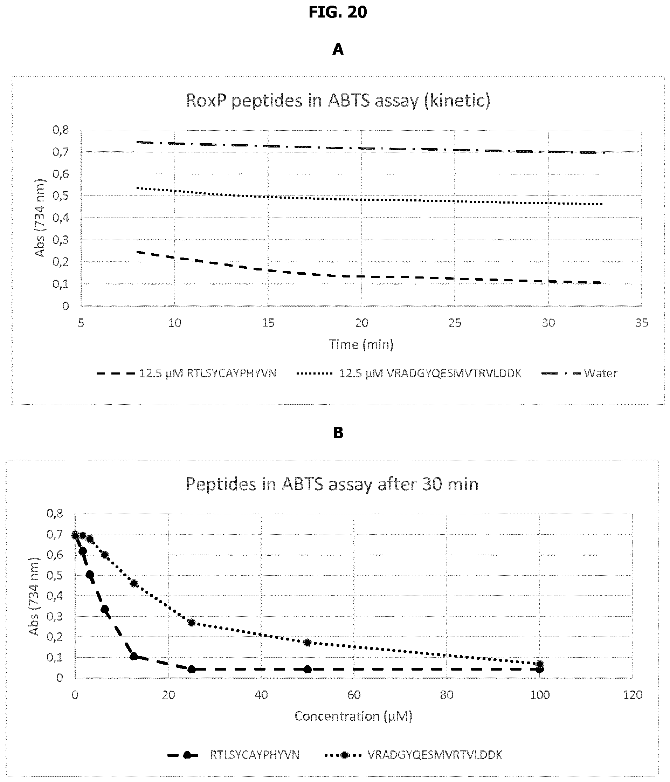

**Figure 20****:** ABTS radical cation assay using RoxP-Peptide 1 (dotted curve) or RoxP-Peptide 2 (dashed curve), and water as a control (dashed and dotted curve). **(A)** kinetics analysis, **(B)** assay of concentration dependence.

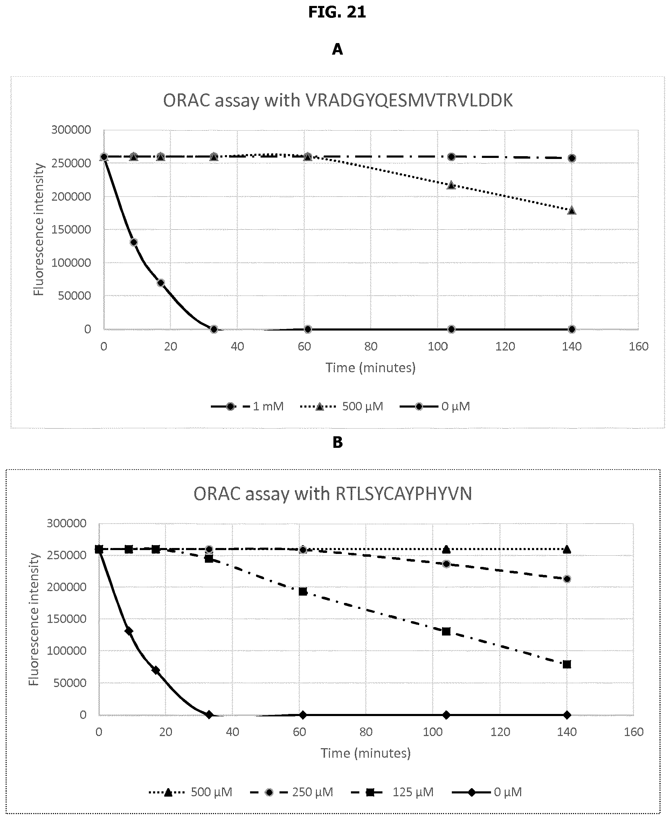

**Figure 21****:** ORAC (oxygen radical absorbance capacity) assay using RoxP-Peptide 1 **(A)** or RoxP-Peptide 2 **(B).**

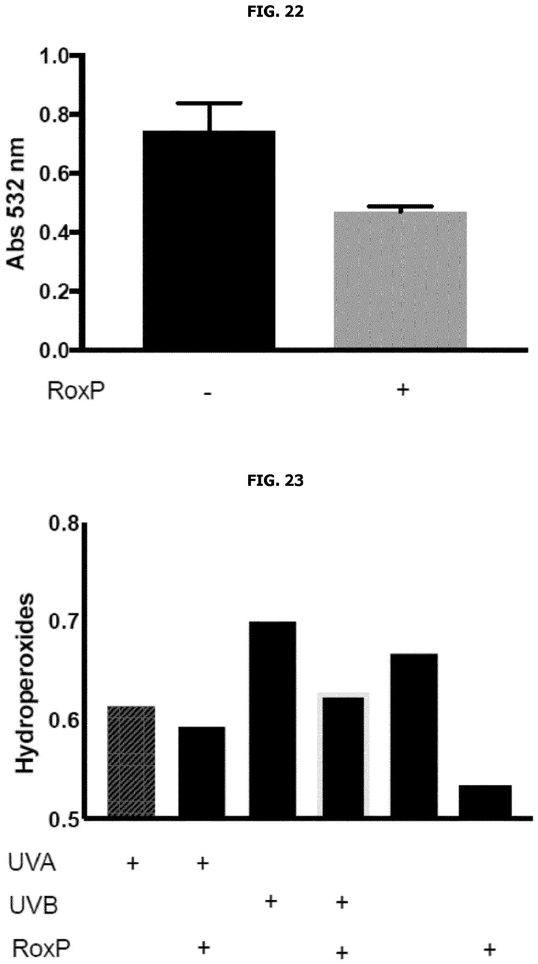

**Figure 22****:** RoxP decreased generation of •OH radicals, as evidenced by reduced absorbance at 532 nm in deoxyribose (TBA-MDA) assay in the presence of RoxP (right bar).
**Figure 23****:** RoxP reduced lipid hydroperoxide levels in UV irradiated and non-irradiated plasma.

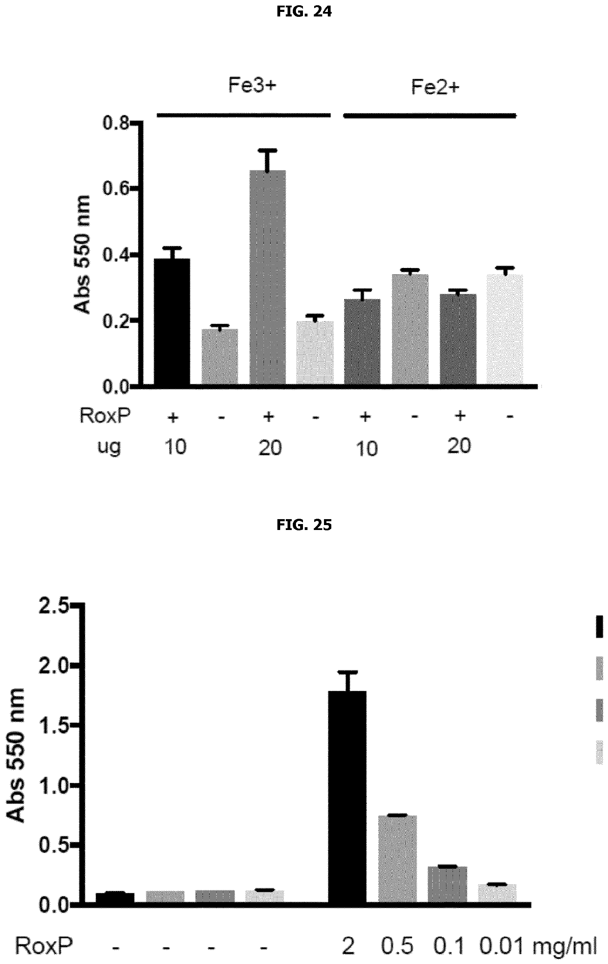

**Figure 24****:** RoxP reduced Fe³⁺ to Fe²⁺, and did not affect redox state of Fe²⁺ in phenantroline assay.
**Figure 25****:** RoxP reduced Fe³⁺ to Fe²⁺ in ferrozine assay.

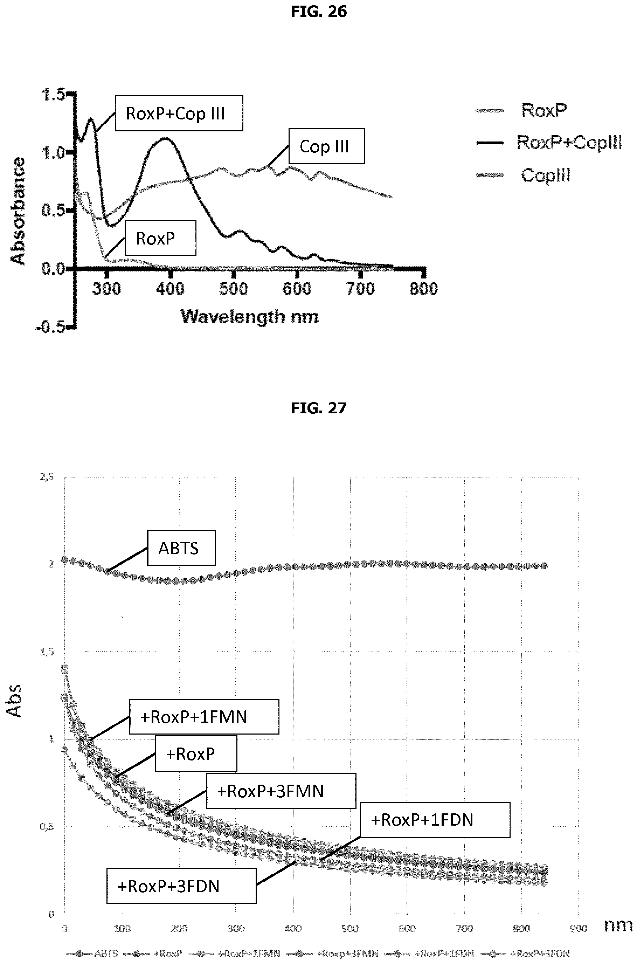

**Figure 26****:** RoxP interaction with coproporphyrin III.
**Figure 27****:** Flavins and RoxP activity in ABTS reduction assays.

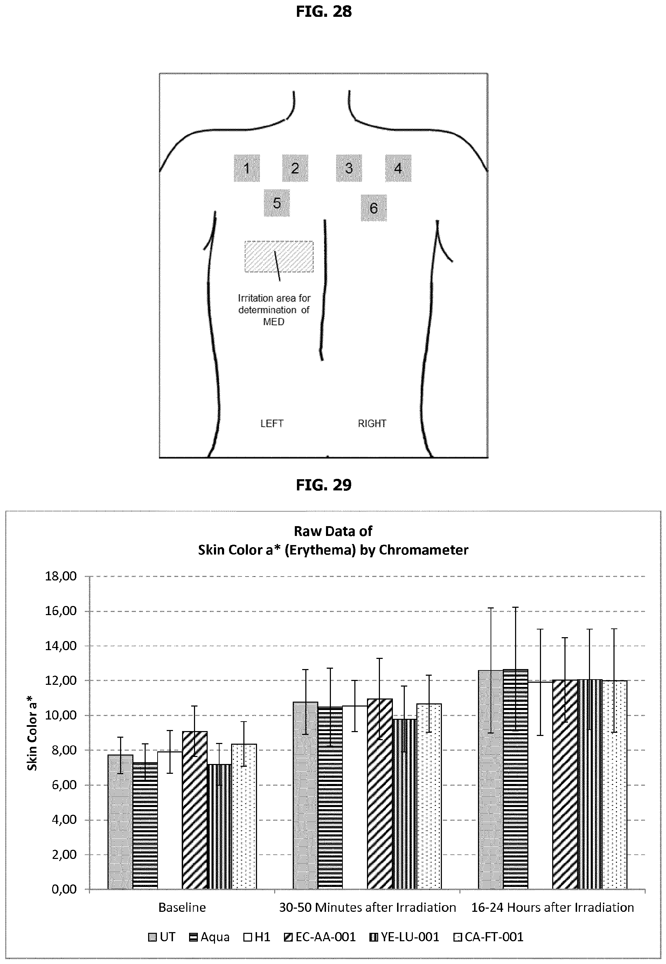

**Figure 28****:** Treatment areas 1 to 6 and irradiation areas in the study according to Example 19.
**Figure 29****:** Skin color a* (erythema) by Chromameter in an *in vivo* study of RoxP (raw data).

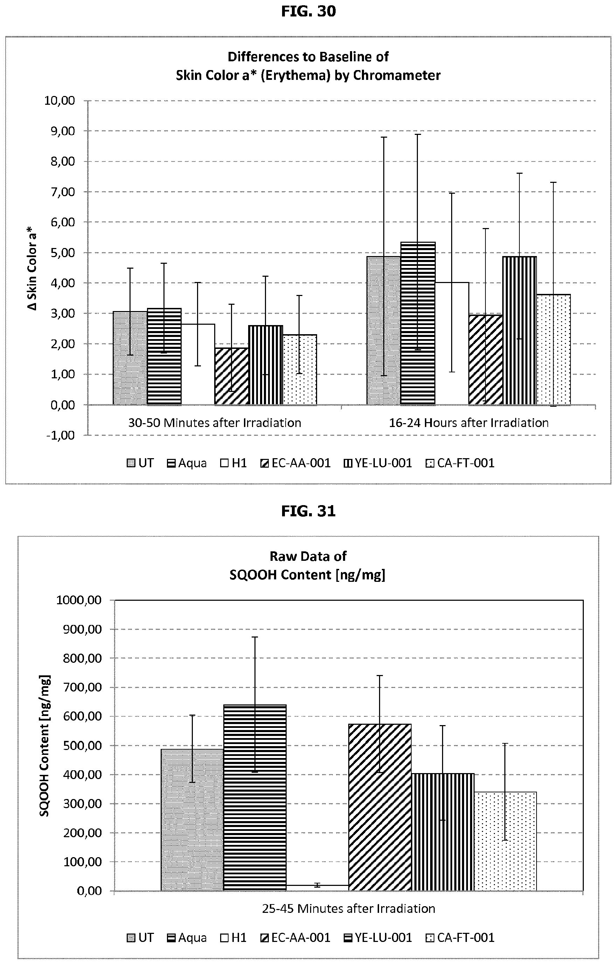

**Figure 30****:** Skin color a* (erythema) by Chromameter in an *in vivo* study of RoxP (differences to baseline).
**Figure 31****:** SQOOH content (ng/mg) in an *in vivo* study of RoxP (raw data).

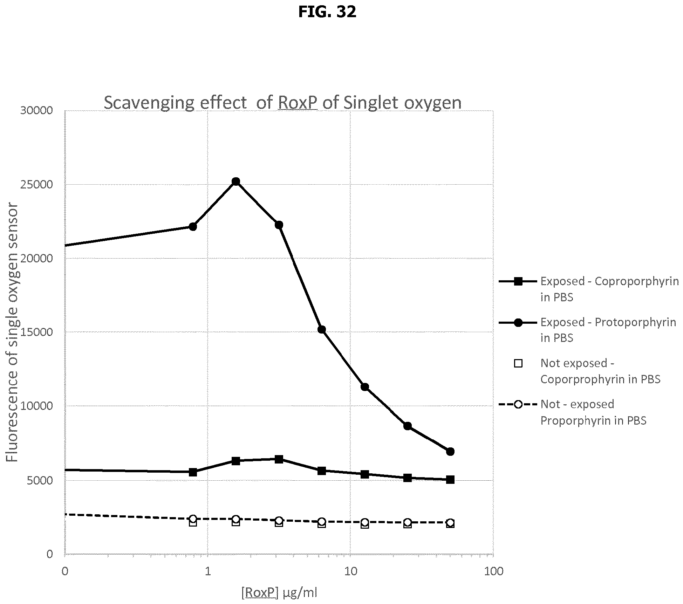

**Figure 32****:** RoxP reduced the generation of radicals formed through porphyrins.

### DETAILED DESCRIPTION OF THE INVENTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of", which enjoy well-established meanings in patent terminology.

The recitation of numerical ranges by endpoints includes all numbers and fractions subsumed within the respective ranges, as well as the recited endpoints. This applies to numerical ranges irrespective of whether they are introduced by the expression "from... to..." or the expression "between... and..." or another expression.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation or meaning is meant to apply throughout this specification, i.e., also in the context of other aspects or embodiments of the invention, unless otherwise defined.

In the following passages, different aspects or embodiments of the invention are defined in more detail.

As corroborated by the experimental section, which illustrates certain representative embodiments of the invention, by employing relevant model systems informative of treatment outcomes, the inventors for the first time identified and validated RoxP or C. *acnes* bacteria secreting RoxP as useful actives for cosmetic, prophylactic or therapeutic interventions to prevent or reduce skin ageing, or to prevent or treat oxidative stress-associated skin diseases in subjects. The experimental section demonstrates that the compositions described herein have antioxidant properties. Hence, without wishing to be bound by theory, the cosmetic, prophylactic or therapeutic approaches disclosed herein may operate by protecting skin cells and/or cellular or extracellular components of the skin, such as proteins, lipids or nucleic acids, from oxidative stress, particularly by quenching or neutralising free radicals including reactive oxygen species (ROS) in the skin by the RoxP administered to the skin or delivered to the skin by *C*. *acnes* bacteria secreting RoxP.

Accordingly, an aspect of the invention provides a cosmetic use of a composition comprising a live *Cutibacterium acnes* strain secreting RoxP (radical oxygenase of *Propionibacterium acnes)* for preventing or reducing skin ageing in a subject. A related aspect provides a cosmetic method for preventing or reducing skin ageing in a subject, the method comprising administering to the skin of the subject a cosmetically effective amount of a composition comprising a live *C*. *acnes* strain secreting RoxP.

Another aspect provides a composition comprising a live *C*. *acnes* strain secreting RoxP for use in a method of preventing or treating an oxidative stress-associated skin disease, wherein he oxidative stress-associated skin disease is selected from the group comprising actinic keratosis (AK) and basal cell carcinoma (BCC).

A further aspect provides a cosmetic use of a composition comprising RoxP for preventing or reducing skin ageing in a subject. A related aspect provides a cosmetic method for preventing or reducing skin ageing in a subject, the method comprising administering to the skin of the subject a cosmetically effective amount of a composition comprising RoxP or a biologically active variant or fragment thereof.

Another aspect provides a composition comprising RoxP for use in a method of preventing or treating an oxidative stress-associated skin disease, wherein the oxidative stress-associated skin disease is selected from the group comprising actinic keratosis (AK) and basal cell carcinoma (BCC).

The adjective "cosmetic" in connection with uses and methods disclosed herein denotes that such uses and methods are designed to maintain, restore, improve or enhance a subject's appearance, more particularly the appearance of the subject's skin, including without limitation the tone, colour, complexion, texture, smoothness or softness of the subject's skin. Cosmetic uses or methods as envisaged herein address normal, natural, or physiological processes, and can be distinguished from therapy including curative and prophylactic treatments, the purpose of which is to restore a subject from a pathological state to its original healthy condition, or to at least alleviate the symptoms of pain and suffering caused by the pathology, or to prevent pathology in the first place. Cosmetic uses or methods as intended herein can thus be denoted as "non-therapeutic". Cosmetic uses or methods as intended herein generally employ cosmetic compositions configured for topical application to the skin.

The term "skin" as used herein generally refers to the layer of tissue forming the natural outer covering of the body of animals, in particular vertebrates. Mammalian skin is composed of two primary layers: the epidermis which constitutes the outermost layer of the skin, includes stratified squamous epithelium with an underlying basal lamina, and comprises Merkel cells, keratinocytes, melanocytes and Langerhans cells; and the dermis, which constitutes the layer of skin beneath the epidermis, and comprises connective tissue, mechanoreceptors, hair follicles, sweat glands, sebaceous glands, apocrine glands, lymphatic vessels and blood vessels. The term "skin" includes without limitation the skin on or in the face, neck, chest, abdomen, back, arms, hands, legs, and scalp.

Whereas competing schools of thought may exist as to whether ageing constitutes a natural, physiological process or 'the greatest disease of them all', as used herein the terms "ageing" or "aging" generally refer to the process of growing old, separate from any age-related pathology, whereby subjects display the normal, natural or physiological effects or characteristics of increasing age.

In certain embodiments, the present cosmetic uses or methods may prevent or reduce the appearance of one or more external signs of the ageing of the skin. In certain embodiments, the one or more external signs of the ageing of the skin may be perceivable by visual or tactile inspection. In certain embodiments, the one or more external signs of the ageing of the skin may be visible by naked eye. In certain embodiments, the one or more external signs of the ageing of the skin may be selected from the group comprising, consisting essentially of, or consisting of wrinkles, fine lines, sagging skin, loss of elasticity of skin fibres, withered skin, thinned skin, alteration in skin pigmentation, dull skin, skin without radiance, and combinations thereof. Signs of skin ageing that may be particularly associated with or caused by oxidative stress include fine lines, wrinkles, alteration in skin pigmentation, and thinned skin. Hence, in certain embodiments, the one or more external signs of the ageing of the skin may be selected from the group comprising, consisting essentially of, or consisting of fine lines, wrinkles, alteration in skin pigmentation, thinned skin, and combinations thereof.

The term "to prevent", or its alternative forms such as "preventing" or "prevention", commonly means to keep something from occurring, happening or existing, or to delay the occurrence or onset of something, especially by one or more precautionary (preventative) measures. For example, the present cosmetic uses or methods may be applied to a subject not yet displaying one or more external signs of skin ageing, and may prevent, such as prevent for a given duration of time (delay), the onset or arrival of said one or more external signs of skin ageing.

The term "to reduce", or its alternative forms such as "reducing" or "reduction", commonly means to diminish something in size, amount, extent, or number, especially by one or more measures, usually compared to the state or situation before applying said measure(s) or to the state or situation that would ensue if the measure(s) were not applied. For example, the present cosmetic uses or methods may be applied to a subject already displaying one or more external signs of skin ageing, and may either lessen said one or more external signs (restoration, improvement) or may thwart or slow down further development or worsening of said one or more external signs (maintenance, management). The term "to reduce" can be interchangeable with terms such as "to decrease", "to inhibit", "to diminish", "to interfere with", or "to slow down".

The term "composition" generally refers to a thing composed of two or more components, and more specifically denotes a combination or mixture of two or more materials, such as elements, molecules or substances, as well as reaction products and decomposition products formed from the materials of the composition. Having regard to their usage, the present compositions may be configured as cosmetic compositions or as pharmaceutical compositions. Cosmetic compositions typically comprise one or more cosmetically active ingredients (chemically and/or biologically active substances having one or more cosmetic effects) and one or more cosmetically acceptable carriers. Pharmaceutical compositions typically comprise one or more pharmacologically active ingredients (chemically and/or biologically active substances having one or more pharmacological effects) and one or more pharmaceutically acceptable carriers. Compositions as typically used herein may be liquid or solid, and may include solutions or dispersions. In certain embodiments, a composition may be composed of components that are provided to a consumer or a practitioner as a mixture, i.e., the composition components are already admixed. In certain embodiments, a composition may be composed of components one or more of which are provided to a consumer or practitioner physically separated from (e.g., in separate containers or vials) from one or more other components of the composition, although typically as part of the same product package or dispensing device. By means of an example and without limitation, the composition may comprise one or more components provided in one container, such as one chamber of a multiple-chamber device (e.g., a two-chamber dispensing system), and one or more components provided in another container, such as another chamber of the multiple-chamber device. Such arrangement allows the consumer or practitioner to admix the components of the composition shortly before use. For example, the composition may comprise the live *Cutibacterium acnes* strain or strains secreting RoxP as taught herein provided in one container, such as one chamber of a multiple-chamber device (e.g., a two-chamber dispensing system), and one or more cosmetically or pharmaceutically acceptable carriers provided in another container, such as another chamber of the multiple-chamber device, to be admixed by the consumer or practitioner before use. Multiple-chamber (such as two- or dual-chamber) dispensers, such as bottles, tubes, jars, pumps, etc. are generally known and commercially available. By means of an example and not limitation, WO 2012/153206, WO 2017/168263, and WO 2018/060799 by Bormioli Rocco SPA (Fidenza, Italy) describe systems, marketed *inter alia* as the New Shaker^{®} single-dose dispenser, comprising a container and a closure capsule for closing the container, wherein the closure capsule includes an enclosure defined at least partly by a frangible bottom or mouth. Changing the configuration of the frangible bottom or mouth from intact to open allows to mix the previously separated contents of the container and the enclosure directly before use. In certain embodiments, the live *Cutibacterium acnes* strain or strains secreting RoxP may be in a lyophilised form. Typically, such form is itself a composition comprising the live *Cutibacterium acnes* strain or strains and components of a suitable bacterial lyophilisation medium. In certain embodiments, the RoxP thereof may be in a lyophilised form. Typically, such form is itself a composition comprising the RoxP or biologically active variant or fragment thereof and optionally suitable protein lyophilisation excipient(s).

In certain embodiments, whereas a composition may be a mixture, one or more components of the composition may nonetheless be substantially separated from (substantially not in contact with) one or more other components of the composition. Such may be the case for example for dispersions, such as colloids or suspensions, which comprise discrete particles dispersed in a continuous phase. Hence, component(s) of a dispersion which are comprised by the discrete particles will be substantially separated from component(s) of a dispersion comprised by the continuous phase. By means of an example, RoxP may be formulated with one or more excipients to form discrete particles in a continuous phase composed of one or more other excipients, or *vice versa.* By means of another example, a live *Cutibacterium acnes* strain or strains secreting RoxP may be formulated with one or more excipients to form discrete particles in a continuous phase composed of one or more other excipients, or *vice versa.* In certain embodiments, a dispersion as intended herein may be an emulsion (liquid dispersed phase, liquid continuous phase), sol or suspension (solid dispersed phase, liquid continuous phase), gel (liquid dispersed phase, solid continuous phase), or solid sol (solid dispersed phase, solid continuous phase). Another example includes compositions in which one or more components of the composition are admixed and microencapsulated, before being admixed with one or more further components of the composition. By means of an example, RoxP may be formulated with one or more excipients and microencapsulated, and the resulting microcapsules may be subsequently formulated with (dispersed in) one or more excipients. By means of another example, a live *Cutibacterium acnes* strain or strains secreting RoxP may be formulated with one or more excipients and microencapsulated, and the resulting microcapsules may be subsequently formulated with (dispersed in) one or more excipients. In certain embodiments, the microencapsulated formulation may be liquid or solid, such as a liquid or solid solution or dispersion. In certain embodiments, the microcapsules may be admixed with a formulation that is liquid or solid, such as a liquid or solid solution or dispersion. Methods for microencapsulation are generally known, and the shell, coating, or membrane of microcapsules may be generally made of materials such as ethyl cellulose, polyvinyl alcohol, gelatin, or sodium alginate.

Advantageously, in such embodiments, a live *C*. *acnes* strain or strains or RoxP thereof may be maintained substantially in separation from components of the composition which, while being generally useful in the context of cosmetic or pharmaceutical compositions, may detract from the viability, stability or other desirable characteristics of the active.

As mentioned previously, in certain embodiments, the live *Cutibacterium acnes* strain or strains secreting RoxP, or the RoxP, may be in a lyophilised form; optionally included in one chamber of a two-chamber dispensing system, the other chamber containing a suitable resuspension composition. Methods for lyophilisation of bacteria such as *C*. *acnes* are generally known (see for example, C Morgan & G Vesey: "Freeze-Drying of Microorganisms", In: Encyclopedia of Microbiology, 3rd ed., 2009, Academic Press, 162-173). By means of an example, *C*. *acnes* strain or strains may be grown in a suitable growth medium as described elsewhere in this specification and harvested in the exponential or stationary phase. The bacteria are separated from the media by centrifugation or ultrafiltration and washed in a suitable buffer. After washing the bacteria are resuspended in mixture of excipients stabilising the bacteria. Commonly used excipients include but not limited to sucrose, starch, mannitol, skim milk, bovine serum albumin (BSA), and combinations thereof. The excipients mix usually contains an external cryoprotectant (preventing ice crystal formation) an internal lyoprotectant (replacing the water as hydrogen bond donor) and storage stabilisers. For each mix of excipients the critical temperature is determined by an appropriate method, such as lyostat measurement or differential scanning calorimetry. The product containing the bacteria and excipients is frozen in the freeze dryer below the critical temperature. The programme parameters of the freeze dryer are chosen so that the product stays during entire freeze drying cycle at a temperature below the critical temperature of the mixture. After evaporation of all water from the product it can be removed from the freeze dryer and processed for the final packaging.

Lyophilised bacteria may be provided as a lyophilised powder, optionally admixed with a solid carrier to increase the volume, or may be dispersed in a suitable oil or combination of oils. Non-limiting examples of suitable oils include Simmondsia Chinensis (Jojoba) Seed Oil, Sesamum Indicum (Sesame) Seed Oil, Butyrospermum Parkii (Shea) Butter, Hydrogenated Vegetable Oil, Isoamyl Laurate, Sclerocarya Birrea (Marula) Seed Oil, Helianthus Annuus (Sunflower) Seed Oil, and combinations thereof. Such and other oils, in particular hydrophilic oils, prevent moisture from reaching the bacteria, thereby preventing rehydration of the bacteria and extending the shelf life of the product.

Lyophilisation of proteins is also a well-established procedure in the art and typically employs an aqueous solution of the protein to be lyophilised, optionally admixed with one or more suitable lyophilisation excipients, such as trehalose, sucrose, raffinose, maltodextrin, mannitol, and/or dextran. The inventors have experimentally determined that lyophilisation of RoxP dissolved in water satisfactorily preserved the activity of RoxP, even in the absence of further excipients, while the addition of excipients, including those recited above each individually, was well-tolerated and could lead to improvements of cake in terms of its stability and easiness of reconstitution. The inventors have additionally observed that mannitol, and particularly 2-6% w/v, preferably 2-5% w/v, more preferably 2-4% w/v, or even more preferably 2-3% w/v mannitol (relative to the volume of the solution to be lyophilised) was especially advantageous for production of stable cake and preservation of RoxP activity.

In certain other embodiments, bacteria may be applied without an intermediate lyophilisation step. For example but without limitation, bacteria may be grown to and harvested at exponential phase, pelleted and resuspended in dilute peptone solution (0,25% w/v tryptic peptone in sterile distilled water), which is then thickened with hydroxyethylcellulose (also known as HEC or Natrosol), at a suitable final concentration of bacteria, such as about 10⁶ colony forming units per gram. The resulting gel can be stored at -80°C or -20°C or for short term at room temperature before it is applied to subjects.

In certain embodiments, the composition is a cosmetic composition comprising a live C. *acnes* strain secreting RoxP and a cosmetically acceptable carrier. In certain embodiments, the composition is a cosmetic composition comprising RoxP and a cosmetically acceptable carrier. In certain embodiments, the composition is a cosmetic composition comprising both i) a live C. *acnes* strain secreting RoxP and ii) RoxP, and a cosmetically acceptable carrier. The cosmetic compositions may further comprise one or more additional cosmetically active ingredients. Such additional cosmetically active ingredient(s) may be capable of preventing or reducing skin ageing, or may possess cosmetic effects unrelated to skin ageing.

In certain embodiments, the composition is a pharmaceutical composition comprising a live C. *acnes* strain secreting RoxP and a pharmaceutically acceptable carrier. In certain embodiments, the composition is a pharmaceutical composition comprising RoxP and a pharmaceutically acceptable carrier. In certain embodiments, the composition is a pharmaceutical composition comprising both i) a live *C*. *acnes* strain secreting RoxP and ii) RoxP, and a pharmaceutically acceptable carrier. The pharmaceutical compositions may further comprise one or more additional pharmaceutically active ingredients. Such additional pharmaceutically active ingredient(s) may be capable of preventing or treating the oxidative stress-associated skin disease, or may possess pharmaceutical effects unrelated to said disease.

By means of example and without limitation, such cosmetic or pharmaceutical actives may include skin-applicable anti-oxidants, such as without limitation vitamin C, vitamin A, vitamin E, resveratrol, coenzyme Q10, niacinamide, polyphenols, flavonoids, or combinations thereof.

By means of example and without limitation, such cosmetic or pharmaceutical actives may include alpha-lipoic acid, folic acid, phytoene, biotin, alpha-glucosyl rutin, carnitine, carnosine, natural and/synthetic isoflavones, flavonoide, creatine, creatinine, taurine, B-alanine, dihydroxyacetone, 8-hexadecene- 1, 16-dicarboxylic acid, glyceryl glucoside, licorice extract, aloe vera, hyaluronic acid, aloe barbadensis leaf juice, (2-hydroxyethyl)urea, niacinamide, vitamin A, or combinations thereof.

The terms "cosmetically acceptable" or "pharmaceutically acceptable" as used herein are consistent with the art and mean compatible with the other ingredients of a cosmetic or pharmaceutical composition and not deleterious to the recipient thereof.

Certain of the present compositions comprise a live *C*. *acnes* strain secreting RoxP. In certain embodiments, the compositions may comprise exactly one live *C*. *acnes* strain secreting RoxP. In certain embodiments, the compositions may comprise two or more live *C*. *acnes* strains secreting RoxP. For example, the compositions may comprise exactly two live *C*. *acnes* strains secreting RoxP. In certain embodiments, the compositions may comprise exactly 3, 4, 5, 6, 7, 8, 9, 10, 11, 12, 13, 14, 15, 16, 17, 18, 19, or 20 or may comprise more than 20 live *C*. *acnes* strains secreting RoxP. In certain embodiments, the compositions may comprise exactly 3, 4, or 5 live *C*. *acnes* strains secreting RoxP.

*Cutibacterium acnes* (formerly *Propionibacterium acnes)* is a species of facultative anaerobic Gram-positive rod bacteria. *C*. *acnes* is largely commensal and is an abundant colonizer of human skin thriving in the nutritious environment of sebum rich skin. The bacterium causes no symptoms in the majority of carriers, but certain strains have a suggested pro-inflammatory role in acne vulgaris. Moreover, due to its biofilm-forming capacity, the bacterium is frequently isolated from various orthopaedic implant-associated infections.

Studies by Johnson and Cummins (1972 J Bacteriol 109:1047-66) first revealed two distinct phenotypes of *Propionibacterium acnes,* known as types I and II, that could be distinguished based on serological agglutination tests and cell wall sugar analysis. Subsequent studies, involving among others sequence analysis of the *recA* gene demonstrated that *P. acnes* comprises four highly distinct evolutionary lineages, known as type IA, IB, II and III that display differences in inflammatory properties, production of virulence determinants and association with various conditions (McDowell et al. 2005 J Clin Microbiol 43:326-334; McDowell et al. 2008 J Med Microbiol 57:218-224; Valanne et al. 2005 Microbiology 151:1369-1379; Lodes et al. 2006 Microbiology 152: 3667-3681). Type IA has been further subdivided into subtypes IA1 and IA2, and type IC has been identified using a multilocus sequence typing scheme (eMLST) based on six housekeeping genes and two putative virulence gene (McDowell et al., 2012 PLoS One 7:e41480). Scholz et al. (2014 PLoS ONE 9:e104199) later developed a single-locus sequence typing (SLST) scheme for *P. acnes* strains, involving PCR amplification and DNA sequencing of the target locus PPA2385. A publically available *P*. *acnes* database and SLST allocation tool associated with the SLST scheme described in by Scholz et al. is available online at medbac.dk/slst/pacnes. Exemplary SLST types for *P. acnes* strains include SLST types A1 to A24, B1, C1 to C4, D1 to D3, E1 to E9, F1 to F10, G1, H1 to H5, K1 to K14, and L1 to L6. Other *P. acnes* strain typing schemes are know as MLST9, MLST8, and Ribotype schemes, reviewed in Scholz et al., especially Figure 1. Where SLST types are referred to in this specification, the types according to the SLST typing scheme of Scholz et al. are meant. As further guidance but without limitation, Table 1 on p. 20-31 of WO 2018/073651, incorporated by reference herein, lists several allelic sequences of the target locus PPA2385 used in Scholz et al.'s SLST to identify SLST types of *P. acnes* strains.

Illustrative but non-limiting strains of *C*. *acnes* useful herein can be obtained from public microorganism collections maintained for example by Leibniz Institute DSMZ-German Collection of Microorganisms and Cell Cultures (Inhoffenstr. 7B, D-38124 Braunschweig, Germany, https://www.dsmz.de/catalogues.html), or by American Type Culture Collection (ATCC) (10801 University Blvd. Manassas, Virginia 20110-2209, USA) or by European Collection of Cell Cultures (ECACC) (Health Protection Agency - Porton Down Salisbury, Wiltshire SP4 0JG, United Kingdom), including *C*. *acnes* strain KPA171202 (DSMZ acc. no. DSM-16379), *C*. *acnes* subsp. *defenders* strain (acc. no. ATCC 11828), *C*. *acnes* strain (acc. no. ATCC 6919), *C*. *acnes* subsp. *elongatum* strain (ECACC acc. no. NCTC 13655), and others.

Further, *C*. *acnes* strains of virtually any type or subtype useful herein can be readily sampled and isolated from the skin of adult humans, preferably healthy adults, or from *C*. *acnes* biofilms on infected orthopaedic prostheses. Any available protocol for isolation, culture and expansion of *C*. *acnes* can be adopted. Hence, in certain embodiments, a composition can be formed by isolating and culturing one or more *C*. *acnes* from a donor subject, particularly a donor subject not having oxidative stress-associated skin disease and preferably a donor subject having overall healthy skin, and combining it with other one or more carriers to form the composition.

By means of example and without limitation, one suitable protocol for obtaining *C*. *acnes* isolates from the skin was described in Holmberg et al. 2009 Clin Microbiol Infect 15:787-95). Therein, an area of 3x3 cm of the forehead of a human subject was swabbed with a cotton-tipped swab, moist with sterile physiological saline, and plated on blood agar (LabM Ltd, Heywood, Bury, United Kingdom) containing horse blood (4%). The plates were incubated under anaerobic conditions (10% H₂, 10% COz, 80% N₂) at 37°C for 72 hours. Isolates were characterised as *C*. *acnes* using routine microbiological criteria, including characteristic macroscopic appearance (typical small colonies), Gram-staining characteristics, and the production of catalase and indole. Isolates were frozen at -80°C in glycerol (50% v/v). The bacteria could be further cultivated in Bacto Brain Heart Infusion broth (BHI; Becton and Dickinson, Sparks, MD, USA) supplemented with glucose (0.5%) and kept in an anaerobic chamber for 72 h at 37°C. When performed on 48 healthy individuals, this yielded 48 *C*. *acnes* isolates belonging to types IA, IB, II (see Table 2 of Holmberg et al., incorporated by reference herein).

*C*. *acnes* cells can be further cultured and expanded in a variety of media, including rich or minimal media, preferably rich media such as those mentioned above. As would be understood by one of ordinary skill in the art, routine optimisation would allow for use of a variety of types of media. Media can be supplemented with various additional components, including sugar sources. Some non-limiting examples of supplemental components include glucose, amino acids, vitamins, lipids, glycerol, and ATCC Trace Mineral Supplement. Similarly, other aspects of the medium, and growth conditions of the cells of the invention can be optimised through routine experimentation. For example, pH, temperature, and concentration of components within the compositions are non-limiting examples of factors which can be optimised. Liquid and/or solid cultures used to grow *C*. *acnes* cells can be housed in any of the culture vessels known and used in the art. In some embodiments, the *C*. *acnes* strains are grown in batches. In some embodiments, the bacterial strains are grown in fermenters. In some embodiments, compositions comprising the bacterial strains are packaged. In certain embodiments, compositions comprising the bacterial strains are packaged in enteral syringes or sachets. In certain embodiments, the bacterial strains may be freeze-dried.

It was previously reported that some strains of *C*. *acnes* are associated with, contribute to or are causative of acne, while other strains show no such connection to acne (Fitz-Gibbon et al. 2013 J Invest Dermatol 133:2152-2160; Lomholt et al. 2010 PLoS ONE 5: e12277). These authors also characterised certain genetic and metabolic determinants of the ability of *C*. *acnes* to contribute to acne. Similarly, WO 2018/073651 described metabolic assays by which pathogenic and non-pathogenic strains of *C*. *acnes* could be identified and selected based on their expression of active linoleic acid isomerase which specifically converts cis-9, cis-12 linoleic acid into trans-10, cis-12 linoleic acid. Accordingly, in certain embodiments, the *C*. *acnes* strain or strains as intended herein are not associated with, do not contribute to and are not causative of acne. Such *C*. *acnes* strains can be readily isolated from healthy human skin (as opposed to human skin affected by acne).

The term "strain" is well-understood as a low-level taxonomic rank used within a species. In microbiology, a strain may be typically defined operatively - a strain is made up of the descendants of a single isolation in pure culture and usually is made up of a succession of cultures ultimately derived from an initial single colony; or alternatively, as an isolate or group of isolates that can be distinguished from other isolates of the same genus and species by phenotypic characteristics or genotypic characteristics or both. As used in the practice of the present invention, the term "strain" may be synonymous with the operative terms "isolate" or "clone".

The term "live" as used herein is synonymous with "viable" and refers to any living intact state of a microorganism, such as active growth or dormancy, from which state it can multiply and/or reproduce itself in a medium capable of supporting the growth of the microorganism. Typically, substantially all *C*. *acnes* bacteria comprised by the compositions intended herein may be live or viable. For example, at least 50%, preferably at least 60%, more preferably at least 75%, still more preferably at least 90%, such as at least 95%, 96%, 97%, 98%, 99% or 100% of the bacteria in the composition are viable, such as capable of forming colonies when plated on a suitable solid medium.

The *C*. *acnes* strain is capable of secreting RoxP. The term "to secrete", or its alternative forms such as "secreting" or "secretion", commonly refers to a movement of a material from a cell interior to a periplasmic space or to an extracellular environment. The term may more particularly denote the translocation of a polypeptide or protein from a cell interior across the plasma membrane, such that the protein can be released as soluble protein to the extracellular environment (cell supernatant).

Holland et al. 2010 (BMC Microbiol 10:230) reported that the protein corresponding to *C*. *acnes* Gene ID PPA1939 (annotated merely as "hypothetical protein, specific to *P. acnes")* was found in the secretome *of P. acnes.* Accordingly to Holland et al., the protein was secreted by *P*. *acnes* phylotypes IA, IB, II, and III, appeared highly expressed by semi-quantitative assessment of Coomassie stained gels, and contained a signal sequence. The protein was annotated under Genbank (http://www.ncbi.nlm.nih.gov/) accession number AAT83657.1. In a subsequent study, Allhorn et al. (2016 Sci Rep 6:36412) showed that the PPA1939 protein is a secreted enzyme displaying antioxidant activity in certain *in vitro* conditions, and renamed the protein RoxP, standing for radical oxygenase of *Propionibacterium acnes.* Allhorn et al. retrieved the RoxP protein sequence from all *P. acnes* genomes available at that moment and showed that RoxP was highly conserved among the vast majority of P. acnes strains, with 58 strains having an identical homolog, 33 strains having highly similar homologs (99% identity), and a subgroup of 11 RoxP homologs from type II and type III strains of *P. acnes* being more distant (83% identity). The authors also taught primers to PCR-amplify and sequence the roxP gene from other isolates (see Table 1 of Allhorn et al.), and deposited the RoxP protein sequences of isolates AD9, AD20, AD24, AD26, AS4, AS8, AS23, AS26, and AS39 under Genbank accession numbers AFJ11206.1, AFJ11207.1, AFJ11208.1, AFJ11209.1, AFJ11210.1, AFJ11211.1, AFJ11212.1, AFJ11213.1, and AFJ11214.1, respectively. By analysing an even larger number of *C*. *acnes* isolates, the present inventors uncovered that the majority (approximately 93 %) of isolates expressed RoxP homologues with 99-100% sequence identity, whereas a subset of strains (approximately 7 %) classified as *C*. *acnes* phylotypes II and III expressed a more distantly related protein, with approximately 83 % amino acid identity. Conveniently, RoxP homologues from various *C*. *acnes* strains can be characterised in terms of overall sequence identity to the RoxP protein from *C. acnes* strain KPA171202 (DSMZ acc. no. DSM-16379).

By means of an example and not limitation, the amino acid sequence of the native RoxP polypeptide from *C*. *acnes* strain KPA171202 is:

MFVQIAASLAAASSIALGIPGAATPIDESQLPVGPQVSVTDSAQHTGPFAASSPLTITVKP
GAPCVRADGYQESMVTRVLDDKGHQVWTGTFDESKLIGGTGLGTATFHVGSPAAAFN
FHGSERTTYRTLSYCAYPHYVNGTRERLSQVSVKTFMVDPALN (SEQ ID NO: 1)

By means of an example and not limitation, the amino acid sequence of the native RoxP polypeptide from *C*. *acnes* strain 266 (showing 99.4% sequence identity to RoxP from KPA171202) is:

MFVQIAASLAAASSIALGIPGAATPIDESQLPVGPQVSVTDSAQHTGPFAASSPLTITVKP
GAPCVRADGYQESMVTRVLDDKGHQVWTGTFDESKLIGGTGLGTATFHVGSPAAAFN
FHGSERTTYRTLSYCAYPHYVNGTRERLSQVSVKTFMVDPTLN (SEQ ID NO: 2)

By means of an example and not limitation, the amino acid sequence of the native RoxP polypeptide from *C*. *acnes* strain PMH-7 (showing 97.5% sequence identity to RoxP from KPA171202) is:

MFVQIAASLAAASSIALGMPGAATPIDESQLPVGPQVSVTDSAQHTGPFAASSPLAITVK
PGAPCVRADGYQESMVTRVLDDKGHQVWTGTFDESKLIGGTGLGTATFHVESPAAAF
NFHGNERTTYRTLSYCAYPHYVNGTRERLSQVSVKTFMVDPALN (SEQ ID NO: 3)

By means of an example and not limitation, the amino acid sequence of the native RoxP polypeptide from *C*. *acnes* strain 09-09 (showing 83.2% sequence identity to RoxP from KPA171202) is:

MFVQIAASLAAASTIALGVPGAATPLNGSQLPTGPQVSVTDPAQHTGPFSTATPLTIKVK
PSAPCVRADGRQQSLVARVLDDKGHQVWSGTFDESQLIGGTGRGTATFHVGSAAAAF
NFHGGKRTTYRALGYCAYPHYVNGTRERLSQVSVKTFTVDPAIN (SEQ ID NO: 4)

By means of an example and not limitation, the amino acid sequence of the native RoxP polypeptide from *C*. *acnes* strain AD24 (showing 83% sequence identity to RoxP from KPA171202) is:

MFVQIAASLAAASTIALGVPGAATPLNGSQLPTGPQVSVTDPAQHTGPFSTATPLTIKVK
PSAPCVRADGRQQSLVARVLDDKGHQVWSGTFDESQLIGGTGRGTATFHVGSAAAAF
NFHGGKRTTYRALGYCAYPHYVNGTRERLSQVSVKTFTVDPAIN (SEQ ID NO: 19)

By means of an example and not limitation, the amino acid sequence of the native RoxP polypeptide from *C*. *acnes* strain 09-109 is identical (i.e., 100% sequence identity) to RoxP from KPA171202 (SEQ ID NO: 1).

The reference to RoxP thus denotes the RoxP polypeptide or protein as commonly known under this designation in the art, of any *C*. *acnes* strain or isolate. The skilled person can appreciate that the above RoxP sequences or RoxP sequences deposited in sequence databases, or RoxP sequencing obtained by sequencing additional *C*. *acnes* strains, can be of the RoxP precursor, including a signal peptide that may or may not be proteolytically removed in the secreted RoxP protein. For example, protein sequence analysis tools predict that amino acids 1 to 22 of RoxP set forth in the above SEQ ID NO: 1 to 4 constitute respective signal peptides. The skilled person can further appreciate that the N-terminal Met residue of RoxP can be post-translationally removed from and thus absent in the secreted RoxP protein. The skilled person will also appreciate that certain post-translational modifications, such as phosphorylation, S-thiolation, hydroxylation, citrullination, or N-glycosylation, may occur in bacterial cells, and that the present disclosure encompasses all RoxP proteins, irrespective of whether they do or do not contain any post-translational modifications.

In certain embodiments where the live *C*. *acnes* strain secreting RoxP is administered, the *C*. *acnes* strain may secrete RoxP endogenously. The terms "endogenous" or "endogenously" commonly refer to a material, such as a polypeptide, protein, or nucleic acid, which is native to or own to a cell, and does not originate from outside of the cell, such as in particular is not introduced into the cell or into a predecessor of the cell by the action of man. For example, an endogenous nucleic acid or gene may be present within the natural genome of the cell, and an endogenous polypeptide or protein may be encoded by the natural genome of the cell. Hence, in such embodiments, the *C*. *acnes* strain secretes RoxP naturally, and need not be and is not genetically engineered to secrete RoxP.

In further embodiments where the live *C*. *acnes* strain secreting RoxP is administered, the *C*. *acnes* strain is not genetically engineered. The term "genetically engineered" broadly refers to a cell that has been subjected to recombinant DNA manipulations, resulting for example in a random or directed alteration of its genome, or in transient or stable introduction of exogenous nucleic acid molecule(s). A genetically engineered cell displays a genetic makeup not found originally in nature. Hence, preferably in such embodiments, not only is the cell not genetically engineered to secrete RoxP, but also does not contain any other man-made genetic alterations.

In certain embodiments, the *C*. *acnes* strain, or the or the two or more *C*. *acnes* strains individually or collectively, comprised by the present cosmetic or pharmaceutical compositions may secrete, at least 1 µg RoxP per mL of medium when measured in stationary phase, such as for example at least 2 µg/mL, preferably at least 5 µg/mL, more preferably at least 8 µg/mL, and most preferably at least 10 µg/mL, such as at least 15 µg/mL, at least 20 µg/mL, at least 25 µg/mL or at least 30 µg/mL (µg RoxP per mL of medium when measured in stationary phase).

To measure the amount of RoxP secreted into the medium, *C*. *acnes* may be cultured in a rich complex medium, such as Trypticase Soy Broth (TSB, available for example from Beckton Dickinson, Franklin Lakes, New Jersey), or Brain Heart Infusion broth (BHI, available for example from Sigma-Aldrich), at 37°C, in anoxic conditions (such as 10% H₂, 10% COz, 80% N₂), until reaching stationary phase, typically for 3-7 days. The supernatant (medium) can be separated from the cells by centrifugation (such as at 3600 g for 10 min).

Any separation, detection and quantification method may be used to measure the quantity of RoxP in the supernatant / medium. Such methods may include densitometric methods, including SDS-PAGE plus Silver or Coomassie staining, biochemical assay methods, including *inter alia* assays of enzymatic activity, immunological assay methods, such as enzyme-linked immunosorbent assay (ELISA) and ELISPOT based techniques, radioimmunoassay (RIA), Western blot, etc., mass spectrometry (MS) analysis methods, such as matrix-assisted laser desorption/ionisation time-of-flight (MALDI-TOF) MS, chromatography methods, such as high-performance liquid chromatography (HPLC), cation or anion exchange chromatography, size exclusion chromatography (SEC), and the like.

The present inventors also extensively characterised naturally-occurring *C*. *acnes* RoxP upstream sequences including RoxP promoters, and identified the existence of at least 8 dominant sequences of the RoxP upstream region, set forth in SEQ ID NO: 5 to 12 in Fig. 8. The inventors further identified native -10 and -35 box sequences that may be advantageous for achieving high endogenous secretion of RoxP by the *C*. *acnes,* and may thus also be employed to select potentially high-expressing *C*. *acnes* strains.

Hence, in certain embodiments, the endogenous RoxP promoter of the *C*. *acnes* strain, or of at least one of the two or more *C*. *acnes* strains, may comprise the nucleic acid sequence as set forth in any one of SEQ ID NO: 5 to 12. In certain further embodiments, the endogenous RoxP promoter of the *C*. *acnes* strain, or of at least one of the two or more *C*. *acnes* strains, may comprise a nucleic acid sequence which is at least about 80%, preferably at least about 85%, more preferably at least about 90%, such as at least about 91%, at least about 92%, at least about 93%, or at least about 94%, and even more preferably at least about 95%, such as at least about 96%, at least about 97%, at least about 98%, or at least about 99% identical (preferably overall sequence identity) to the nucleic acid sequence as set forth in any one of SEQ ID NO: 5 to 12.

In certain embodiments, type I *C*. *acnes* strains may be preferred as comparatively high expressors of endogenous RoxP. Hence, in certain embodiments, the endogenous RoxP promoter of the *C*. *acnes* strain, or of at least one of the two or more *C*. *acnes* strains (particularly wherein the *C*. *acnes* strain or strains is or are type I strains), may comprise the nucleic acid sequence as set forth in SEQ ID NO: 8, or a nucleic acid sequence which is at least about 80%, preferably at least about 85%, more preferably at least about 90%, such as at least about 91%, at least about 92%, at least about 93%, or at least about 94%, and even more preferably at least about 95%, such as at least about 96%, at least about 97%, at least about 98%, or at least about 99% identical (preferably overall sequence identity) to the nucleic acid sequence as set forth in SEQ ID NO: 8. In certain further embodiments, the endogenous RoxP promoter of the *C*. *acnes* strain, or of at least one of the two or more *C*. *acnes* strains (particularly wherein the *C*. *acnes* strain or strains is or are type I strains), may comprise the nucleic acid sequence as set forth in SEQ ID NO: 9, or a nucleic acid sequence which is at least about 80%, preferably at least about 85%, more preferably at least about 90%, such as at least about 91%, at least about 92%, at least about 93%, or at least about 94%, and even more preferably at least about 95%, such as at least about 96%, at least about 97%, at least about 98%, or at least about 99% identical (preferably overall sequence identity) to the nucleic acid sequence as set forth in SEQ ID NO: 9. In certain further embodiments, the endogenous RoxP promoter of the *C*. *acnes* strain, or of at least one of the two or more *C*. *acnes* strains (particularly wherein the *C*. *acnes* strain or strains is or are type I strains), may comprise the nucleic acid sequence as set forth in SEQ ID NO: 10, or a nucleic acid sequence which is at least about 80%, preferably at least about 85%, more preferably at least about 90%, such as at least about 91%, at least about 92%, at least about 93%, or at least about 94%, and even more preferably at least about 95%, such as at least about 96%, at least about 97%, at least about 98%, or at least about 99% identical (preferably overall sequence identity) to the nucleic acid sequence as set forth in SEQ ID NO: 10. In certain further embodiments, the endogenous RoxP promoter of the *C*. *acnes* strain, or of at least one of the two or more *C*. *acnes* strains (particularly wherein the *C*. *acnes* strain or strains is or are type I strains), may comprise the nucleic acid sequence as set forth in SEQ ID NO: 12, or a nucleic acid sequence which is at least about 80%, preferably at least about 85%, more preferably at least about 90%, such as at least about 91%, at least about 92%, at least about 93%, or at least about 94%, and even more preferably at least about 95%, such as at least about 96%, at least about 97%, at least about 98%, or at least about 99% identical (preferably overall sequence identity) to the nucleic acid sequence as set forth in SEQ ID NO: 12.

In certain embodiments, type III C. *acnes* strains may be preferred as comparatively high expressors of endogenous RoxP. Hence, in certain embodiments, the endogenous RoxP promoter of the *C*. *acnes* strain, or of at least one of the two or more *C*. *acnes* strains (particularly wherein the *C*. *acnes* strain or strains is or are type III strains), may comprise the nucleic acid sequence as set forth in SEQ ID NO: 7, or a nucleic acid sequence which is at least about 80%, preferably at least about 85%, more preferably at least about 90%, such as at least about 91%, at least about 92%, at least about 93%, or at least about 94%, and even more preferably at least about 95%, such as at least about 96%, at least about 97%, at least about 98%, or at least about 99% identical (preferably overall sequence identity) to the nucleic acid sequence as set forth in SEQ ID NO: 7.

In certain embodiments, certain type II C. *acnes* strains may be preferred as comparatively high expressors of endogenous RoxP. Hence, in certain embodiments, the endogenous RoxP promoter of the *C*. *acnes* strain, or of at least one of the two or more C. *acnes* strains (particularly wherein the *C*. *acnes* strain or strains is or are type II strains), may comprise the nucleic acid sequence as set forth in SEQ ID NO: 11, or a nucleic acid sequence which is at least about 80%, preferably at least about 85%, more preferably at least about 90%, such as at least about 91%, at least about 92%, at least about 93%, or at least about 94%, and even more preferably at least about 95%, such as at least about 96%, at least about 97%, at least about 98%, or at least about 99% identical (preferably overall sequence identity) to the nucleic acid sequence as set forth in SEQ ID NO: 11.

In still further embodiments, the endogenous RoxP promoter of the C. *acnes* strain, or of at least one of the two or more C. *acnes* strains, may comprise a -10 box having a sequence selected from TGCTATACT or TGCTACACT, preferably TGCTATACT. Such -10 box may be frequently found in comparatively high RoxP-expressing type I (including type IA or IB) C. *acnes* strains, such as for example the strains exemplified in Fig. 8. Such -10 box may also be found in some type II C. *acnes* strains, which may thus be expected to express endogenous RoxP at a comparatively higher level than other type II C. *acnes* strains (see for example the type II strain 09-23 in Table 2 having the RoxP promoter of SEQ ID NO: 11). Such -10 box may further be frequently found in comparatively high RoxP-expressing SLST types A, C, H or K (including K5 and K1) C. *acnes* strains, such as for example the strains shown in **Fig. 8** and **Table 2** (see type II/K1 strain 09-23).

Hence, in certain embodiments, the C. *acnes* strain, or at least one of the two or more C. *acnes* strains, is a type I C. *acnes* strain, such as a type IA or type IB C. *acnes* strain, comprising a -10 box in the endogenous RoxP promoter, said -10 box having a sequence selected from TGCTATACT or TGCTACACT, preferably TGCTATACT. In certain embodiments, the C. *acnes* strain, or at least one of the two or more C. *acnes* strains, is a type IA C. *acnes* strain comprising a -10 box in the endogenous RoxP promoter, said -10 box having the sequence TGCTATACT. In certain embodiments, the C. *acnes* strain, or at least one of the two or more C. *acnes* strains, is a type IB C. *acnes* strain comprising a -10 box in the endogenous RoxP promoter, said -10 box having the sequence TGCTACACT. In certain embodiments, the C. *acnes* strain, or at least one of the two or more C. *acnes* strains, is a type II C. *acnes* strain comprising a -10 box in the endogenous RoxP promoter, said -10 box having the sequence TGCTACACT. In certain embodiments, the C. *acnes* strain, or at least one of the two or more C. *acnes* strains, is a SLST type A, C, H or K (such as K5 or K1) C. *acnes* strain comprising a - 10 box in the endogenous RoxP promoter, said -10 box having a sequence selected from TGCTATACT or TGCTACACT. In certain embodiments, the *C. acnes* strain, or at least one of the two or more C. *acnes* strains, is a SLST type A C. *acnes* strain comprising a -10 box in the endogenous RoxP promoter, said -10 box having the sequence TGCTATACT. In certain embodiments, the C. *acnes* strain, or at least one of the two or more C. *acnes* strains, is a SLST type C C. *acnes* strain comprising a -10 box in the endogenous RoxP promoter, said -10 box having the sequence TGCTATACT. In certain embodiments, the C. *acnes* strain, or at least one of the two or more C. *acnes* strains, is a SLST type H C. *acnes* strain comprising a -10 box in the endogenous RoxP promoter, said -10 box having the sequence TGCTACACT. In certain embodiments, the C. *acnes* strain, or at least one of the two or more C. *acnes* strains, is a SLST type K5 or K1 C. *acnes* strain comprising a -10 box in the endogenous RoxP promoter, said -10 box having the sequence TGCTACACT.

In still further embodiments, the endogenous RoxP promoter of the C. *acnes* strain, or of at least one of the two or more C. *acnes* strains, may comprise a -10 box having the sequence TGCTACGCT. Such -10 box may be frequently found in comparatively high RoxP-expressing type I (preferably type IA) C. *acnes* strains, such as for example the strain 523 exemplified in **Fig. 8****.** Such -10 box may also be found in some type III C. *acnes* strains, which may thus be expected to express endogenous RoxP at a comparatively higher level than other type III C. *acnes* strains. Such -10 box may further be frequently found in comparatively high RoxP-expressing SLST types G or L C. *acnes* strains, such as for example the strains shown in **Fig. 8****.**

Hence, in certain embodiments, the C. *acnes* strain, or at least one of the two or more C. *acnes* strains, is a type I C. *acnes* strain, preferably type IA C. *acnes* strain, comprising a -10 box in the endogenous RoxP promoter, said -10 box having the sequence TGCTACGCT. In certain embodiments, the C. *acnes* strain, or at least one of the two or more C. *acnes* strains, is a type III C. *acnes* strain comprising a -10 box in the endogenous RoxP promoter, said -10 box having the sequence TGCTACGCT. In certain embodiments, the C. *acnes* strain, or at least one of the two or more *C. acnes* strains, is a SLST type L or G C. *acnes* strain comprising a -10 box in the endogenous RoxP promoter, said -10 box having the sequence TGCTACGCT.

In still further embodiments, the endogenous RoxP promoter of the C. *acnes* strain, or of at least one of the two or more C. *acnes* strains, may comprise a -35 box having a sequence selected from ACTTCGAT or ACTTCAAT.

Such -35 box may be frequently found in comparatively high RoxP-expressing type I (including type IA or IB) C. *acnes* strains, such as for example the strains exemplified in **Fig. 8****.** Such -35 box may also be found in some type II C. *acnes* strains, which may thus be expected to express endogenous RoxP at a comparatively higher level than other type II C. *acnes* strains. Such -35 box may further be frequently found in comparatively high RoxP-expressing SLST types A, C, G, H or K (including K5 and K1) C. *acnes* strains, such as for example the strains shown in **Fig.** 8 and **Table 2** (see strain 9-23).

Hence, in certain embodiments, the C. *acnes* strain, or at least one of the two or more C. *acnes* strains, is a type I C. *acnes* strain, such as a type IA or type IB C. *acnes* strain, comprising a -35 box in the endogenous RoxP promoter, said -35 box having a sequence selected from ACTTCGAT or ACTTCAAT. In certain embodiments, the C. *acnes* strain, or at least one of the two or more C. *acnes* strains, is a type IA C. *acnes* strain comprising a -35 box in the endogenous RoxP promoter, said -35 box having a sequence selected from ACTTCGAT or ACTTCAAT. In certain embodiments, the C. *acnes* strain, or at least one of the two or more C. *acnes* strains, is a type IB C. *acnes* strain comprising a -35 box in the endogenous RoxP promoter, said -35 box having the sequence ACTTCGAT. In certain embodiments, the C. *acnes* strain, or at least one of the two or more C. *acnes* strains, is a type II C. *acnes* strain comprising a -35 box in the endogenous RoxP promoter, said -35 box having the sequence ACTTCGAT. In certain embodiments, the C. *acnes* strain, or at least one of the two or more C. *acnes* strains, is a SLST type A, C, G, H or K (such as K5 and K1) C. *acnes* strain comprising a -35 box in the endogenous RoxP promoter, said -35 box having a sequence selected from ACTTCGAT or ACTTCAAT. In certain embodiments, the C. *acnes* strain, or at least one of the two or more C. *acnes* strains, is a SLST type A, C, H, K5 or K1 *C. acnes* strain comprising a -35 box in the endogenous RoxP promoter, said -35 box having the sequence ACTTCGAT. In certain embodiments, the C. *acnes* strain, or at least one of the two or more C. *acnes* strains, is a SLST type G C. *acnes* strain comprising a -35 box in the endogenous RoxP promoter, said -35 box having the sequence ACTTCAAT.

In still further embodiments, the endogenous RoxP promoter of the C. *acnes* strain, or of at least one of the two or more C. *acnes* strains, may comprise a -10 box having a sequence selected from TGCTATACT or TGCTACACT, preferably TGCTATACT, and a -35 box having a sequence selected from ACTTCGAT or ACTTCAAT. Such configurations may be particularly advantageous as they can provide for high endogenous RoxP expression and secretion.

Hence, in certain embodiments, the C. *acnes* strain, or at least one of the two or more C. *acnes* strains, is a type I C. *acnes* strain, such as a type IA or type IB C. *acnes* strain, comprising a -10 box and a -35 box in the endogenous RoxP promoter, said -10 box having a sequence selected from TGCTATACT or TGCTACACT, preferably TGCTATACT, and said -35 box having a sequence selected from ACTTCGAT or ACTTCAAT. In certain embodiments, the C. *acnes* strain, or at least one of the two or more C. *acnes* strains, is a type IA C. *acnes* strain comprising a -10 box and a -35 box in the endogenous RoxP promoter, said -10 box having the sequence TGCTATACT, and said -35 box having a sequence selected from ACTTCGAT or ACTTCAAT. In certain embodiments, the C. *acnes* strain, or at least one of the two or more C. *acnes* strains, is a type IB C. *acnes* strain comprising a -10 box and a -35 box in the endogenous RoxP promoter, said -10 box having the sequence TGCTACACT and said -35 box having the sequence ACTTCGAT. In certain embodiments, the C. *acnes* strain, or at least one of the two or more C. *acnes* strains, is a type II C. *acnes* strain comprising a -10 box and a -35 box in the endogenous RoxP promoter, said -10 box having the sequence TGCTACACT and said -35 box having the sequence ACTTCGAT. In certain embodiments, the C. *acnes* strain, or at least one of the two or more C. *acnes* strains, is a SLST type A, C, H or K (such as K5 or K1) C. *acnes* strain comprising a -10 box and a -35 box in the endogenous RoxP promoter, said -10 box having a sequence selected from TGCTATACT or TGCTACACT and said -35 box having a sequence selected from ACTTCGAT or ACTTCAAT. In certain embodiments, the C. *acnes* strain, or at least one of the two or more C. *acnes* strains, is a SLST type G C. *acnes* strain comprising a -10 box and a -35 box in the endogenous RoxP promoter, said -10 box having the sequence TGCTACGCT and said -35 box having the sequence ACTTCAAT. In certain embodiments, the C. *acnes* strain, or at least one of the two or more C. *acnes* strains, is a SLST type A C. *acnes* strain comprising a -10 box and a -35 box in the endogenous RoxP promoter, said -10 box having the sequence TGCTATACT and said -35 box having the sequence ACTTCGAT. In certain embodiments, the C. *acnes* strain, or at least one of the two or more C. *acnes* strains, is a SLST type C C. *acnes* strain comprising a -10 box and a -35 box in the endogenous RoxP promoter, said -10 box having the sequence TGCTATACT and said -35 box having the sequence ACTTCGAT. In certain embodiments, the C. *acnes* strain, or at least one of the two or more C. *acnes* strains, is a SLST type H C. *acnes* strain comprising a -10 box and a -35 box in the endogenous RoxP promoter, said -10 box having the sequence TGCTACACT and said -35 box having the sequence ACTTCGAT. In certain embodiments, the C. *acnes* strain, or at least one of the two or more C. *acnes* strains, is a SLST type K5 or K1 C. *acnes* strain comprising a -10 box and a -35 box in the endogenous RoxP promoter, said -10 box having the sequence TGCTACACT and said -35 box having the sequence ACTTCGAT.

The C. *acnes* strain or strains comprised by the present cosmetic or pharmaceutical compositions may be of any type. In certain embodiments, the C. *acnes* strain, or at least one of the two or more C. *acnes* strains each independently, comprised by the present cosmetic or pharmaceutical compositions may be a type I, II or III C. *acnes.* The present inventors uncovered that type I, including subtype IA and IB, C. *acnes* strains display an advantageously high endogenous secretion of RoxP. Hence, in certain preferred embodiments, the C. *acnes* strain, or at least one of the two or more C. *acnes* strains each independently, may be type I, such as type IA or type IB. Type IA strains may be particularly preferred. RoxP secretion by type III strains was also comparatively high. Hence, in certain preferred embodiments, the C. *acnes* strain, or at least one of the two or more C. *acnes* strains each independently, may be type III. In certain preferred embodiments, the C. *acnes* strain, or at least one of the two or more C. *acnes* strains each independently, may be type I or III. In certain preferred embodiments, the C. *acnes* strain, or at least one of the two or more C. *acnes* strains each independently, may be type IA, IB or III. Some type II strains also show adequately high secretion of RoxP, while majority of type II strains displays RoxP secretion lower than that of types I and III. By means of example, Table 2 in Example 1 shows that the type II strain 09-23 expresses RoxP at level comparable to or even higher than many type I and III strains. Hence, in certain embodiments, the C. *acnes* strain, or at least one of the two or more C. *acnes* strains each independently, may be type II. High RoxP-expressing C. *acnes* strains type II may be particularly advantageous herein, since type II strains have been typically reported as not associated with acne, i.e., associated with normal or healthy skin. Hence, in certain embodiments, the C. *acnes* strain, or at least one of the two or more C. *acnes* strains each independently, may be SLST type K1, particularly type K1 comprising endogenous RoxP promoter as shown in SEQ ID NO: 11. In certain further embodiments, the C. *acnes* strain, or at least one of the two or more C. *acnes* strains each independently, may be type IA, IB, II or III.

The C. *acnes* strain or strains comprised by the present cosmetic or pharmaceutical compositions may be of any SLST type. The present inventors further realised that some SLST types may be preferred, for example because of their high secretion of RoxP and/or other characteristics (e.g., not being associated with acne). Hence, in certain embodiments, the C. *acnes* strain, or at least one of the two or more C. *acnes* strains each independently, comprised by the present cosmetic or pharmaceutical compositions may be SLST type A, C, G, H, L or K (such as K5 or K1). SLST types A or C including type IA strains may be particularly preferred.

In certain embodiments, the C. *acnes* strain or strains may be selected from the group comprising, consisting essentially of or consisting of any one of the strains shown in the drawings, such as PM H5, 09-09, ATCC11828, 523, PM H7, 12.1.R1, 12.1.L1, KPA171202, 15.1.R1, 09-23, 266, 21.1.L1, 09-323, 09-109, 11-79, 30.2.L1, 10-43, AD24, and combinations thereof.

In certain embodiments, the C. *acnes* strain or strains comprised by the present cosmetic or pharmaceutical compositions may grow equally well under aerobic and anaerobic conditions.

The C. *acnes* strain, or the or the two or more C. *acnes* strains individually or collectively, comprised by the present cosmetic or pharmaceutical compositions may be present in the composition at any amount compatible with the desired cosmetic, prophylactic or therapeutic effect of the compositions. In certain embodiments, the C. *acnes* strain is, or the two or more C. acnes strains individually or collectively are, present in the composition at an amount of at least 100 colony forming units (CFU) per gram of the composition. In other embodiments, the C. *acnes* strain is, or the two or more C. *acnes* strains individually or collectively are, present in the composition at an amount of at least 200, at least 500, at least 1000, at least 5000, at least 1×10⁴, at least 5×10⁴, at least 1×10⁵, at least 5×10⁵, or at least at least 1×10⁶ CFU per gram of the composition. In other embodiments, the C. *acnes* strain is, or the two or more C. *acnes* strains individually or collectively are, present in the composition at an amount of less than 1×10⁴, or less than 1×10³, or less than 500, or less than 200 CFU per gram of the composition.

In certain embodiments where RoxP is administered, the protein or polypeptide may be isolated or purified from a naturally-occurring source of RoxP, such as from a RoxP-expressing C. *acnes* strain, or may be produced recombinantly by a suitable host cell or host organism expression system and isolated therefrom, or may be produced recombinantly by cell-free transcription or translation, or by non-biological peptide synthesis. Hence, the reference to RoxP encompasses naturally, recombinantly, semi-synthetically or synthetically produced proteins.

The term "purified" in this context does not require absolute purity. Instead, it denotes that a protein or polypeptide is in a discrete environment in which its abundance (conveniently expressed in terms of mass or weight or concentration) relative to other components is greater than in the original material. A discrete environment denotes a single medium, such as for example a single solution, gel, precipitate, lyophilisate, etc. Purified proteins or polypeptides may preferably constitute by weight ≥ 10%, more preferably ≥ 50%, such as ≥ 60%, yet more preferably ≥ 70%, such as ≥ 80%, and still more preferably ≥ 90%, such as ≥ 95%, ≥ 96%, ≥ 97%, ≥ 98%, ≥ 99% or even 100%, of the protein content of the discrete environment. Protein content may be determined, e.g., by the Lowry method (Lowry et al. 1951 J Biol Chem 193:265), optionally as described by Hartree 1972 Anal Biochem 48:422-427. Purity of peptides, polypeptides, or proteins may be determined by SDS-PAGE under reducing or nonreducing conditions using Coomassie blue or, preferably, silver stain.

Illustrative but non-limiting ways to isolate or purify native RoxP secreted by C. *acnes* are set forth in the Examples section. For example, the methodology of Allhorn et al. 2016 *(supra)* may be adopted. Therein, a combination of ammonium sulphate precipitation, a two-step ionexchange chromatography (anionic and cationic) and size exclusion was employed to purify RoxP from C. *acnes* supernatant to approximately 95% homogeneity. More specifically, in an embodiment of such purification method, C. *acnes* grown to stationary phase is collected by centrifugation at 3600 g for 10 min; the culture supernatant is sterile filtered (0.22 µm) and precipitated with 50% ammonium sulphate; the pellet is resolved in 10 mM Tris-HCl pH 8.8 and dialyzed overnight (MWCO 3,500); the sample is loaded on an equilibrated HiTrap Q FF column (GE Healthcare, Uppsala, Sweden), and eluted with 50 mM NaCl (10 mM Tris-HCl pH 8.8); a second dialysis is performed (50 mM NaOAc-HOAc pH 5.0); samples are run on a HiTrap SP FF column (GE Healthcare), and eluted with 100 mM NaCl (50 mM NaOAc-HOAc pH 5.0). Finally, fractions are run through a 50 kDa MWCO-filter (Amicon Ultra, Ultracel-50K). In another example, a combination of ammonium sulphate precipitation of C. *acnes* supernatant and purification on a cryogel column can be employed. In another example, C. *acnes* supernatant can be concentrated by Tangential flow filtrations, using a 30kDa cut-off in a first step and a 3kDa cut-off in a second step.

Accordingly, in certain embodiments, the composition may comprise, consist essentially of or consist of the supernatant of a cultured live C. *acnes* strain secreting RoxP, preferably endogenously secreting RoxP. In certain other embodiments, the composition may comprise, consist essentially of or consist of a RoxP-enriched fraction of the supernatant of a cultured live *C. acnes* strain secreting RoxP, preferably endogenously secreting RoxP. The term "Rox-P enriched fraction" of the supernatant denotes a fraction or a discrete environment obtained by processing the supernatant, in which the abundance of RoxP is increased compared to the abundance of RoxP in the supernatant. For example, RoxP may represent ≥ 50%, such as ≥ 60%, yet more preferably ≥ 70%, such as ≥ 80%, and still more preferably ≥ 90%, such as ≥ 95%, ≥ 96%, ≥ 97%, ≥ 98%, ≥ 99% or even 100%, of the protein content of the enriched fraction.

In certain embodiments, RoxP may be produced recombinantly by a suitable host cell or host organism expression system and isolated therefrom. To this end, an expression cassette or an expression vector is constructed comprising a nucleic acid molecule comprising a nucleic acid sequence encoding RoxP (optionally codon-optimised for expression in a particular host) and a promoter operably linked to the nucleic acid molecule. The expression cassette or expression vector is then introduced into suitable host cells or a host organism which allows for the expression and preferably secretion of RoxP. Nucleic acid sequences encoding RoxP are readily available. For example, the nucleic acid encoding RoxP from C. acnes isolate AD26, which shows only one mismatch at amino acid level with native RoxP polypeptide from C. *acnes* strain KPA171202 as shown in SEQ ID NO: 1, is annotated under Genbank accession number JN051668.1. Further, Allhorn et al. (2016 Sci Rep 6:36412) taught primers to PCR-amplify and isolate nucleic acid sequence of the *roxP* gene broadly from various C. *acnes* isolates.

As used herein, the term "promoter" refers to a DNA sequence that enables a gene to be transcribed. A promoter is recognised by RNA polymerase, which then initiates transcription. Thus, a promoter contains a DNA sequence that is either bound directly by, or is involved in the recruitment, of RNA polymerase. A promoter sequence can also include "enhancer regions", which are one or more regions of DNA that can be bound with proteins (namely the trans-acting factors) to enhance transcription levels of genes in a gene-cluster. The enhancer, while typically at the 5' end of a coding region, can also be separate from a promoter sequence, e.g., can be within an intronic region of a gene or 3' to the coding region of the gene.

An "operable linkage" is a linkage in which regulatory sequences and sequences sought to be expressed are connected in such a way as to permit said expression. For example, sequences, such as, e.g., a promoter and an ORF, may be said to be operably linked if the nature of the linkage between said sequences does not: (1) result in the introduction of a frame-shift mutation, (2) interfere with the ability of the promoter to direct the transcription of the ORF, (3) interfere with the ability of the ORF to be transcribed from the promoter sequence. Hence, "operably linked" may mean incorporated into a genetic construct so that expression control sequences, such as a promoter, effectively control expression of a coding sequence of interest.

The promotor may be a constitutive or inducible (conditional) promoter. A constitutive promoter is understood to be a promoter whose expression is constant under the standard culturing conditions. Inducible promoters are promoters that are responsive to one or more induction cues. For example, an inducible promoter can be chemically regulated (e.g., a promoter whose transcriptional activity is regulated by the presence or absence of a chemical inducing agent such as an alcohol, tetracycline, a steroid, a metal, or other small molecule) or physically regulated (e.g., a promoter whose transcriptional activity is regulated by the presence or absence of a physical inducer such as light or high or low temperatures). An inducible promoter can also be indirectly regulated by one or more transcription factors that are themselves directly regulated by chemical or physical cues.

The host cell may be a bacterial cell, a fungal cell, including yeast cells, an animal cell, or a mammalian cell, including human cells and non-human mammalian cells. The host organism may for example be a plant organism whereby the secretion of RoxP is directed to the plant as a whole or to a particular part of the plant such as to leaves; or may be a lactating non-human mammal, whereby the secretion of RoxP is directed to the product of the mammary gland of the mammal.

Expression systems (host cells) that can be used for small or large scale production of polypeptides include, without limitation, microorganisms such as bacteria (e.g., *Escherichia. coli, Yersinia enterocolitica, Brucella* sp., *Salmonella tymphimurium, Serratia marcescens,* or *Bacillus subtilis),* for example transformed with recombinant bacteriophage DNA, plasmid DNA, or cosmid DNA expression vectors; or fungal cells (e.g., *Yarrowia lipolytica, Arxula adeninivorans,* methylotrophic yeast (e.g., methylotrophic yeast of the genus *Candida, Hansenula, Oogataea, Pichia* or *Torulopsis,* e.g., *Pichia pastoris, Hansenula polymorpha, Ogataea minuta,* or *Pichia methanolica),* or filamentous fungi of the genus *Aspergillus, Trichoderma, Neurospora, Fusarium,* or *Chrysosporium,* e.g., *Aspergillus niger, Trichoderma reesei,* or yeast of the genus *Saccharomyces or Schizosaccharomyces,* e.g., *Saccharomyces cerevisiae,* or *Schizosaccharomyces pombe),* for example transformed with recombinant fungal expression vectors. Useful expression systems also include insect cell systems (e.g., cells derived from *Drosophila melanogaster,* such as Schneider 2 cells, cell lines derived from the army worm *Spodoptera frugiperda,* such as Sf9 and Sf21 cells, or cells derived from the cabbage looper *Trichoplusia ni,* such as High Five cells) infected with recombinant virus expression vectors (e.g., baculovirus) containing the nucleic acid molecules, and plant cell systems infected with recombinant virus expression vectors (e.g., tobacco mosaic virus) or transformed with recombinant plasmid expression vectors (e.g., Ti plasmid). Mammalian expression systems include human and non-human mammalian cells, such as rodent cells, primate cells, or human cells. Mammalian cells, such as human or non-human mammalian cells, may include primary cells, secondary, tertiary etc. cells, or may include immortalised cell lines, including clonal cell lines. Preferred animal cells can be readily maintained and transformed in tissue culture. Non-limiting example of human cells include the human HeLa (cervical cancer) cell line. Other human cell lines common in tissue culture practice include *inter alia* human embryonic kidney 293 cells (HEK cells), DU145 (prostate cancer), Lncap (prostate cancer), MCF-7 (breast cancer), MDA-MB-438 (breast cancer), PC3 (prostate cancer), T47D (breast cancer), THP-1 (acute myeloid leukemia), U87 (glioblastoma), SHSY5Y (neuroblastoma), or Saos-2 cells (bone cancer). A non-limiting example of primate cells are Vero (African green monkey Chlorocebus kidney epithelial cell line) cells, and COS cells. Non-limiting examples of rodent cells are rat GH3 (pituitary tumor), CHO (Chinese hamster ovary), PC12 (pheochromocytoma) cell lines, or mouse MC3T3 (embryonic calvarium) cell line. Such cells, prior to the genetic engineering as specified herein, can be obtained from a variety of commercial sources and research resource facilities, such as, for example, the American Type Culture Collection (Rockville, MD). Various promoters may be suitable for expression in mammalian cells, e.g., the metallothionein promoter, the adenovirus late promoter, or the cytomegalovirus promoter. Such tools and methods for heterologous protein expression are generally well known in the art. Bacterial host expression systems, such as *E*. *coli,* or yeast expression systems, such as *Pichia pastoris,* may be preferred. Depending on the expression system, or on chemical or enzymatic post-expression manipulation, RoxP may carry one or more co- or post-expression-type modifications of the polypeptide chain, such as, without limitation, glycosylation, acetylation, phosphorylation, sulphonation, methylation, ubiquitination, signal peptide removal, N-terminal Met removal, etc. Non-naturally occurring post-translational modifications are also foreseen.

The term "variant" of RoxP encompasses proteins or polypeptides the amino acid sequence of which is substantially identical (i.e., largely but not wholly identical) to the amino acid sequence of RoxP, such as to the amino acid sequence of a native or wild-type *C*. *acnes* RoxP, for example at least about 80% identical or at least about 83% identical or at least about 85% identical, e.g., preferably at least about 90% identical, e.g., at least 91% identical, at least 92% identical, more preferably at least about 93% identical, e.g., at least 94% identical, even more preferably at least about 95% identical, e.g., at least 96% identical, yet more preferably at least about 97% identical, e.g., at least 98% identical, and most preferably at least 99% identical. Preferably, a variant may display such degrees of identity to RoxP when the whole sequence of the variant and RoxP are queried in the sequence alignment (i.e., overall sequence identity). Further preferably, the variant may display at least about 80%, or at least about 83%, or at least about 85%, or at least about 90%, or at least about 91%, or at least about 92%, or at least about 93%, or at least about 94%, or at least about 95%, or at least about 96%, or at least about 97%, or at least about 98%, or at least about 99% sequence identity to RoxP of C. *acnes* strain KPA171202 as set forth in SEQ ID NO: 1 above, or to the portion of said RoxP of SEQ ID NO: 1 lacking the N-terminal signal peptide.

Sequence identity between proteins or polypeptides or nucleic acids as envisaged herein may be determined using suitable algorithms for performing sequence alignments and determination of sequence identity as know *per se.* Exemplary but non-limiting algorithms include those based on the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250), for example using the published default settings or other suitable settings (such as, e.g., for the BLASTN algorithm: cost to open a gap = 5, cost to extend a gap = 2, penalty for a mismatch = -2, reward for a match = 1, gap x_dropoff = 50, expectation value = 10.0, word size = 28; or for the BLASTP algorithm: matrix = Blosum62 (Henikoff et al., 1992, Proc. Natl. Acad. Sci., 89:10915-10919), cost to open a gap = 11, cost to extend a gap = 1, expectation value = 10.0, word size = 3). Sequence identity as envisaged herein may preferably denote overall sequence identity, i.e., sequence identity calculated from aligning the whole sequences of the compared proteins, polypeptides or nucleic acids. An example procedure to determine the percent identity between a particular amino acid sequence and the amino acid sequence of a query polypeptide will entail aligning the two amino acid sequences using the Blast 2 sequences (B12seq) algorithm, available as a web application or as a standalone executable programme (BLAST version 2.2.31+) at the NCBI web site (www.ncbi.nlm.nih.gov), using suitable algorithm parameters. An example of suitable algorithm parameters include: matrix = Blosum62, cost to open a gap = 11, cost to extend a gap = 1, expectation value = 10.0, word size = 3). If the two compared sequences share homology, then the output will present those regions of homology as aligned sequences. If the two compared sequences do not share homology, then the output will not present aligned sequences. Once aligned, the number of matches will be determined by counting the number of positions where an identical amino acid residue is presented in both sequences. The percent identity is determined by dividing the number of matches by the length of the query polypeptide, followed by multiplying the resulting value by 100. The percent identity value may, but need not, be rounded to the nearest tenth. For example, 78.11, 78.12, 78.13, and 78.14 may be rounded down to 78.1, while 78.15, 78.16, 78.17, 78.18, and 78.19 may be rounded up to 78.2. It is further noted that the detailed view for each segment of alignment as outputted by B12seq already conveniently includes the percentage of identities.

A variant of RoxP may but need not be a homologue (e.g., orthologue or paralogue) of RoxP. As used herein, the term "homology" generally denotes structural similarity between two macromolecules from same or different taxons, wherein said similarity is due to shared ancestry.

A variant of RoxP may comprise one or more amino acid additions, deletions, or substitutions relative to (i.e., compared with) the corresponding RoxP protein or polypeptide.

For example, a variant (substitution variant) of RoxP may comprise up to 30 (e.g., not more than one, two, three, four, five, six, seven, eight, nine, ten, 12, 15, 20, 25, or 30) conservative amino acid substitutions relative to (i.e., compared with) the corresponding RoxP protein or polypeptide; or a variant (substitution variant) of RoxP may comprise up to 30 (e.g., not more than one, two, three, four, five, six, seven, eight, nine, ten, 12, 15, 20, 25, or 30) non-conservative amino acid substitutions relative to the corresponding RoxP protein or polypeptide; or a variant (substitution variant) of RoxP may comprise up to 30 (e.g., not more than one, two, three, four, five, six, seven, eight, nine, ten, 12, 15, 20, 25, or 30) conservative and non-conservative amino acid substitutions combined relative to the corresponding RoxP protein or polypeptide. A conservative amino acid substitution is a substitution of one amino acid for another with similar characteristics. Conservative amino acid substitutions include substitutions within the following groups: valine, alanine and glycine; leucine, valine, and isoleucine; aspartic acid and glutamic acid; asparagine and glutamine; serine, cysteine, and threonine; lysine and arginine; and phenylalanine and tyrosine. The nonpolar hydrophobic amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (i.e., basic) amino acids include arginine, lysine and histidine. The negatively charged (i.e., acidic) amino acids include aspartic acid and glutamic acid. Any substitution of one member of the above-mentioned polar, basic, or acidic groups by another member of the same group can be deemed a conservative substitution. By contrast, a non-conservative substitution is a substitution of one amino acid for another with dissimilar characteristics.

Alternatively or in addition, a variant (deletion variant) of RoxP may lack up to 30 (e.g., not more than one, two, three, four, five, six, seven, eight, nine, ten, 12, 15, 20, 25, or 30) amino acids relative to the corresponding RoxP protein or polypeptide. When two or more amino acid are deleted, these may non-contiguous or two or more or all of the deleted amino acids may be contiguous in the corresponding RoxP protein or polypeptide.

The term "fragment" of RoxP typically refers to N-terminally and/or C-terminally deleted or truncated forms of RoxP protein or polypeptide. The term encompasses fragments arising by any mechanism, such as, without limitation, by heterologous expression of a truncated form of the full-length RoxP protein or polypeptide, or by physical, chemical or enzymatic proteolysis of the full length RoxP protein or polypeptide *in vivo* or *in vitro.* Without limitation, a fragment of RoxP may represent at least about 50% (by amino acid number), e.g., at least about 60%, or at least about 70%, or at least about 80%, or at least about 90%, or at least about 91%, or at least about 92%, or at least about 93%, or at least about 94%, or at least about 95%, or at least about 96%, or at least about 97%, or at least about 98%, or at least about 99% of the contiguous amino acid sequence of said RoxP protein or polypeptide. For example, a fragment of RoxP may include a sequence of at least about 80, or at least about 90, or at least about 100, or at least about 110, or at least about 120, or at least about 130, or at least about 140, or at least about 150, or at least about 155 consecutive amino acids of the corresponding full-length RoxP protein or polypeptide. In certain embodiments, a fragment of RoxP may be N-terminally and/or C-terminally truncated by between 1 and about 20 amino acids, such as by between 1 and about 15 amino acids, or by between 1 and about 10 amino acids, or by between 1 and about 5 amino acids, compared with the corresponding full-length RoxP protein or polypeptide.

In certain embodiments, a fragment of RoxP may comprise, consist essentially of or consist of a contiguous stretch of amino acids corresponding to positions 66-83 of the native RoxP protein shown in SEQ ID NO: 1. For example, a fragment of RoxP may include the contiguous stretch of amino acids corresponding to positions 66-83 of the native RoxP protein shown in SEQ ID NO: 1 and may further include: a) 1-5 or 6-10 or 11-15 or 16-20 or 21-30 or 31-50 contiguous amino acids immediately N-terminally adjacent to said stretch in the RoxP protein and/or b) 1-5 or 6-10 or 11-15 or 16-20 or 21-30 or 31-50 contiguous amino acids immediately C-terminally adjacent to said stretch in the RoxP protein. For example, a fragment of RoxP may comprise, consist essentially of or consist of the contiguous stretch of amino acids VRADGYQESMVTRVLDDK (SEQ ID NO: 20). For example, a fragment of RoxP may be the peptide denoted herein as "RoxP-Peptide 1" the amino acid sequence of which is as set forth in SEQ ID NO: 20. Notably, RoxP-Peptide 1 was shown to display antioxidant activity.

In certain embodiments, a fragment of RoxP may comprise, consist essentially of or consist of a contiguous stretch of amino acids corresponding to positions 128-140 of the native RoxP protein shown in SEQ ID NO: 1. For example, a fragment of RoxP may include the contiguous stretch of amino acids corresponding to positions 128-140 of the native RoxP protein shown in SEQ ID NO: 1 and may further include: a) 1-5 or 6-10 or 11-15 or 16-20 or 21-30 or 31-50 contiguous amino acids immediately N-terminally adjacent to said stretch in the RoxP protein and/or b) 1-5 or 6-10 or 11-15 or 16-20 or 21 contiguous amino acids immediately C-terminally adjacent to said stretch in the RoxP protein. For example, a fragment of RoxP may comprise, consist essentially of or consist of the contiguous stretch of amino acids RTLSYCAYPHYVN (SEQ ID NO: 21). For example, a fragment of RoxP may be the peptide denoted herein as "RoxP-Peptide 2" the amino acid sequence of which is as set forth in SEQ ID NO: 21. Notably, RoxP-Peptide 2 was shown to display antioxidant activity.

Reference to "fragment or variant" or "variant or fragment" of any protein or polypeptide encompasses fragments of variants of such protein or polypeptide, and variants of fragments of such protein or polypeptide.

The term "biologically active" is interchangeable with terms such as "functionally active" or "functional", denoting that the fragment and/or variant at least partly retains the biological activity or intended functionality of the corresponding RoxP protein or polypeptide. Reference to the "activity" of a protein or polypeptide, or peptide may generally encompass any one or more aspects of the biological activity of the protein or polypeptide, such as without limitation any one or more aspects of its biochemical activity, enzymatic activity, signalling activity, interaction activity, ligand activity, and/or structural activity, e.g., within a cell, tissue, organ or an organism. By means of a preferred example, a biologically active fragment or variant of RoxP shall at least partly retain the antioxidant activity of the corresponding RoxP protein or polypeptide.

Preferably, a functionally active fragment or variant of RoxP may retain at least about 20%, e.g., at least about 25%, or at least 30%, or at least about 40%, or at least about 50%, e.g., at least 60%, more preferably at least about 70%, e.g., at least 80%, yet more preferably at least about 85%, still more preferably at least about 90%, and most preferably at least about 95% or even about 100% of the antioxidant activity or functionality compared with the corresponding RoxP protein or polypeptide. In certain embodiments, a functionally active fragment or variant of RoxP may even display higher antioxidant activity or functionality compared with the corresponding RoxP protein or polypeptide, for example may display at least about 100%, or at least about 150%, or at least about 200%, or at least about 300%, or at least about 400%, or at least about 500% of the antioxidant activity or functionality compared with the corresponding RoxP protein or polypeptide. By means of an example, where the antioxidant activity can be readily measured in an assay with a quantitative output or signal, a functionally active fragment or variant of RoxP may produce a signal which is at least about 20%, or at least about 25%, or at least 30%, or at least about 40%, or at least about 50%, or at least 60%, more preferably at least about 70%, or at least 80%, or at least about 85%, or at least about 90%, or at least about 95%, or at least about 100%, or at least about 150%, or at least about 200%, or at least about 300%, or at least about 400%, or at least about 500% of the signal produced by the corresponding RoxP protein or polypeptide. Representative but non-limiting quantitative tests of antioxidant activity are described in the examples, such as in Examples 2 and 3 (ABTS radical cation assay), Example 4 (protection of human cells *in vitro* from paraquat-caused oxidative damage), and Examples 7-11 (protection of reconstituted human epidermis from UV or ozone-caused oxidative damage), and may be used in this context. The ABTS radical cation assay may be particularly straightforward and may be preferred.

RoxP may be comprised by the present cosmetic or pharmaceutical compositions at any amount or quantity compatible with the desired cosmetic, prophylactic or therapeutic effect of the compositions. In some embodiments, particularly the pharmaceutical compositions may comprise at least 1×10⁻⁹ M RoxP, such as for example at least at least 1×10^{-g} M, at least 5×10⁻⁸ M, at least 1×10⁻⁷ M, or at least 5×10⁻⁷ M, preferably at least 1×10⁻⁶ M, at least 5×10⁻⁶ M, at least 1×10⁻⁵ M, or at least 5×10⁻⁵ M, and more preferably at least 1×10⁻⁴ M, such as at least 2×10⁻⁴ M, at least 3×10⁻⁴ M, at least 4×10⁻⁴ M, at least 5×10⁻⁴ M, at least 6×10⁻⁴ M, at least 7×10⁻⁴ M, at least 8×10⁻⁴ M, at least 9×10⁻⁴ M, or at least 1×10⁻³ M RoxP. For example, the pharmaceutical compositions may comprise between 1×10⁻⁹ M and 1×10⁻³ M, or between 1×10⁻⁷ M and 1×10⁻³ M, or between 1×10⁻⁶ M and 1×10⁻³ M, or between 1×10⁻⁵ M and 1×10⁻³ M, or between 1×10⁻⁴ M and 1×10⁻³ M RoxP. Whereas the cosmetic compositions may comprise comparable quantities of RoxP, substantially lower quantities may meet the cosmetic expectations of the compositions. Hence, in some embodiments, particularly the cosmetic compositions may comprise 1×10⁻⁹ M or less RoxP, such as 5×10⁻¹⁰ M or less, 1×10⁻¹¹ M or less, 5×10⁻¹² M or less, or 1×10⁻¹³ M or less RoxP.

In certain embodiments, the cosmetic or pharmaceutical composition comprising one or more live C. *acnes* strains secreting RoxP as taught herein, may further comprise RoxP as taught herein. Such compositions will allow for co-administration of both actives as disclosed herein. In certain embodiments, the cosmetic or pharmaceutical composition comprising one or more live C. *acnes* strains secreting RoxP as taught herein, and the cosmetic or pharmaceutical composition comprising RoxP, may be provided as a combined product or kit of parts for simultaneous, separate or sequential (in any order) administration of the respective actives.

As used throughout this specification, the terms "therapy" or "treatment" refer to the alleviation or measurable lessening of one or more symptoms or measurable markers of a pathological condition such as a disease or disorder. Measurable lessening includes any statistically significant decline in a measurable symptom or marker. Generally, the terms encompass both curative treatments and treatments directed to reduce symptoms and/or slow progression of the disease. The terms encompass both the therapeutic treatment of an already developed pathological condition, as well as prophylactic or preventative measures, wherein the aim is to prevent or lessen the chances of incidence of a pathological condition. Beneficial or desired clinical results include, but are not limited to, prevention of a disease, reduction of the incidence of a disease, alleviation of symptoms associated with a disease, diminishment of extent of a disease, stabilisation of the disease, delay or slowing of the progression of a disease, amelioration or palliation of a disease, or combinations thereof. In certain embodiments, the terms may relate to therapeutic treatments. In certain other embodiments, the terms may relate to preventative treatments. Treatment of a chronic pathological condition during the period of remission may also be deemed to constitute a therapeutic treatment. The term may encompass *ex vivo* or *in vivo* treatments as appropriate in the context of the present invention.

The term "subject" typically and preferably denotes humans, but may also encompass reference to non-human animals, preferably warm-blooded animals, even more preferably mammals, such as, e.g., non-human primates, rodents, canines, felines, equines, ovines, porcines, etc. The term "non-human animals" includes all vertebrates, e.g.,"oxidativ mammals, such as non-human primates, particularly higher primates, sheep, dogs, rodents (e.g., mice or rats), guinea pigs, goats, pigs, cats, rabbits, cows, and non-mammals such as chickens, amphibians, reptiles etc. In certain embodiments, the subject is a non-human animal. In certain embodiments, the subject is a non-human mammal. In certain embodiments, the subject is a transgenic non-human animal or non-human mammal. In preferred embodiments, the subject is human. In other embodiments, the subject is an experimental animal or animal disease model. The term does not denote a particular age or sex, and includes *inter alia* newborns, children, adolescents, and adults including the elderly, whether male or female.

In aspects and embodiments relating to therapeutic or prophylactic interventions the terms "subject" and "patient" may be used interchangeably. Suitable subjects or patients in need of preventing or treating a disease as taught herein include those that would benefit from treating the disease or those in whom said disease is to be prevented. Such subjects or patients include without limitation patients presenting to a physician for a screening for the disease, patients presenting to a physician with symptoms and signs indicative of the disease, patients diagnosed with the disease, patients prone to contract or develop the disease, patients who have received or are undergoing treatment of the disease, and patients having a disease as taught herein in remission. In aspects and embodiments relating to cosmetic treatments, the term "subject" which does not imply the presence of a pathological condition in the subject, may be preferred over the term "patient". A phrase such as "a subject in need of" a certain intervention, such as the treatment of a given condition, includes subjects that would benefit from the treatment of that condition. The presence or absence of a need for the subject to receive a given intervention such as treatment may be inferred by various diagnostic or benefit-evaluation methods, including the methods and uses described elsewhere in the specification.

As used herein "oxidative stress-associated skin disease" broadly refers to diseases or symptoms of diseases associated with, contributed to, caused by or resulting from an imbalance between the production and detoxification of free radicals such as in particular reactive oxygen species (ROS) or reactive nitrogen species (RNS) in favour of the pro-oxidant, in skin tissue or cells leading to tissue or cell damage in the skin. Non-limiting examples of ROS include superoxide anion, hydroperoxide anion, hydroxyl radical, singlet oxygen, hypochloride anion, tert-Butyl hydroperoxide, and the like. Non-limiting examples of RNS include nitric oxide (NO), peroxynitrite (ONOO⁻), and the like. An oxidative stress-associated skin disease may affect only skin or may affect one or more other tissues or organs in addition to the skin. An oxidative stress-associated skin disease may be chronic or acute. Without wishing to be bound to any theory, oxidative stress in the skin may lead to chronic inflammation, which in turn can cause collagen fragmentation and disorganisation of collagen fibres and skin cell functions; moreover oxidative stress was reported to also participate in most if not all stages of carcinogenesis.

In certain embodiments, the oxidative stress-associated skin disease is selected from the group comprising, consisting essentially of, or consisting of actinic keratosis (AK), basal cell carcinoma (BCC), squamous cell carcinoma (SCC), dandruff, seborrheic dermatitis, acne, inflammation, dermatitis, psoriasis, eczema, rosacea, urticaria and vitiligo. In certain preferred embodiments, the oxidative stress-associated skin disease is AK. In further preferred embodiments, the oxidative stress-associated skin disease is BCC. In yet further preferred embodiments, the oxidative stress-associated skin disease is SCC. In yet further preferred embodiments, the oxidative stress-associated skin disease is dandruff. In yet further preferred embodiments, the oxidative stress-associated skin disease is seborrheic dermatitis.

Any such diseases may include components, aspects or processes other than those related to the oxidative stress. In certain embodiments, the present prophylactic uses or methods treat, address or impinge on the oxidative stress component(s), aspect(s) or process(es) of any such disease, for example, they improve or restore the redox balance in the skin tissue or cells.

The cosmetic uses or methods as taught herein generally comprise administering to the skin of the subject a cosmetically effective amount of the cosmetic composition as taught herein, that is an amount sufficient to elicit the cosmetic effect relating to the prevention or reduction of skin aging in the subject, that is being sought by the cosmetician or beautician, in either a single or multiple doses. The prophylactic or therapeutic uses or methods as taught herein generally comprise administering to the skin of the subject a prophylactically or therapeutically effective amount of the pharmaceutical composition as taught herein. The term "therapeutically effective amount" generally denotes an amount sufficient to elicit the pharmacological effect or medicinal response in a subject that is being sought by a medical practitioner such as a medical doctor, clinician, surgeon, veterinarian, or researcher, which may include *inter alia* alleviation of the symptoms of the disease being treated, in either a single or multiple doses. The term "prophylactically effective amount" generally denotes an amount sufficient to elicit the preventative effect, such as inhibition or delay of the onset of a disease, in a subject that is being sought by the medical practitioner, in either a single or multiple doses. Appropriate cosmetically effective doses of the present compositions may be determined by a cosmetician with due regard to the age and skin condition of the patient. Appropriate prophylactically or therapeutically effective doses of the present compositions may be determined by a qualified physician with due regard to the nature and severity of the disease, and the age and condition of the patient. The effective amount of the compositions described herein to be administered can depend on many different factors and can be determined by one of ordinary skill in the art through routine experimentation. Several non-limiting factors that might be considered include biological activity of the active ingredient, nature of the active ingredient, characteristics of the subject to be treated, etc.

The term "to administer" generally means to dispense or to apply, and typically includes both *in vivo* administration and *ex vivo* administration to a tissue, preferably *in vivo* administration. Generally, compositions may be administered systemically or locally. Given the nature of the present cosmetic, prophylactic or therapeutic treatments, and the nature of the active ingredient, the present compositions may be preferably configured for topical administration to the skin of the subject.

The term "topical administration" as generally used in the cosmetic and medical fields denotes the application of compositions directly to a part of the body. Particularly in the present case the term refers to topical administration onto the skin of a subject, more particularly onto the surface of the skin of the subject, and even more particularly onto a part, region or area of the surface of the subject's skin where the cosmetic or pharmacological effect is sought. Topical administration is typically not intended to and does not elicit any systemic effects.

The cosmetic or pharmaceutical compositions disclosed herein typically comprise, in addition to the one or more actives, one or more cosmetically or pharmaceutically or acceptable carriers.

As used herein, the terms "carrier" or "excipient" are used interchangeably and broadly include any and all solvents, diluents, buffers (such as, e.g., neutral buffered saline, phosphate buffered saline, or optionally Tris-HCl, acetate or phosphate buffers), solubilisers (such as, e.g., Tween^{®} 80, Polysorbate 80), colloids, dispersion media, vehicles, fillers, chelating agents (such as, e.g., EDTA or glutathione), amino acids (such as, e.g., glycine), proteins, disintegrants, binders, lubricants, wetting agents, emulsifiers, sweeteners, colorants, flavourings, aromatisers, thickeners, agents for achieving a depot effect, coatings, antifungal agents, preservatives (such as, e.g., ThimerosalTM, benzyl alcohol), antioxidants (such as, e.g., ascorbic acid, sodium metabisulfite), tonicity controlling agents, absorption delaying agents, adjuvants, bulking agents (such as, e.g., lactose, mannitol) and the like. The use of such media and agents for the formulation of pharmaceutical and cosmetic compositions is well known in the art.

Aqueous, powder or oily bases, or thickeners may also be used in preparations for topical administration to the skin. Examples of such ingredients include various hydroxylated compounds, such as monomeric glycols, e.g., propylene glycol, ethyl alcohol, glycerin and butylene glycol, polymeric moisturizers such as polyglycerylmethacrylate, derivatives of palmitates and stearates, triglycerides of fatty acids, lanolin, vegetable or mineral oils, and waxes.

In certain embodiments, the compositions comprising a live C. *acnes* strain may further comprise media for stabilising the bacteria. These media may include, for example, pure water, phosphate buffer saline (PBS), peptone (e.g. trypsin-digested or acid-digested peptone from casein or meat), a diluted or undiluted version of a suitable growth medium or any combination thereof. In some embodiments, the percentage of peptone in the bacterial composition is about 0.05% w/v or about 0.1% w/v, and may range from 0.005% w/v to 1% w/v, or from 0.05% w/v to 1% w/v. In some embodiments, the percentage of peptone is less than 0.005% w/v, or is greater than 1% w/v, or is about 0.25% w/v. In other embodiments, a suitable growth medium is used in addition to or instead of peptone.

In certain embodiments, the compositions may comprise a buffer. The buffer can help stabilizing the pH of the composition. In embodiments, the pH is between 4.5 and 8. For example, the pH can be approximately 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 or 8.0, including any value in between. In some embodiments, the pH is approximately 7.0. Non-limiting examples of buffers include ACES, acetate, ADA, ammonium hydroxide, AMP (2-amino-2-methyl-l-propanol), AMPD (2-amino-2-methyl-l,3-propanediol), AMPSO, BES, BICINE, bis-tris, BIS-TRIS propane, borate, CABS, cacodylate, CAPS, CAPSO, carbonate (pKl), carbonate (pK2), CHES, citrate (pKl), citrate (pK2), citrate (pK3), DIPSO, EPPS, HEPPS, ethanolamine, formate, glycine (pHI), glycine (pK2), glycylglycine (pKl), glycylglycine (pK2), HEPBS, HEPES, HEPPSO, histidine, hydrazine, imidazole, malate (pKl), malate (pK2), maleate (pKl), maleate (pK2), MES, methylamine, MOBS, MOPS, MOPSO, phosphate (pKl), phosphate (pK2), phosphate (pK3), piperazine (pKl), piperazine (pK2), piperidine, PIPES, POPSO, propionate, pyridine, pyrophosphate, succinate (pKl), succinate (pK2), TABS, TAPS, TAPSO, taurine (AES), TES, tricine, triethanolamine (TEA), and Trizma (tris).

In some embodiments, the compositions may comprise a thickener. Non-limiting examples of thickeners include hydroxyethylcelluloses (e.g. NATROSOL^{®}), starch, gums such as gum arabic, kaolin or other clays, hydrated aluminum silicate, fumed silica, carboxyvinyl polymer, sodium carboxymethyl cellulose or other cellulose derivatives, ethylene glycol monostearate and sodium alginates. In some embodiments, the viscosity type of hydroxyethylcellulose is HHR-P, HH, H4, H, MH, M, K, G, E or L. In some embodiments, the concentration of the thickener, such as hydroxyethyl cellulose, is between approximately 1%-5% w/v. For example, the concentration can be about 1, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, or 5.0% w/v. In other embodiments, the concentration of thickener, such as hydroxyethyl cellulose, is less than 1% w/v or more than 5% w/v. In some embodiments, the concentration of thickener, such as hydroxyethyl cellulose, is approximately 1.5% w/v. In some embodiments, the concentration of thickener, such as hydroxyethyl cellulose, is approximately 2.5% w/v.

In some embodiments, the compositions may comprise a solvent or a surfactant. Non-limiting examples of solvents include water, ethyl alcohol, toluene, methylene chloride, isopropanol, n-butyl alcohol, castor oil, ethylene glycol monoethyl ether, diethylene glycol monobutyl ether, diethylene glycol monoethyl ether, dimethyl sulphoxide, dimethyl formamide and tetrahydrofuran. i) Anionic surfactants, such as metallic or alkanolamine salts of fatty acids for example sodium laurate and triethanolamine oleate; alkyl benzene sulphones, for example triethanolamine dodecyl benzene sulphonate; alkyl sulphates, for example sodium lauryl sulphate; alkyl ether sulphates, for example sodium lauryl ether sulphate (2 to 8 EO); sulphosuccinates, for example sodium dioctyl sulphonsuccinate; monoglyceride sulphates, for example sodium glyceryl monostearate monosulphate; isothionates, for example sodium isothionate; methyl taurides, for example Igepon T; acylsarcosinates, for example sodium myristyl sarcosinate; acyl peptides, for example Maypons and lamepons; acyl lactylates, polyalkoxylated ether glycollates, for example trideceth-7 carboxylic acid; phosphates, for example sodium dilauryl phosphate; Cationic surfactants, such as amine salts, for example sapamin hydrochloride; quartenary ammonium salts, for example Quaternium 5, Quaternium 31 and Quaternium 18; Amphoteric surfactants, such as imidazol compounds, for example Miranol; N-alkyl amino acids, such as sodium cocaminopropionate and asparagine derivatives; betaines, for example cocamidopropylebetaine; Nonionic surfactants, such as fatty acid alkanolamides, for example oleic ethanolamide; esters or polyalcohols, for example Span; polyglycerol esters, for example that esterified with fatty acids and one or several OH groups; Polyalkoxylated derivatives, for example polyoxy:polyoxyethylene stearate; ethers, for example polyoxyethe lauryl ether; ester ethers, for example Tween^{®} such as Tween 80 (polysorbate 80); amine oxides, for example coconut and dodecyl dimethyl amine oxides.

In certain embodiments, the compositions may comprise an emollient. Non-limiting examples of emollients include stearyl alcohol, glyceryl monoricinoleate, glyceryl monostearate, propane-1,2-diol, butane- 1,3-diol, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, cetyl palmitate, dimethylpolysiloxane, di-n-butyl sebacate, isopropyl myristate, isopropyl palmitate, isopropyl stearate, butyl stearate, polthylene glycol, triethylene glycol, lanolin, sesame oil, coconut oil, arrachis oil, castor oil, acetylated lanolin alcohols, petroleum, mineral oil, butyl myristate, isostearic acid, palmitic acid, isopropyl linoleate, lauryl lactate, myristyl lactate, decyl oleate, myristyl myristate.

In certain embodiments, the compositions may comprise a humectant. Non-limiting examples of humectants include glycerin, sorbitol, sodium 2-pyrrolidone-5-carboxylate, soluble collagen, dibutylphthalate, or gelatin.

In some embodiments, the compositions may comprise an astringent agent. Non-limiting examples of astringent agents include arnica flowers or extracts thereof, lower alkyl alcohols, witch hazel, boric acid, lactic acid, methol, camphor, zinc phenol sulphonate, aluminum acetate, aluminum sulfate, and zinc chloride or sulfate.

In some embodiments, the compositions may comprise a pigment. Non-limiting examples of pigments include titanium dioxide, micas, iron oxides, barium lake, calcium lake, aluminum lake, bismuth oxychloride, zirconium lake and calcium oxides.

In some embodiments, the compositions comprise a coloring agent. Non-limiting examples of coloring agent include shikonin, β-carotene, paprika, monascus, safflower red, safflower yellow, red cabbage color, purple sweet potato color, lycopene, cacao color, grape color, cochineal, lac color, beet red, hematein, Red. No. 215, Red. No. 218, Red. No. 223, Red. No. 225, Orange No. 201, Orange No. 206, Yellow No. 201, Green No. 202, and Purple No. 201, Red. No. 2, Red. No. 3, Red. No. 102, Red. No. 104 (1), Red. No. 105 (1), Red. No. 106, Yellow No. 4, Yellow No. 5, Green No. 3, Blue No. 1, Blue No. 2, Red. No. 201, Red. No. 213, Red. No. 214, Red. No. 227, Red. No. 230 (1), Red. No. 230 (2), Red. No. 231, Red. No. 232, Orange No. 205, Orange No. 207, Yellow No. 202 (1), Yellow No. 202 (2), Yellow No. 203, Green No. 201, Green No. 204, Green No. 205, Blue No. 202, Blue No. 203, Blue No. 205, and Brown No. 201.

In some embodiments, the compositions may comprise any one or more of UV-A and UV-B radiation filters, sunscreens, free-radical blockers, vitamin extracts, or antioxidants.

In some embodiments, the compositions may comprise a perfume.

Excipients that may be included in compositions disclosed herein are further described herein.

In certain preferred embodiments, one or more thickening agents may be selected from the group consisting of carbomer, acrylates/C 10-30 alkyl acrylate crosspolymer, ammonium acryloyldimethyltaurate/VP copolymer, xanthan gum, sodium polyacrylate, calcium carboxymethyl cellulose, carboxymethyl cellulose acetate butyrate, carboxymethyl hydroxyethylcellulose, cellulose gum, cellulose acetate propionate carboxylate, cetyl hydroxyethylcellulose, ethylcellulose, hydrolyzed cellulose gum, hydroxybutyl methylcellulose, hydroxyethylcellulose, hydroxyethyl ethylcellulose, hydroxypropylcellulose, hydroxypropyl methylcellulose, methylcellulose, methyl ethylcellulose, methyl hydroxyethylcellulose, microcrystalline cellulose, sodium cellulose sulfate, acrylates copolymer, acrylates crosspolymer-4, polyacrylate- 1 crosspolymer, acrylates/steareth-20 methacrylate copolymer, acrylates/beheneth-25 methacrylate copolymer, and combinations thereof.

In certain preferred embodiments, one or more UV-filters may be selected from the group consisting of 2-phenyl-3H-benzimidazole-5-sulfonic acid and/or the salt thereof; phenylene-l ,4-bis-(2-benzimidazyl)-3,3'-5,5'-tetrasulfonic acid and salts thereof (For example (CTFA): Disodium Phenyl Dibenzimidazole Tetrasulfonate); l ,4-di(2-oxo-10-sulfo-3-bornyliden-methyl) benzene and salts thereof; 4-Hydroxy-2-methoxy-5-(oxo-phenylmethyl)benzenesulfonic acid and salts thereof (For example (CTFA): Benzophenone-4, Benzophenone-5); 4-(2-oxo-3-bornylidenemethyl)benzene sulfonic acid and salts thereof; 2-methyl-5-(2-oxo-3-bornylidenemethyl) sulfonic acid and salts thereof; terephthalylidene dicamphor sulfonic acid; and combinations thereof.

Also suitable for protection from UV radiation is a UVA filter substance and / or at least one UVB filter substance, inorganic pigment, bisresorcinyltriazine derivatives, 2,4-bis - {[4- (2-ethylhexyloxy) -2-hydroxy] phenyl} -6- (4-methoxyphenyl) -1,3,5-triazine Dioctylbutamidotriazone, octyl Triazone, Bisresorcinyltriazine derivatives, 2,4-bis - {[4- (3-sulfonato) -2-hydroxy-propyloxy) -2-hydroxy] phenyl} -6- (4-methoxyphenyl) -1, 3,5-triazine sodium salt, 2,4-bis - {[4- (3- (2-propyloxy) -2-hydroxy-propyloxy) -2-hydroxy] phenyl} -6- (4-methoxyphenyl) - 1,3,5-triazine, 2,4-bis - {[4- (2-ethyl-hexyloxy) -2-hydroxy] phenyl} -6- [4-(2-methoxyethyl-carboxyl) -phenylamino] -1,3,5-triazine, 2,4-bis - {[4- (3- (2-propyloxy) -2-hydroxypropyloxy) -2-hydroxy] phenyl} -6- [4- (2- ethyl-carboxyl) -phenylamino] -1,3,5-triazine, 2,4-bis - ({[4- (2-ethyl-hexyloxy) -2-hydroxy] phenyl} -6- 1-methyl- pyrrol-2-yl) - 1,3,5-triazine, 2,4-bis - {[4-tris (trimethylsiloxy-silylpropyloxy) -2-hydroxy] phenyl} -6- (4-methoxyphenyl) -1 , 3,5-triazine, 2,4-bis - {[(- methylpropenyloxy 2 ") -2-hydroxy 4-] phenyl} - 6- (4-methoxyphenyl) -1,3,5-triazine and 2,4-bis - {[4- (1 ', 1', 1 ', 3', 5 ', 5', 5'-Heptamethylsiloxy-2 " - methylpropyloxy) -2-hydroxy] phenyl} - 6- (4-methoxyphenyl) -1,3,5-triazine, Bisimidazylate, phenylbenzimidazole sulfonic acid, Terephthalidene dicamphor sulfonic acid, 4- (2-oxo-3-bornylidenemethyl) benzenesulfonic acid, 2-methyl-5- (2-oxo-3-bornylidenemethyl) sulfonic acid, 1,4-di (2-oxo-10-sulfo-3-bornylidenemethyl) benzene, 3-benzylidene camphor derivatives, 4-aminobenzoic acid derivatives, benzophenone derivatives, homomenthyl salicylate, 2-ethylhexyl-2-cyano-3,3-diphenylacrylate, 2-ethylhexyl-2-hydroxybenzoate, 4-methoxycinnamic acid (2-ethylhexyl ) ester, Homosalate, Octocrylene, Octyl Salicylate, Octyl Methoxycinnamate, Isoamyl p-Methoxycinnamate, (3- (4- (2,2-bis-ethoxycarbonylvinyl) phenoxy) propenyl) - methylsiloxane / dimethylsiloxane copolymer, dibenzoylmethane derivatives, 4- (tert-butyl) -4'-methoxydibenzoylmethane, 4-isopropyl-dibenzoylmethane, Tinosorb M^{®}, benzotriazole, Drometrizole Trisiloxane [2,4'-dihydroxy-3-(2H-benzotriazol-2-yl) -5- (1,1,3,3-tetramethylbutyl) -2'-n-octoxy 5'-benzoyl] diphenylmethane, 2,2 'methylene-bis- [6- (2N-benzotriazol-2-yl) -4- (methyl) phenol], 2,2'-methylenebis [6- (2H - benzotiazol-2-yl) -4- (1,1,3,3-tetramethylbutyl) phenol], 2- (2'-hydroxy-5'-octylphenyl) benzotriazole, 2- (2'-hydroxy-3 ' benzotriazole, 5'-di-t-amylphenyl) benzotriazole and 2- (2'-hydroxy-5'-methylphenyl), phenylene-1,4-bis- (2-benzimidazyl) -3,3'-5,5'-tetrasulphonic acid and its salts, salts of 2-phenylbenzimidazole-5-sulfonic acid, 1,4-di (2-oxo-10-sulpho-3-bornylidenemethyl) benzene, sulphonic acid derivatives of 3-benzylidenecamphor, Aniso Triazine, Diethylhexylbutamidotriazone, ethylhexyl Triazone, 2,2'-methylene-bis- (6- (2H-benzotriazol-2-yl) -4- (1,1,3,3-tetramethylbutyl) -phenol), Drometrizole Trisiloxane, 3-benzylidene camphor derivatives, 4-aminobenzoic acid derivatives, benzophenone derivatives, 3- (4- (2,2-bis ethoxycarbonylvinyl) phenoxy) propenyl) -methoxysiloxan / dimethylsiloxane dibenzoylmethane derivatives [for example, 4- (tert-butyl) -4'-methoxydibenzoylmethane, phenylene-1,4-bis- (2-benzimidazyl) -3,3'-5,5 ' -tetrasulfonsäure and / or its salts, 1,4-di (2-oxo-10-sulpho-3-bornylidenemethyl) benzene and / or its salts and / or 2,4-bis - {[4- (2 ethyl-hexyloxy) -2-hydroxy] phenyl} -6- (4-methoxyphenyl) -1,3,5-triazine, metal oxides, TiO ₂, ZnO, Fe ₂ O ₃, ZrO ₂, SiO ₂, MnO, Al ₂ O ₃, Ce ₂ O ₃, Aerosil ^{®}, surface-treated inorganic pigments, dibromodicyanobutane (2-bromo-2-brommethylglutarodinitril), 3-iodo-2-propynyl butyl carbamate, 2-bromo-2-nitro-propane-1,3-diol, imidazolidinyl urea, 5-Chloro -2-methyl-4-isothiazolin-3-one, 2-chloroacetamide, benzalkonium chloride, benzyl alcohol, Formaldehyde donors, and combinations thereof.

In certain preferred embodiments, compositions may comprise polar oils, for example those from the group of lecithins and fatty acid triglycerides, namely the triglycerol esters of saturated and / or unsaturated, branched and / or unbranched alkanecarboxylic acids having a chain length of 8 to 24, in particular 12 to 18 C-atoms. The fatty acid triglycerides can, for example, advantageously be chosen from the group of synthetic, semisynthetic and natural oils, such as olive oil, sunflower oil, soybean oil, peanut oil, rapeseed oil, almond oil, palm oil, coconut oil, castor oil, wheat germ oil, grape seed oil, safflower oil, evening primrose oil, macadamia nut and the like , Particularly preferred examples of polar oils are Triisostearin, caprylic / capric triglycerides or carbonates, such as di-n-octyl carbonates, and combinations thereof.

More polar oil components can be selected from the group of esters of saturated and / or unsaturated, branched and / or unbranched alkanecarboxylic acids having a chain length of 3 to 30 carbon atoms and saturated and / or unsaturated, branched and / or unbranched alcohols having a chain length of 3 to 30 carbon atoms, and from the group of esters of aromatic carboxylic acids and saturated and / or unsaturated, branched and / or unbranched alcohols with chain length of 3 to 30 carbon atoms, and combinations thereof. Such esters can then advantageously be selected from the group consisting of isopropyl myristate, isopropyl palmitate, isopropyl stearate, isopropyl oleate, n-butyl stearate, n-hexyl laurate, n-decyl oleate, isooctyl stearate, Isooctylnonanoate, Isononylstearat, isononyl isononanoate, isodecylneopentanoate, cetearyl isononanoate, octyl cocoate, tridecyl, Hexacaprat, tridecyl, Cetearylisononanoate, Hexacaprylat, dicaprate, Dicaprilat, 2-ethylhexyl palmitate, 2-ethylhexyl laurate, 2-hexyldecyl stearate, 2-octyldodecyl palmitate, oleyl oleate, oleyl erucate, erucyl oleate, erucyl erucate and synthetic, semi-synthetic and natural mixtures of such esters such as jojoba oil.

For water / oil emulsions, the oil phase can advantageously be chosen from the group of dialkyl ethers, the group of saturated or unsaturated, branched or unbranched alcohols, such as octyldodecanol. For example, the oil phase of W / O emulsions may have a content of C₁₂₋₁₅-alkyl benzoate.

The use of Guerbet alcohols in cosmetics is known *per se.* Such species are then in most cases characterised by the structure

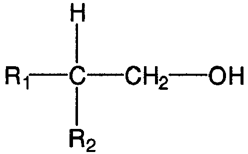

As a rule, R₁ and R₂ are unbranched alkyl radicals.

For example, the Guerbet alcohols may be selected from the group, in which Ri is propyl, butyl, pentyl, hexyl, heptyl or octyl and R₂ is hexyl, heptyl, octyl, nonyl, decyl, undecyl, dodecyl, tridecyl or tetradecyl.

Preferred Guerbet alcohols may be 2-butyl octanol: 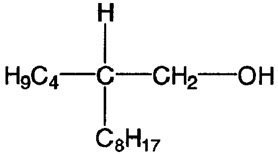 or 2-hexyl decanol: 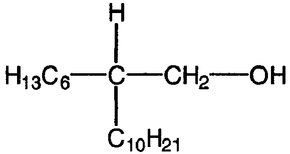 or mixtures thereof, available commercially.

The total amount of Guerbet alcohols in finished cosmetic or dermatological preparations may be advantageously chosen from a range up to 25.0 wt %, preferably 0.5-15.0 wt %, based on the total weight of the preparations.

A further suitable ester is cetyl palmitate.

Non-polar oils may be, for example, chosen from the group of branched and unbranched hydrocarbons and waxes, in particular petroleum jelly (petrolatum), paraffin oil, squalane and squalene, polyolefins and hydrogenated polyisobutenes. Among the polyolefins, polydecenes are the preferred substances.

Other excipients may be selected from the group of cyclic and / or linear silicones, and silicone oils.

Phenyltrimethicone may be advantageously chosen as silicone oil. Other silicone oils, such as dimethicone, phenyldimethicone, cyclomethicone (octamethylcyclotetrasiloxane), for example hexamethylcyclotrisiloxane, polydimethylsiloxane, poly (methylphenylsiloxane), cetyl dimethicone, behenoxydimethicone may advantageously be used.

Mixtures of cyclomethicone and isotridecyl isononanoate, as well as those of cyclomethicone and 2-ethylhexyl isostearate are furthermore advantageous. It is also advantageous to choose silicone oils of similar constitution to the above-described compounds whose organic side chains are derivatized, for example, polyethoxylated and / or polypropoxylated. For this purpose, polysiloxane-polyalkyl-polyether copolymers, such as cetyl dimethicone copolyol, include, for example (cetyl dimethicone copolyol (and) polyglyceryl-4 isostearate (and) hexyl laurate). Other excipients may be selected from the group of vegetable waxes, animal waxes, mineral waxes, petrochemical waxes, and combinations thereof. Such as candelilla wax, carnauba wax, Japan wax, esparto grass, cork wax, Guarumawachs, rice germ oil wax, sugarcane wax, berry wax, ouricury wax, montan wax, jojoba wax, shea butter, beeswax, shellac wax, spermaceti, lanolin (wool wax), uropygial grease, ceresin, ozokerite (earth wax), paraffin waxes and micro waxes, Syncrowax HRC (glyceryl tribehenate), Syncrowax HGLC (C ₁₆₋₃₆ - fatty acid triglycerid) and Syncrowax AW 1C (C ₁₈₋₃₆ fatty acid) available from Croda GmbH and Dynasantypen, Montanesterwachse, sasol waxes, hydrogenated jojoba waxes, synthetic or modified beeswax (for example, dimethicone copolyol beeswax and / or C ₃₀-₅₀ alkyl beeswax), polyalkylene waxes, polyethylene glycol waxes, but also chemically modified fats such as hydrogenated vegetable oils (for example hydrogenated castor oil and / or hydrogenated coconut fatty glycerides), triglycerides such as trihydroxystearin, fatty acids, fatty acid ester and Glykolester such as C₂₀₋₄₀ alkyl stearate, C₂₀₋₄₀ Alkylhydroxystearoylstearat and / or glycol montanate, and combinations thereof. Also advantageous may be certain organosilicon compounds which have similar physical properties to the specified fat and / or wax components, such as stearoxytrimethylsilane.

An oil phase may be advantageously selected from the group 2-ethylhexyl isostearate, octyldodecanol, isotridecyl, butylene glycol dicaprylate / dicaprate, C12-15-alkyl benzoate, caprylic-capric acid triglyceride, dicaprylyl ether, and combinations thereof.

Advantageous may be mixtures of octyldodecanol, caprylic-capric acid triglyceride, dicaprylyl ether, octyl carbonates, cocoglycerides, or mixtures of C12-15-alkyl benzoate and 2-ethylhexyl isostearate, mixtures of C12-15-alkyl benzoate and butylene glycol dicaprylate / dicaprate and mixtures of C12 -15-alkyl benzoate, 2-ethylhexyl isostearate, isotridecyl, and combinations thereof.

Of the hydrocarbons, paraffin oil, cycloparaffin, squalane, squalene, hydrogenated polyisobutene, polydecene, and combinations thereof may be used advantageously.

Further excipients may include lecithin, lanolin, microcrystalline wax (Cera microcristallina) mixed with paraffin oil (Paraffinum liquidum), ozokerite, hydrogenated castor oil, polyglyceryl-3 oleate, wool wax acid mixtures, wool wax alcohol mixtures Pentaerythrithylisostearat, polyglyceryl-3-diisostearate, beeswax (Cera alba) and stearic acid, Natriumdihydroxycetylphosphat in admixture with Isopropylhydroxycetylether, methyl glucose dioleate, methyl glucose mixed with hydroxystearate wax and beeswax, mineral oil mixed with petrolatum and ozokerite and glyceryl oleate and lanolin, petrolatum mixture with ozokerite and hydrogenated castor oil and glyceryl and polyglyceryl-3 oleate, PEG-7 hydrogenated castor oil, ozokerite and hydrogenated castor oil, polyglyceryl-4 isostearate, polyglyceryl-4 isostearate in a mixture with hexyl laurate and cetyl dimethicone copolyol, lauryl methicone copolyol, cetyl dimethicone copolyol, acrylate / C ₁₀-₃₀ alkyl acrylate crosspolymer, poloxamer 101, polyglyceryl-3-Methylglucosediste stearate, Polyglyceryl-2 dipolyhydroxystearate, polyglyceryl-3 diisostearate, polyglyceryl-4 dipolyhydroxystearate, PEG-30 dipolyhydroxystearate, Diisostearoylpolyglyceryl- 3-diisostearate, polyglyceryl-2-dipolyhydroxystearate, polyglyceryl-3 dipolyhydroxystearate, polyglyceryl-4 dipolyhydroxystearate, polyglyceryl-3 dioleate, and combinations thereof.

Water / oil emulsions may, if desired, contain one or more co-emulsifiers, especially advantageously selected from the group of the following substances that act generally as O / W emulsifiers:
Glyceryl stearate in a mixture with ceteareth-20, ceteareth-25, ceteareth-6 in a mixture with stearyl alcohol, Triceteareth-4 phosphate, sodium cetylstearyl sulfate, lecithin trilaureth-4 phosphate, laureth-4 phosphate, stearic acid, propylene glycol stearate SE, PEG-25 hydrogenated castor oil, PEG-54 hydrogenated castor oil, PEG-6 caprylic / capric acid glycerides, glyceryl oleate in the mixture with propylene glycol ceteth-2, ceteth-20, polysorbate 60, glyceryl stearate in a mixture with PEG-100 stearate, Laureth-4, ceteareth-3, isostearyl glyceryl ether, cetylstearyl alcohol in a mixture with sodium cetylstearyl sulfate, laureth-23, steareth-2, glyceryl stearate in a mixture with PEG-30 stearate, PEG-40 stearate, glycol distearate, PEG-22 dodecyl glycol copolymer, polyglyceryl-2 PEG-4 stearate, ceteareth-20, methyl glucose, steareth-10, PEG-20 stearate, Steareth-2 in a mixture with PEG-8 distearate, steareth-21, steareth-20, isosteareth 20, PE G-45 / dodecyl glycol copolymer, methoxy PEG-22 / dodecyl glycol copolymer, PEG-20 glyceryl stearate, PEG-20 glyceryl stearate, PEG-8 beeswax, polyglyceryl-2 laurate, Isostearyldiglycerylsuccinat, stearamidopropyl PG-dimonium chloride phosphate, glyceryl stearate SE, ceteth-20, triethyl citrate, PEG-20 methyl glucose sesquistearate, ceteareth-12, glyceryl stearate, cetyl phosphate, triceteareth-4 phosphate, trilaureth-4-phosphate, Polyglycerylmethylglucosedistearat, potassium cetyl phosphate, isosteareth-10, polyglyceryl-2-sesquiisostearat, ceteth -10, oleth-20, isoceteth-20, glyceryl stearate in a mixture with ceteareth-20, ceteareth-12, cetostearyl alcohol and cetyl alcohol, cetylstearyl alcohol in a mixture with PEG-20 stearate, PEG-30 stearate, PEG-40 stearate, PEG-100 stearate.

Also suitable may be silicone emulsifiers, Alkylmethiconco and / or alkyldimethicone copolyols, ABIL ^{®} B 8842, ABIL^{®} B 8843, ABIL ^{®} B 8847, ABIL ^{®} B 8851, ABIL ^{®} B 8852, ABIL ^{®} B 8863, ABIL ^{®} B 8873 and ABIL ^{®} B 8883.

The skilled person shall appreciate that any number of such carriers or excipients may be included in the compositions.

In certain embodiments, the compositions may be formulated as a gel, cream, ointment, lotion, drops, spray including aerosol sprays, foam, or powder. In certain embodiments, the compositions may be comprised by a mask, pad, patch, a two-chamber device (two-component dispensing system), or make-up. The present compositions may typically be intended as 'leave-on' compositions.

The present compositions can be administered once or multiple times. In certain embodiments, the composition is administered at regular intervals, while in other embodiments it is administered at irregular intervals. For example, the composition may be administered four times a day, three times a day, or twice a day, or daily, or every 2 days, 3 days, 4 days, or 5 days, or once a week, or once in 2 weeks, 3 weeks, 4 weeks, 5 weeks, 6 weeks, 7 weeks, or 8 weeks or more or less frequently including all values in between.

The duration of the administration of the compositions may be limited or may be unlimited or open-ended. For example, the administration may be sustained for 1 week, 2 weeks, 3 weeks, 4 weeks, 6 weeks, 2, 3, 4, 5, 6, 7, 8, 9, 10, 11, or 12 months, or 1, 2, 3, 4, or 5 or more years.

Also provided are any of the herein-mentioned compositions in kits. In some embodiments, a kit comprises a container housing live bacteria or a container housing freeze-dried live bacteria. Kits can include a second container including media such as peptone. In some embodiments, kits can include antibiotic(s), disinfectant(s) (e.g., BPO) and/or salicylic acid. In some embodiments, the antibiotic(s), disinfectant(s) and/or salicylic acid are used to pre- treat the skin before application of the composition comprising live bacteria. Kits can also include instructions for administering the composition. In certain embodiments, instructions are provided for mixing the bacterial strains with other components of the composition. In some embodiments, a kit further includes an applicator to apply the bacterial composition to a subject.

Also disclosed but not part of the claimed subject-matter is a method for diagnosing oxidative stress-associated skin disease in a subject, the method comprising determining the quantity of RoxP or the quantity of *C. acnes* in a skin sample from the subject. Said oxidative stress-associated skin disease may be selected from the group comprising AK, BCC, SCC, dandruff, seborrheic dermatitis, acne, inflammation, dermatitis, psoriasis, eczema, rosacea, urticaria and vitiligo.

Also disclosed but not part of the claimed subject-matter is a method for determining whether a subject would benefit from administration of RoxP or a live C. *acnes* strain secreting RoxP, the method comprising determining the quantity of RoxP or the quantity of C. *acnes* in a skin sample from the subject.

The term "diagnosis" is commonplace and well-understood in medical practice. By means of further explanation and without limitation the term "diagnosis", or its alternative forms such as "diagnosing", generally refers to the process or act of recognising, deciding on or concluding on a disease or condition in a subject on the basis of symptoms and signs and/or from results of various diagnostic procedures (such as, for example, from knowing the presence, absence and/or quantity of one or more biomarkers characteristic of the diagnosed disease or condition).

The term "diagnosis" as used herein also broadly encompasses aspects of diagnostic practice which may be more specifically referred to as monitoring, prognosis or prediction of a disease. The term "monitoring" generally refers to the follow-up of a disease or a condition in a subject for any changes which may occur over time. The term "prognosis" generally refers to an anticipation on the progression of a disease or condition and the prospect (e.g., the probability, duration, and/or extent) of recovery. A good prognosis of a disease or condition may encompass anticipation of a satisfactory partial or complete recovery from the disease or condition, preferably within an acceptable time period. A good prognosis may also commonly encompass anticipation of not further worsening or aggravating of the disease or condition, preferably within a given time period. A poor prognosis of a disease or condition may generally encompass anticipation of a substandard recovery and/or unsatisfactorily slow recovery, or substantially no recovery or even further worsening of the disease or condition.

The terms "predicting" or "prediction" generally refer to an advance declaration, indication or foretelling of a disease or condition in a subject not (yet) having said disease or condition. For example, a prediction of a disease or condition in a subject may indicate a probability, chance or risk that the subject will develop said disease or condition, for example within a certain time period or by a certain age. Said probability, chance or risk may be indicated *inter alia* as an absolute value, range or statistics, or may be indicated relative to a suitable control subject or subject population (such as, e.g., relative to a general, normal or healthy subject or subject population). Hence, the probability, chance or risk that a subject will develop a disease or condition may be advantageously indicated as increased or decreased, or as fold-increased or fold-decreased relative to a suitable control subject or subject population.

The present methods for diagnosing diseases or conditions or for determining whether a subject would benefit from a given course of action or treatment may be adequately qualified as *in vitro* methods in that they apply one or more *in vitro* processing and/or analysis steps to a sample removed from the subject. The term "*in vitro*" generally denotes outside, or external to, a body, e.g., an animal or human body.

The present methods or uses may preferably allow for sensitivity and/or specificity (preferably, sensitivity and specificity) of at least 50%, at least 60%, at least 70% or at least 80%, e.g., ≥ 85% or ≥ 90% or ≥95%, e.g., between about 80% and 100% or between about 85% and 95%.

Hence, in such methods, RoxP or *C. acnes* constitute biomarkers to be determined in a skin sample from the subject. Accordingly, also disclosed is the use of RoxP or *C. acnes* as a biomarker for diagnosing oxidative stress-associated skin disease in a subject or for determining whether a subject would benefit from administration of RoxP or a live *C. acnes* strain secreting RoxP.

In certain embodiments, the present methods or uses may evaluate a single variable, such as a single biomarker. In other embodiments, the present methods or uses may evaluate two or more variables, such as two or more biomarkers. For example, the methods or uses may evaluate RoxP and *C. acnes.* Each so-measured variable, such as each biomarker, may be evaluated separately and independently, or one may generate a profile from the values or quantities for the two or more variables. It shall thus also be understood by the skilled man that any value or quantity as referred to herein may also encompass a profile. Similarly, any reference value as referred to herein may also encompass a reference profile.

The term "biomarker" is widespread in the art and commonly broadly denotes a biological entity or substance, such as for example a biological molecule and/or a detectable portion thereof, or a cell such as a microbial (e.g., bacterial) cell, whose qualitative and/or quantitative evaluation in a subject is predictive or informative (e.g., predictive, diagnostic and/or prognostic) with respect to one or more aspects of the subject's phenotype and/or genotype, such as, for example, with respect to the status of the subject as to a given disease or condition, or as to an expected benefit of a certain course of action or treatment of the subject

Typically, biomarkers may be cell-based, or peptide-, polypeptide- and/or protein-based, or nucleic acid-based. For example, a biomarker may be a microbial (e.g., bacterial) cell of a given genus, species, type (e.g., phylotype), subtype or strain, or a microbial (e.g., bacterial) cell of a given phenotype or genotype, or expressing a given protein or polypeptide, or carrying a given genetic element or sequence. For example, a biomarker may be comprised of peptides, polypeptides and/or proteins encoded by a given gene. For example, nucleic acid-based biomarkers may encompass DNA, RNA and DNA/RNA hybrid molecules, such as DNA of a given gene or a RNA-molecule transcribed from a given gene, or any RNA or DNA (e.g., copy DNA, cDNA) molecule originating from or derived from the transcribed RNA-molecule.

The reference herein to any biomarker, whether peptide-, polypeptide-, protein-, or nucleic acid-based, also encompasses fragments thereof. Particularly, the reference to RoxP as a biomarker encompasses full-length RoxP and also encompasses any fragments thereof. Hence, the reference herein to determining or measuring (the quantity of) a given peptide, polypeptide, protein, or nucleic acid biomarker, may encompass measuring (the quantity of) the recited peptide, polypeptide, protein, or nucleic acid, and/or measuring one or more fragments thereof. For example, any biomarker peptide, polypeptide, protein, or nucleic acid, and/or one or more fragments thereof may be measured collectively, such that the measured quantity corresponds to the sum amounts of the collectively measured species. In another example, any biomarker peptide, polypeptide, protein, or nucleic acid, and/or one or more fragments thereof may be measured each individually. Accordingly, "RoxP" in this context encompasses biomarkers based on RoxP polypeptide or protein or fragments thereof, or RoxP nucleic acid or fragments thereof, including *inter alia* the RoxP promoter or any part thereof and/or the RoxP coding sequence or any part thereof.

The term "fragment" in relation to peptides, polypeptides or proteins has been generally addressed elsewhere in this specification. In the present context, any peptide, polypeptide or protein fragments which carry the information requisite for the performance of the present diagnostic or benefit-evaluation methods are intended herein. Such fragments may arise by any mechanism, *in vivo* and/or *in vitro,* such as, without limitation, by alternative transcription or translation, exo- and/or endo-proteolysis, or proteolytic degradation of the peptide, polypeptide, or protein, such as, for example, by physical, chemical and/or enzymatic proteolysis. Such fragments may preferably comprise at least about 30%, e.g., at least about 50% or at least about 70%, preferably at least about 80%, e.g., at least about 85%, more preferably at least about 90%, and yet more preferably at least about 95% or even about 99% of the contiguous amino acid sequence length of the full-length biomarker peptide, polypeptide, or protein, such as of RoxP. For example, such RoxP fragment may include a sequence of ≥ 5 consecutive amino acids, or ≥ 10 consecutive amino acids, or ≥ 20 consecutive amino acids, or ≥ 30 consecutive amino acids, e.g., ≥40 consecutive amino acids, such as for example ≥ 50 consecutive amino acids, e.g., ≥ 60, ≥ 70, ≥ 80, ≥ 90, ≥ 100, ≥ 110, ≥ 120, ≥ 130, ≥ 140, or ≥ 150 consecutive amino acids of the corresponding full-length RoxP peptide, polypeptide, or protein.

The term "fragment" with reference to a nucleic acid (polynucleotide) generally denotes a 5'- and/or 3'-truncated form of a nucleic acid. In the present context, any nucleic acid fragments which carry the information requisite for the performance of the present diagnostic or benefit-evaluation methods are intended herein. Such fragments may arise by any mechanism, *in vivo* and/or *in vitro,* such as, without limitation, by alternative transcription, exo- and/or endo-nucleolysis, or nucleolytic degradation of the nucleic acid, such as, for example, by physical, chemical and/or enzymatic nucleolysis. Such fragments may preferably comprise at least about 30%, e.g., at least about 50% or at least about 70%, preferably at least about 80%, e.g., at least about 85%, more preferably at least about 90%, and yet more preferably at least about 95% or even about 99% of the contiguous nucleic acid sequence of the full-length biomarker nucleic acid, such as of RoxP nucleic acid. For example, such RoxP nucleic acid fragments may include a sequence of ≥ 15 consecutive nucleotides, or ≥ 30 consecutive nucleotides, or ≥ 60 consecutive nucleotides, or ≥ 90 consecutive nucleotides, e.g., ≥ 120 consecutive nucleotides, such as for example ≥ 150 consecutive nucleotides, e.g., ≥ 180, ≥ 210, ≥ 240, ≥ 270, ≥ 300, ≥ 330, ≥ 360, ≥ 390, ≥ 420, or ≥ 450 consecutive nucleotides of the corresponding full-length RoxP nucleic acid, such as of the corresponding full-length RoxP promoter and/or RoxP coding sequence.

The terms "quantity", "amount" and "level" are synonymous and generally well-understood in the art. The terms as used herein may particularly refer to an absolute quantification of a biomarker in a sample, or to a relative quantification of a biomarker in a sample, i.e., relative to another value such as relative to a reference value, or to a range of values indicating a base-line of the biomarker. Such reference values or ranges may be obtained from a single subject or from a group of subjects.

An absolute quantity of a biomarker such as a peptide-, protein-, polypeptide, or nucleic acid-based biomarker, in a sample may be advantageously expressed as weight or as molar amount, or more commonly as a concentration, e.g., weight per volume or mol per volume, or weight per surface area of skin, or mol per surface area of skin, such as 1.0 cm² of skin. An absolute quantity of a cell-based biomarker in a sample may be advantageously expressed as the number of cells or colony-forming units (CFU), or more commonly as the number of cells or CFU per volume of sample or per surface area of skin, such as 1.0 cm² of skin. A relative quantity of a biomarker in a sample may be advantageously expressed as an increase or decrease or as a fold-increase or fold-decrease relative to said another value, such as relative to a reference value. Performing a relative comparison between first and second variables (e.g., first and second quantities) may but need not require determining first the absolute values of said first and second variables. For example, a measurement method may produce quantifiable readouts (such as, e.g., signal intensities) for said first and second variables, wherein said readouts are a function of the value of said variables, and wherein said readouts may be directly compared to produce a relative value for the first variable vs. the second variable, without the actual need to first convert the readouts to absolute values of the respective variables. In certain embodiments, a relative quantity of a cell-based biomarker in a sample may be advantageously expressed as an increase or decrease or as a fold-increase or fold-decrease of the number, CFU or abundance (e.g., determined by genotyping or sequencing) of cells of a given taxon (e.g., phylum, genus, species, type such as phylotype, subtype or strain) compared to cells of one or more or all other taxa in the sample or cell of all taxa in the sample.

A biomarker is "measured" or "determined" in a sample when the presence or absence and/or quantity of said biomarker is detected or determined in the sample, typically substantially to the exclusion of other entities, such as cells, molecules or analytes. Depending on factors that can be evaluated and decided on by a skilled person, such as *inter alia* the type of a biomarker, the type of a sample, the expected abundance of the biomarker in the sample, the type, robustness, sensitivity and/or specificity of the detection method used to detect the biomarker, etc., the biomarker may be measured directly in the sample, or the sample may be subjected to one or more processing steps aimed at achieving an adequate measurement of the biomarker. By means of example, the sample may be subjected to one or more isolation or separation steps, whereby the biomarker is isolated from the sample or whereby a fraction of the sample is prepared which is enriched for the biomarker. For example, if the biomarker is a peptide, polypeptide, or protein, any known protein purification technique may be applied to the sample to isolate peptides, polypeptides, and proteins therefrom. If the biomarker is a nucleic acid, any known nucleic acid purification technique may be applied to the sample to isolate nucleic acids therefrom. Non-limiting examples of methods to purify peptides, polypeptides, proteins, or nucleic acids may include chromatography, preparative electrophoresis, centrifugation, precipitation, affinity purification, purification using molecular imprint cryogel matrix, etc. If the biomarker is a cell, any known cell isolation technique may be applied to the sample to isolate cells therefrom. Non-limiting examples of methods to isolate cells is cell sorting by flow cytometry, cell culture in solid or liquid media, etc.

The terms "sample" or "biological sample" as used herein include any biological specimen obtained from a subject. Useful samples are those which comprise a biomarker or biomarkers of interest as taught herein in detectable quantities. Preferably, a sample may be readily obtainable by minimally invasive methods allowing the removal / isolation of the sample from the subject. The term "skin sample" is broadly used herein to denote any sample obtained by sampling the skin of a subject or the surface of the skin of a subject. A skin sample may but need not contain skin cells of the subject. A convenient but non-limiting method to sample the skin surface of a subject, compatible with the present methods and uses, is to swab a given area of the skin surface (e.g., 1 × 1 cm, or 2 × 2 cm, or 3 × 3 cm, or 4 × 4 cm, or 5 × 5 cm, or between 1 cm² and 25 cm²) with conventional skin swabs (which may preferably buffer pre-soaked) for a given duration of time, such as between 10 seconds and 1 minute, e.g., about 30 seconds. The material collected on the swabs can be released into a suitable liquid medium or solvent by shaking, and prepared for analysis as required. In certain embodiments, suitable skin samples can be obtained by methods which sample (such as preferably unbiasedly) at least the microorganisms, such as bacteria, residing at the skin surface. In certain embodiments, suitable skin samples can be obtained by methods which sample (such as preferably unbiasedly) peptides, polypeptides or proteins residing at the skin surface. In certain embodiments, one or more skin samples (e.g., at least 2, at least 3, at least 4, at least 5 or at least 6) may be used to measure the quantity of biomarkers therein. The use of multiple skin samples can compensate for possible variations in the biomarker quantity due to potentially heterogeneous distribution of the biomarker across skin surface. In certain embodiments, the sampling may particularly target skin areas that are suspected of being diseased or affected by a disease or condition, for example as concluded by visual or microscopic inspection.

Any separation, detection and quantification methods discussed elsewhere in this specification may be used to measure the quantity of biomarker peptides, polypeptides or proteins, such as of RoxP, in the sample. For example, such methods may include biochemical assay methods, immunoassay methods, mass spectrometry analysis methods, chromatography methods, capacitance biosensor methods, or combinations thereof. The term "immunoassay" generally refers to methods known as such for detecting one or more molecules or analytes of interest in a sample, wherein specificity of an immunoassay for the molecule(s) or analyte(s) of interest is conferred by specific binding between a specific-binding agent, commonly but without limitation an antibody, and the molecule(s) or analyte(s) of interest. Immunoassay technologies include without limitation immunohistochemistry, direct ELISA (enzyme-linked immunosorbent assay), indirect ELISA, sandwich ELISA, competitive ELISA, multiplex ELISA, radioimmunoassay (RIA), ELISPOT technologies, and other similar techniques known in the art. Principles of these immunoassay methods are known in the art, for example John R. Crowther, "The ELISA Guidebook", 1st ed., Humana Press 2000, ISBN 0896037282.

By means of further explanation and not limitation, direct ELISA employs a labelled primary binding agent, e.g., antibody, to bind to and thereby quantify target antigen in a sample immobilised on a solid support such as a microwell plate. Indirect ELISA uses a non-labelled primary binding agent, e.g., antibody, which binds to the target antigen and a secondary labelled binding agent, e.g., antibody, that recognises and allows the quantification of the antigen-bound primary binding agent. In sandwich ELISA the target antigen is captured from a sample using an immobilised 'capture' binding agent, e.g., antibody, which binds to one antigenic site within the antigen, and subsequent to removal of non-bound analytes the so-captured antigen is detected using a 'detection' binding agent, e.g., antibody, which binds to another antigenic site within said antigen, where the detection binding agent may be directly labelled or indirectly detectable as above. Competitive ELISA uses a labelled 'competitor' that may either be the primary binding agent, e.g., antibody, or the target antigen. In an example, non-labelled immobilised primary binding agent, e.g., antibody, is incubated with a sample, this reaction is allowed to reach equilibrium, and then labelled target antigen is added. The latter will bind to the primary binding agent wherever its binding sites are not yet occupied by non-labelled target antigen from the sample. Thus, the detected amount of bound labelled antigen inversely correlates with the amount of non-labelled antigen in the sample. Multiplex ELISA allows simultaneous detection of two or more analytes within a single compartment (e.g., microplate well) usually at a plurality of array addresses (see, for example, Nielsen & Geierstanger 2004. J Immunol Methods 290: 107-20 and Ling et al. 2007. Expert Rev Mol Diagn 7: 87-98 for further guidance). As appreciated, labelling in ELISA technologies is usually by enzyme (such as, e.g., horse-radish peroxidase) conjugation and the end-point is typically colourimetric, chemiluminescent or fluorescent, magnetic, piezo electric, pyroelectric and other.

Radioimmunoassay (RIA) is a competition-based technique and involves mixing known quantities of radioactively-labelled (e.g., ¹²⁵I- or ¹³¹I-labelled) target antigen with binding agent, e.g., antibody, to said antigen, then adding non-labelled or 'cold' antigen from a sample and measuring the amount of labelled antigen displaced (see, e.g., "An Introduction to Radioimmunoassay and Related Techniques", by Chard T, ed., Elsevier Science 1995, ISBN 0444821198 for guidance).

Generally, any mass spectrometric (MS) techniques that are capable of obtaining precise information on the mass of peptides, and preferably also on fragmentation and/or (partial) amino acid sequence of selected peptides (e.g., in tandem mass spectrometry, MS/MS; or in post source decay, TOF MS), are useful herein. Suitable peptide MS and MS/MS techniques and systems are well-known *per se* (see, *e.g.,* Methods in Molecular Biology, vol. 146: "Mass Spectrometry of Proteins and Peptides", by Chapman, ed., Humana Press 2000, ISBN 089603609x; Biemann 1990. Methods Enzymol 193: 455-79; or Methods in Enzymology, vol. 402: "Biological Mass Spectrometry", by Burlingame, ed., Academic Press 2005, ISBN 9780121828073) and may be used herein. MS arrangements, instruments and systems suitable for biomarker peptide analysis may include, without limitation, matrix-assisted laser desorption/ionisation time-of-flight (MALDI-TOF) MS; MALDI-TOF post-source-decay (PSD); MALDI-TOF/TOF; surface-enhanced laser desorption/ionization time-of-flight mass spectrometry (SELDI-TOF) MS; electrospray ionization mass spectrometry (ESI-MS); ESI-MS/MS; ESI-MS/(MS)ⁿ (n is an integer greater than zero); ESI 3D or linear (2D) ion trap MS; ESI triple quadrupole MS; ESI quadrupole orthogonal TOF (Q-TOF); ESI Fourier transform MS systems; desorption/ionization on silicon (DIOS); secondary ion mass spectrometry (SIMS); atmospheric pressure chemical ionization mass spectrometry (APCI-MS); APCI-MS/MS; APCI- (MS)ⁿ; atmospheric pressure photoionization mass spectrometry (APPI-MS); APPI-MS/MS; and APPI- (MS)ⁿ. Peptide ion fragmentation in tandem MS (MS/MS) arrangements may be achieved using manners established in the art, such as, e.g., collision induced dissociation (CID). Detection and quantification of biomarkers by mass spectrometry may involve multiple reaction monitoring (MRM), such as described among others by Kuhn et al. 2004 (Proteomics 4: 1175-86). MS peptide analysis methods may be advantageously combined with upstream peptide or protein separation or fractionation methods, such as for example with the chromatographic and other methods described herein below.

Chromatography may also be used for measuring biomarkers. As used herein, the term "chromatography" encompasses methods for separating chemical substances, referred to as such and vastly available in the art. In a preferred approach, chromatography refers to a process in which a mixture of chemical substances (analytes) carried by a moving stream of liquid or gas ("mobile phase") is separated into components as a result of differential distribution of the analytes, as they flow around or over a stationary liquid or solid phase ("stationary phase"), between said mobile phase and said stationary phase. The stationary phase may be usually a finely divided solid, a sheet of filter material, or a thin film of a liquid on the surface of a solid, or the like. Chromatography is also widely applicable for the separation of chemical compounds of biological origin, such as, e.g., amino acids, proteins, fragments of proteins or peptides, *etc.*

Chromatography as used herein may be preferably columnar (i.e., wherein the stationary phase is deposited or packed in a column), preferably liquid chromatography, and yet more preferably HPLC. While particulars of chromatography are well known in the art, for further guidance see, e.g., Meyer M., 1998, ISBN: 047198373X, and "Practical HPLC Methodology and Applications", Bidlingmeyer, B. A., John Wiley & Sons Inc., 1993. Exemplary types of chromatography include, without limitation, high-performance liquid chromatography (HPLC), normal phase HPLC (NP-HPLC), reversed phase HPLC (RP-HPLC), ion exchange chromatography (IEC), such as cation or anion exchange chromatography, hydrophilic interaction chromatography (HILIC), hydrophobic interaction chromatography (HIC), size exclusion chromatography (SEC) including gel filtration chromatography or gel permeation chromatography, chromatofocusing, affinity chromatography such as immuno-affinity, immobilised metal affinity chromatography, and the like.

Chromatography, including single-, two- or more-dimensional chromatography, may be used as a peptide fractionation method in conjunction with a further peptide analysis method, such as for example, with a downstream mass spectrometry analysis as described elsewhere in this specification.

Further peptide or polypeptide separation, identification or quantification methods may be used, optionally in conjunction with any of the above described analysis methods, for measuring biomarkers in the present disclosure. Such methods include, without limitation, chemical extraction partitioning, isoelectric focusing (IEF) including capillary isoelectric focusing (CIEF), capillary isotachophoresis (CITP), capillary electrochromatography (CEC), and the like, one-dimensional polyacrylamide gel electrophoresis (PAGE), two-dimensional polyacrylamide gel electrophoresis (2D-PAGE), capillary gel electrophoresis (CGE), capillary zone electrophoresis (CZE), micellar electrokinetic chromatography (MEKC), free flow electrophoresis (FFE), etc.

Capacitive biosensors are affinity biosensors that operate by registering direct binding between a biorecognition element on the sensor surface and the cognate biomarker. A biorecognition element may be for example an immunological molecule such as an antibody, or a molecularly imprinted polymer (MIP) containing biorecognition cavities for the biomarker. The biosensors measures the changes in dielectric properties and/or thickness of the dielectric layer at the electrolyte/electrode interface. See for example Ertürk et al. (2018 PLoS ONE 13:e0193754).

The level of biomarkers at the nucleic acid level may be detected using standard quantitative DNA, RNA or cDNA measurement tools known in the art. Non-limiting examples include hybridisation-based analysis, microarray expression analysis, digital gene expression (DGE), RNA-in-situ hybridisation (RISH), Northern-blot analysis and the like; PCR, RT-PCR, RT-qPCR, end-point PCR, digital PCR, droplet digital PCR, or the like; supported oligonucleotide detection, pyrosequencing, polony cyclic sequencing by synthesis, simultaneous bi-directional sequencing, single-molecule sequencing, single molecule real time sequencing, true single molecule sequencing, hybridization-assisted nanopore sequencing, sequencing by synthesis, or the like.

To determine the microbiome composition of a sample, DNA may be isolated from a sample, and a suitable genetic element or region (such as for example ribosomal RNA gene, such as preferably 16S rRNA, such as more preferably the V1-V3 region of 16S rRNA) may be amplified and sequenced therefrom, typically at high sequencing depth. The resulting reads can be classified using known statistical tools and publically available microbiome sequence databases, such as Human Oral Microbiome Database (HOMD) (http://www.homd.org/) with a manual inclusion of relevant skin bacteria (Chen et al. 2010 Database : the journal of biological databases and curation baq013).

To determine types or subtypes of the *C. acnes* population in skin swab samples, known multilocus sequence typing schemes (MLST) or single-locus sequence typing (SLST) schemes can be employed, generally involving the amplification and sequencing of select loci and sequence analysis of the alleles such as using www.medbac.dk/slst/pacnes (McDowell et al., 2012 PLoS One 7:e41480; Scholz et al. 2014 PLoS ONE 9:e104199).

Various techniques for measuring biomarkers may employ binding agents for said respective biomarkers. Hence, further disclosed are binding agents capable of specifically binding to markers, peptides, polypeptides, proteins, or nucleic acids as taught herein. Binding agents as intended throughout this specification may include *inter alia* an antibody, aptamer, photoaptamer, protein, peptide, peptidomimetic, nucleic acid such as an oligonucleotide, or a small molecule.

The term "specifically bind" as used throughout this specification means that an agent (denoted herein also as "specific-binding agent") binds to one or more desired molecules or analytes substantially to the exclusion of other molecules which are random or unrelated, and optionally substantially to the exclusion of other molecules that are structurally related. The term "specifically bind" does not necessarily require that an agent binds exclusively to its intended target(s). For example, an agent may be said to specifically bind to target(s) of interest if its affinity for such intended target(s) under the conditions of binding is at least about 2-fold greater, preferably at least about 5-fold greater, more preferably at least about 10-fold greater, yet more preferably at least about 25-fold greater, still more preferably at least about 50-fold greater, and even more preferably at least about 100-fold or more greater, than its affinity for a non-target molecule.

Specific binding agents as used throughout this specification may include *inter alia* an antibody, aptamer, spiegelmer (L-aptamer), photoaptamer, protein, peptide, peptidomimetic, nucleic acid such as an oligonucleotide, or a small molecule.

Preferably, the agent may bind to its intended target(s) with affinity constant (K_{A}) of such binding K_{A} ≥ 1×10⁶ M⁻¹, more preferably K_{A} ≥ 1×10⁷ M⁻¹, yet more preferably K_{A} ≥ 1×10⁸ M⁻¹, even more preferably K_{A} ≥ 1×10⁹ M⁻¹, and still more preferably K_{A} ≥ 1×10¹⁰ M⁻¹ or K_{A} ≥ 1×10¹¹ M⁻¹, wherein K_{A} = [SBA_T]/[SBA][T], SBA denotes the specific-binding agent, T denotes the intended target. Determination of K_{A} can be carried out by methods known in the art, such as for example, using equilibrium dialysis and Scatchard plot analysis.

As used herein, the term "antibody" is used in its broadest sense and generally refers to any immunologic binding agent. The term specifically encompasses intact monoclonal antibodies, polyclonal antibodies, multivalent (e.g., 2-, 3- or more-valent) and/or multi-specific antibodies (e.g., bi- or more-specific antibodies) formed from at least two intact antibodies, and antibody fragments insofar they exhibit the desired biological activity (particularly, ability to specifically bind an antigen of interest), as well as multivalent and/or multi-specific composites of such fragments. The term "antibody" is not only inclusive of antibodies generated by methods comprising immunisation, but also includes any polypeptide, e.g., a recombinantly expressed polypeptide, which is made to encompass at least one complementarity-determining region (CDR) capable of specifically binding to an epitope on an antigen of interest. Hence, the term applies to such molecules regardless whether they are produced *in vitro* or *in vivo.*

An antibody may be any of IgA, IgD, IgE, IgG and IgM classes, and preferably IgG class antibody. An antibody may be a polyclonal antibody, e.g., an antiserum or immunoglobulins purified there from (e.g., affinity-purified). An antibody may be a monoclonal antibody or a mixture of monoclonal antibodies. Monoclonal antibodies can target a particular antigen or a particular epitope within an antigen with greater selectivity and reproducibility. By means of example and not limitation, monoclonal antibodies may be made by the hybridoma method first described by Kohler et al. 1975 (Nature 256: 495), or may be made by recombinant DNA methods (e.g., as in US 4,816,567). Monoclonal antibodies may also be isolated from phage antibody libraries using techniques as described by Clackson et al. 1991 (Nature 352: 624-628) and Marks et al. 1991 (J Mol Biol 222: 581-597), for example.

Antibody binding agents may be antibody fragments. "Antibody fragments" comprise a portion of an intact antibody, comprising the antigen-binding or variable region thereof. Examples of antibody fragments include Fab, Fab', F(ab')2, Fv and scFv fragments; diabodies; linear antibodies; single-chain antibody molecules; and multivalent and/or multispecific antibodies formed from antibody fragment(s), e.g., dibodies, tribodies, and multibodies. The above designations Fab, Fab', F(ab')2, Fv, scFv *etc.* are intended to have their art-established meaning.

The term antibody includes antibodies originating from or comprising one or more portions derived from any animal species, preferably vertebrate species, including, e.g., birds and mammals. Without limitation, the antibodies may be chicken, turkey, goose, duck, guinea fowl, quail or pheasant. Also without limitation, the antibodies may be human, murine (e.g., mouse, rat, etc.), donkey, rabbit, goat, sheep, guinea pig, camel (e.g., *Camelus bactrianus* and *Camelus dromaderius*), llama (e.g., *Lama paccos, Lama glama or Lama vicugna*) or horse.

A skilled person will understand that an antibody can include one or more amino acid deletions, additions and/or substitutions (e.g., conservative substitutions), insofar such alterations preserve its binding of the respective antigen. An antibody may also include one or more native or artificial modifications of its constituent amino acid residues (e.g., glycosylation, *etc*.)*.*

Methods of producing polyclonal and monoclonal antibodies as well as fragments thereof are well known in the art, as are methods to produce recombinant antibodies or fragments thereof (see for example, Harlow and Lane, "Antibodies: A Laboratory Manual", Cold Spring Harbour Laboratory, New York, 1988; Harlow and Lane, "Using Antibodies: A Laboratory Manual", Cold Spring Harbour Laboratory, New York, 1999, ISBN 0879695447; "Monoclonal Antibodies: A Manual of Techniques", by Zola, ed., CRC Press 1987, ISBN 0849364760; "Monoclonal Antibodies: A Practical Approach", by Dean & Shepherd, eds., Oxford University Press 2000, ISBN 0199637229; Methods in Molecular Biology, vol. 248: "Antibody Engineering: Methods and Protocols", Lo, ed., Humana Press 2004, ISBN 1588290921).

The term "aptamer" refers to single-stranded or double-stranded oligo-DNA, oligo-RNA or oligo-DNA/RNA or any analogue thereof that specifically binds to a target molecule such as a peptide. Advantageously, aptamers display fairly high specificity and affinity (e.g., K_{A} in the order 1×10⁹ M⁻¹) for their targets. Aptamer production is described *inter alia* in US 5,270,163; Ellington & Szostak 1990 (Nature 346: 818-822); Tuerk & Gold 1990 (Science 249: 505-510); or "The Aptamer Handbook: Functional Oligonucleotides and Their Applications", by Klussmann, ed., Wiley-VCH 2006, ISBN 3527310592. The term "photoaptamer" refers to an aptamer that contains one or more photoreactive functional groups that can covalently bind to or crosslink with a target molecule. The term "spiegelmer" refers to an aptamer which includes L-DNA, L-RNA, or other left-handed nucleotide derivatives or nucleotide-like molecules. Aptamers containing left-handed nucleotides are resistant to degradation by naturally occurring enzymes, which normally act on substrates containing right-handed nucleotides. The term "peptidomimetic" refers to a non-peptide agent that is a topological analogue of a corresponding peptide. Methods of rationally designing peptidomimetics of peptides are known in the art. For example, the rational design of three peptidomimetics based on the sulphated 8-mer peptide CCK26-33, and of two peptidomimetics based on the 11-mer peptide Substance P, and related peptidomimetic design principles, are described in Horwell 1995 (Trends Biotechnol 13: 132-134). The term "small molecule" refers to compounds, preferably organic compounds, with a size comparable to those organic molecules generally used in pharmaceuticals. The term excludes biological macromolecules (e.g., proteins, peptides, nucleic acids, *etc*.). Preferred small organic molecules range in size up to about 5000 Da, *e.g.,* up to about 4000, preferably up to 3000 Da, more preferably up to 2000 Da, even more preferably up to about 1000 Da, e.g., up to about 900, 800, 700, 600 or up to about 500 Da. The term "oligonucleotide" as used herein refers to a nucleic acid (including nucleic acid analogues and mimetics) oligomer or polymer as defined herein. Preferably, an oligonucleotide, such as more particularly an antisense oligonucleotide, is (substantially) single-stranded. Oligonucleotides as intended herein may be preferably between about 10 and about 100 nucleoside units (i.e., nucleotides or nucleotide analogues) in length, preferably between about 15 and about 50, more preferably between about 20 and about 40, also preferably between about 20 and about 30. Oligonucleotides as intended herein may comprise one or more or all non-naturally occurring heterocyclic bases and/or one or more or all non-naturally occurring sugar groups and/or one or more or all non-naturally occurring inter-nucleoside linkages, the inclusion of which may improve properties such as, for example, increased stability in the presence of nucleases and increased hybridization affinity, increased tolerance for mismatches, etc. Nucleic acid binding agents, such as oligonucleotide binding agents, are typically at least partly antisense to a target nucleic acid of interest. The term "antisense" generally refers to an agent (e.g., an oligonucleotide) configured to specifically anneal with (hybridise to) a given sequence in a target nucleic acid, such as for example in a target DNA or RNA, and typically comprises, consist essentially of or consist of a nucleic acid sequence that is complementary or substantially complementary to said target nucleic acid sequence. Antisense agents suitable for use herein may typically be capable of annealing with (hybridising to) the respective target nucleic acid sequences at high stringency conditions, and capable of hybridising specifically to the target under physiological conditions. The terms "complementary" or "complementarity" as used herein with reference to nucleic acids, refer to the normal binding of single-stranded nucleic acids under permissive salt (ionic strength) and temperature conditions by base pairing, preferably Watson-Crick base pairing. By means of example, complementary Watson-Crick base pairing occurs between the bases A and T, A and U or G and C. For example, the sequence 5'-A-G-U-3' is complementary to sequence 5'-A-C-U-3'. The reference to oligonucleotides may in particular but without limitation include hybridisation probes and/or amplification primers and/or sequencing primers, etc., as commonly used in nucleic acid detection technologies.

In some embodiments, reagents such as binding agents as taught herein may comprise a detectable label. The term "label" refers to any atom, molecule, moiety or biomolecule that may be used to provide a detectable and preferably quantifiable read-out or property, and that may be attached to or made part of an entity of interest, such as a binding agent. Labels may be suitably detectable by for example mass spectrometric, spectroscopic, optical, colourimetric, magnetic, photochemical, biochemical, immunochemical or chemical means. Labels include without limitation dyes; radiolabels such as ³²P, ³³P, ³⁵S, ¹²⁵I, ¹³¹I; electron-dense reagents; enzymes (e.g., horse-radish peroxidase or alkaline phosphatase as commonly used in immunoassays); binding moieties such as biotin-streptavidin; haptens such as digoxigenin; luminogenic, phosphorescent or fluorogenic moieties; mass tags; and fluorescent dyes alone or in combination with moieties that may suppress or shift emission spectra by fluorescence resonance energy transfer (FRET). In some embodiments, binding agents may be provided with a tag that permits detection with another agent (e.g., with a probe binding partner). Such tags may be, for example, biotin, streptavidin, his-tag, myc tag, maltose, maltose binding protein or any other kind of tag known in the art that has a binding partner. Example of associations which may be utilised in the probe:binding partner arrangement may be any, and includes, for example biotin:streptavidin, his-tag:metal ion (e.g., Ni²⁺), maltose:maltose binding protein, etc. The biomarker - binding agent conjugate may be associated with or attached to a detection agent to facilitate detection. Examples of detection agents include, but are not limited to, luminescent labels; colourimetric labels, such as dyes; fluorescent labels; or chemical labels, such as electroactive agents (e.g., ferrocyanide); enzymes; radioactive labels; or radiofrequency labels. The detection agent may be a particle. Examples of such particles include, but are not limited to, colloidal gold particles; colloidal sulphur particles; colloidal selenium particles; colloidal barium sulfate particles; colloidal iron sulfate particles; metal iodate particles; silver halide particles; silica particles; colloidal metal (hydrous) oxide particles; colloidal metal sulfide particles; colloidal lead selenide particles; colloidal cadmium selenide particles; colloidal metal phosphate particles; colloidal metal ferrite particles; any of the above-mentioned colloidal particles coated with organic or inorganic layers; protein or peptide molecules; liposomes; or organic polymer latex particles, such as polystyrene latex beads. Preferable particles may be colloidal gold particles.

The present methods or uses may involve comparing the quantity of the one or more biomarker measured in a sample from a subject with a suitable reference value, wherein said reference value represents a known outcome.

Hence, in certain embodiments not falling within the scope of the claimed subject-matter, the methods or uses may comprise:
(a) determining the quantity of RoxP in the skin sample from the subject;
(b) comparing the determined quantity of RoxP with a reference value, said reference value representing a known diagnosis of the oxidative stress-associated skin disease;
(c) finding a deviation or no deviation of the determined quantity of RoxP from said reference value; and
(d) attributing said finding of deviation or no deviation to a particular diagnosis of the oxidative stress-associated skin disease in the subject.

In certain embodiments, not falling within the scope of the claimed subject-matter, the methods or uses may comprise:
(a) determining the quantity *of C. acnes* in the skin sample from the subject;
(b) comparing the determined quantity of *C. acnes* with a reference value, said reference value representing a known diagnosis of the oxidative stress-associated skin disease;
(c) finding a deviation or no deviation of the determined quantity of *C. acnes* from said reference value; and
(d) attributing said finding of deviation or no deviation to a particular diagnosis of the oxidative stress-associated skin disease in the subject.

In certain embodiments, not falling within the scope of the claimed subject-matter, the methods or uses may comprise:
(a) determining the quantity of RoxP and the quantity of *C. acnes* in the skin sample from the subject;
(b) comparing the determined quantity of RoxP and the determined quantity *of C. acnes* with a reference value, said reference value representing a known diagnosis of the oxidative stress-associated skin disease;
(c) finding a deviation or no deviation of the determined quantity of RoxP and the determined quantity *of C. acnes* from said reference value;
(d) attributing said finding of deviation or no deviation to a particular diagnosis of the oxidative stress-associated skin disease in the subject.

In certain embodiments, not falling within the scope of the claimed subject-matter, the methods or uses may comprise:
(a) determining the quantity of RoxP in the skin sample from the subject;
(b) comparing the determined quantity of RoxP with a reference value, said reference value representing a known indication of the benefit of administration of RoxP or a live *C. acnes* strain secreting RoxP;
(c) finding a deviation or no deviation of the determined quantity of RoxP from said reference value; and
(d) attributing said finding of deviation or no deviation to a particular indication of whether the subject would benefit from administration of RoxP or a live *C. acnes* strain secreting RoxP.

In certain embodiments, not falling within the scope of the claimed subject-matter, the methods or uses may comprise:
(a) determining the quantity *of C. acnes* in the skin sample from the subject;
(b) comparing the determined quantity of *C. acnes* with a reference value, said reference value representing a known indication of the benefit of administration of RoxP or a live *C. acnes* strain secreting RoxP;
(c) finding a deviation or no deviation of the determined quantity of *C. acnes* from said reference value; and
(d) attributing said finding of deviation or no deviation to a particular indication of whether the subject would benefit from administration of RoxP or a live *C. acnes* strain secreting RoxP.

In certain embodiments, not falling within the scope of the claimed subject-matter, the methods or uses may comprise:
(a) determining the quantity of RoxP and the quantity of *C. acnes* in the skin sample from the subject;
(b) comparing the determined quantity of RoxP and the determined quantity of *C. acnes* with a reference value, said reference value representing a known indication of the benefit of administration of RoxP or a live *C. acnes* strain secreting RoxP;
(c) finding a deviation or no deviation of the determined quantity of RoxP and the determined quantity *of C. acnes* from said reference value;
(d) attributing said finding of deviation or no deviation to a particular indication of whether the subject would benefit from administration of RoxP or a live *C. acnes* strain secreting RoxP.

Reference values may be established according to known procedures previously employed for other biomarkers. For example, a reference value may be established in an individual or a population of individuals characterised by a particular, known diagnosis of the stress-associated skin disease, or a particular, known indication of the benefit of administration of RoxP or a live *C. acnes* strain secreting RoxP. Such population may comprise without limitation ≥ 2, ≥ 5, ≥ 10, ≥ 50, ≥ 100, or even more individuals. As mentioned before, when two or more biomarkers are to be evaluated, each biomarker may be evaluated separately and independently, or one may generate a profile from the values or quantities for the two or more biomarkers. Accordingly, any reference value as referred to herein may also encompass a reference profile.

By means of example, a method for establishing a reference value of the quantity of RoxP and/or *of C. acnes,* said reference value representing:
(a) a diagnosis that a subject has an oxidative stress-associated skin disease, or
(b) a diagnosis that a subject does not have an oxidative stress-associated skin disease, may comprise:
   (i) measuring the quantity of RoxP and/or *of C. acnes* in:
      a. one or more skin samples from one or more subjects diagnosed as having the oxidative stress-associated skin disease, or
      b. one or more skin samples from one or more subjects diagnosed as not having the oxidative stress-associated skin disease, and
   (ii) storing the quantity of RoxP and/or of *C. acnes:*
      a. as measured in "(i) a." as the reference value representing the diagnosis that the subject has the oxidative stress-associated skin disease, or
      b. as measured in "(i) b." as the reference value representing the diagnosis that the subject does not have the oxidative stress-associated skin disease.

By means of example, a method for establishing a reference value of the quantity of RoxP and/or *of C. acnes,* said reference value representing:
(a) an indication that a subject will benefit from administration of RoxP or a live *C. acnes* strain secreting RoxP, or
(b) an indication that a subject will not benefit from administration of RoxP or a live *C. acnes* strain secreting RoxP,
may comprise:
(i) measuring the quantity of RoxP and/or of *C. acnes* in one or more skin samples from a plurality of subjects (such as preferably subjects not having an oxidative stress-associated disease) and determining the average or median quantity of RoxP and/or of *C. acnes* in said skin samples, and
(ii) storing a quantity of RoxP and/or of *C. acnes:*
   a. lower than the average or median quantity as measured in "(i)." as the reference value indicating that a subject will benefit from administration of RoxP or a live *C. acnes* strain secreting RoxP, or
   b. equal to or higher than the average or median quantity as measured in "(i)." as the reference value indicating that a subject may not benefit from administration of RoxP or a live *C. acnes* strain secreting RoxP.

Comparisons between values, e.g., a value obtained in a sample and a reference value, may generally include any means to determine the presence or absence of at least one difference and optionally of the size of such difference between values being compared. A comparison may include a visual inspection, an arithmetical or statistical comparison of measurements. Such statistical comparisons include, but are not limited to, applying a rule.

In an embodiment, not falling within the scope of the claimed subject-matter, reference value(s) as intended herein may convey absolute quantities of the biomarkers as intended herein. In another embodiment, the quantity of the biomarkers in a sample from a tested subject may be determined directly relative to the reference value (e.g., in terms of increase or decrease, or fold-increase or fold-decrease). Advantageously, this may allow the comparison of the quantity of the biomarkers in the sample from the subject with the reference value (in other words to measure the relative quantity of the biomarkers in the sample from the subject vis-à-vis the reference value) without the need first to determine the respective absolute quantities of the biomarkers.

A "deviation" of a first value from a second value may generally encompass any direction (*e.g.,* increase: first value > second value; or decrease: first value < second value) and any extent of alteration.

For example, a deviation may encompass a decrease in a first value by, without limitation, at least about 10% (about 0.9-fold or less), or by at least about 20% (about 0.8-fold or less), or by at least about 30% (about 0.7-fold or less), or by at least about 40% (about 0.6-fold or less), or by at least about 50% (about 0.5-fold or less), or by at least about 60% (about 0.4-fold or less), or by at least about 70% (about 0.3-fold or less), or by at least about 80% (about 0.2-fold or less), or by at least about 90% (about 0.1-fold or less), relative to a second value with which a comparison is being made.

For example, a deviation may encompass an increase of a first value by, without limitation, at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 150% (about 2.5-fold or more), or by at least about 200% (about 3-fold or more), or by at least about 500% (about 6-fold or more), or by at least about 700% (about 8-fold or more), or like, relative to a second value with which a comparison is being made.

Preferably, a deviation may refer to a statistically significant observed alteration. For example, a deviation may refer to an observed alteration which falls outside of error margins of reference values in a given population (as expressed, for example, by standard deviation or standard error, or by a predetermined multiple thereof, e.g., ±1xSD or ±2xSD, or ±1xSE or ±2xSE). Deviation may also refer to a value falling outside of a reference range defined by values in a given population (for example, outside of a range which comprises ≥40%, ≥ 50%, ≥60%, ≥70%, ≥75% or ≥80% or ≥85% or ≥90% or ≥95% or even ≥100% of values in said population).

In a further embodiment, a deviation may be concluded if an observed alteration is beyond a given threshold or cut-off. Such threshold or cut-off may be selected as generally known in the art to provide for a chosen sensitivity and/or specificity of the prediction methods, e.g., sensitivity and/or specificity of at least 50%, or at least 60%, or at least 70%, or at least 80%, or at least 85%, or at least 90%, or at least 95%.

For example, receiver-operating characteristic (ROC) curve analysis can be used to select an optimal cut-off value of the quantity of a given biomarker based on acceptable sensitivity and specificity, or related performance measures which are well-known *per se,* such as positive predictive value (PPV), negative predictive value (NPV), positive likelihood ratio (LR+), negative likelihood ratio (LR-), Youden index, or similar.

Such comparison may reveal the presence or absence of a deviation or no deviation between the quantity of any one or more biomarker as taught herein measured in a sample from a subject and a given reference value.

In certain preferred embodiments, not falling within the scope of the claimed subject-matter, the quantity of RoxP in a skin sample from a subject may be reduced compared to a reference value representing the diagnosis of the absence of an oxidative stress-associated skin disease (i.e., healthy state). The so-reduced quantity may allow for the diagnosis that the subject has the oxidative stress-associated skin disease and/or will benefit from administration of RoxP or a live *C. acnes* strain secreting RoxP.

In certain preferred embodiments, not falling within the scope of the claimed subject-matter, the quantity of *C. acnes* in a skin sample from a subject may be reduced compared to a reference value representing the diagnosis of the absence of an oxidative stress-associated skin disease (i.e., healthy state). The so-reduced quantity may allow for the diagnosis that the subject has the oxidative stress-associated skin disease and/or will benefit from administration of RoxP or a live *C. acnes* strain secreting RoxP.

In certain preferred embodiments, not falling within the scope of the claimed subject-matter, the quantity of RoxP in a skin sample from a subject may be reduced compared to the mean or median quantity of RoxP in a population of healthy subjects. The so-reduced quantity may allow to conclude that the subject has or is at a risk of developing an oxidative stress-associated skin disease and/or will benefit from administration of RoxP or a live *C. acnes* strain secreting RoxP.

In certain preferred embodiments, not falling within the scope of the claimed subject-matter, the quantity of *C. acnes* in a skin sample from a subject may be reduced compared to the mean or median quantity *of C. acnes* in a population of healthy subjects. The so-reduced quantity may allow to conclude that the subject has or is at a risk of developing an oxidative stress-associated skin disease and/or will benefit from administration of RoxP or a live *C. acnes* strain secreting RoxP.

In certain preferred embodiments, not falling within the scope of the claimed subject-matter, the quantity of *C. acnes* in a skin sample may be assessed relative to other microbial, and more particularly bacterial, taxa, such as genera or species (e.g., % abundance, such as % abundance by deep sequencing of 16S rRNA gene) in that skin sample. For example, relative to one or more genera selected from *Kocuria*, *Corynebacterium, Micrococcus,* or *Staphylococcus.* For example, the relative abundance (%) of *C. acnes* in a skin sample from a subject may be reduced compared to the mean or median relative abundance (%) quantity of *C. acnes* in a population of healthy subjects. The so-reduced relative abundance may allow to conclude that the subject has or is at a risk of developing an oxidative stress-associated skin disease and/or will benefit from administration of RoxP or a live *C. acnes* strain secreting RoxP.

In certain preferred embodiments, not falling within the scope of the claimed subject-matter, the quantity of *C. acnes* types or subtypes may be assessed relative to other *C. acnes* types or subtypes (e.g., % abundance, such as % abundance by deep sequencing of 16S rRNA gene) in a skin sample. Hence, in certain embodiments, the methods and uses may further comprise determining the relative quantity of two or more *C. acnes* types (e.g., phylotypes), MLST types, SLST types, or strains in the skin sample from the subject. For example, the *C. acnes* type I versus type II abundance (e.g., ratio type I / type II) in a skin sample from a subject may be reduced compared to the mean or median *C. acnes* type I versus type II abundance in a population of healthy subjects. The so-reduced *C. acnes* type I versus type II abundance may allow to conclude that the subject has or is at a risk of developing an oxidative stress-associated skin disease and/or will benefit from administration of RoxP or a live *C. acnes* strain secreting RoxP.

In certain preferred embodiments, not falling within the scope of the claimed subject-matter, the quantity of one or more RoxP promoter sequences, RoxP -35 box sequences, and/or RoxP -10 box sequences (for example one or more RoxP promoter sequences, RoxP -35 box sequences, and/or RoxP -10 box sequences shown in **Fig. 8**) may be assessed in absolute terms or relative to all or relative to one or more or all other RoxP promoter sequences, RoxP -35 box sequences, and/or RoxP -10 box sequences (for example relative to all or relative to one or more or all other RoxP promoter sequences, RoxP -35 box sequences, and/or RoxP -10 box sequences shown in **Fig. 8**). For example, the quantity of one or more RoxP promoter sequences associated with comparatively high RoxP expression, such as those of SEQ ID NO: 7-12 in **Fig. 8****,** may be assessed in absolute terms or relative to all RoxP promoter sequences or relative to one or more or all RoxP promoter sequences associated with low RoxP comparatively low RoxP expression, such as those of SEQ ID NO: 5-6 in **Fig. 8**. Such data is informative of the ability of the C. *acnes* present in the skin sample to produce RoxP.

In certain preferred embodiments, not falling within the scope of the claimed subject-matter, the quantity of one or more RoxP coding sequences (for example one or more RoxP coding sequence types shown in **Fig. 10**) may be assessed in absolute terms or relative to all RoxP coding sequences or relative to one or more or all other RoxP coding sequences (for example relative to all RoxP coding sequence types or relative to one or more or all other RoxP coding sequence types shown in **Fig. 10****).** For example, the quantity of one or more RoxP coding sequence types belonging to the second type as characterised in **Fig. 10** (e.g., containing KPA171202 RoxP coding sequence), may be assessed in absolute terms or relative to all RoxP coding sequence types characterised in **Fig. 10****,** or relative to one or both of the first type and third type as characterised in **Fig. 10****.** Such data is informative of the ability of the *C. acnes* present in the skin sample to produce RoxP.

The absolute quantity of one or more RoxP promoter types and/or RoxP coding sequence types in a skin sample may be determined by known quantitative methods for nucleic acid measurements in biological samples, mentioned elsewhere in this specification, such as without limitation quantitative PCR, such as droplet digital PCR. The relative quantity of one or more RoxP promoter types and/or RoxP coding sequence types in a skin sample may be determined by known methods such as amplification of promoter or coding sequence amplicons and sequencing typically at high sequencing depth, and quantitative allocation of the sequence reads to said one or more RoxP promoter types and/or RoxP coding sequence types.

Hence, also disclosed is method for diagnosing oxidative stress-associated skin disease in a subject or for determining whether a subject would benefit from administration of RoxP or a live *C. acnes* strain secreting RoxP, the method comprising determining the absolute or relative quantity of one or more RoxP coding sequence types and/or RoxP promoter types in a skin sample from the subject.

In certain preferred embodiments, not falling within the scope of the claimed subject-matter, the quantity of RoxP secreted by *C. acnes* measured in a skin sample may also be assessed. Hence, in certain embodiments, the methods and uses may further comprise determining the quantity of RoxP secreted by the *C. acnes* in the skin sample from the subject.

For example, the overall amount of RoxP produced by *C. acnes* or the relative abundance of high-RoxP expressing *C. acnes* types, subtypes or strains versus average- or-low RoxP expressing *C. acnes* types, subtypes or strains in a skin sample from a subject may be reduced compared to the corresponding measurements in a population of healthy subjects. The so-reduced overall amount of RoxP produced by *C. acnes* or the relative abundance of high-RoxP expressing *C. acnes* types, subtypes or strains may allow to conclude that the subject has or is at a risk of developing an oxidative stress-associated skin disease and/or will benefit from administration of RoxP or a live *C. acnes* strain secreting RoxP.

In certain embodiments, the present methods and uses may also comprise affected versus unaffected skin areas of the same subject.

Hence, certain embodiments of the method, not falling within the scope of the claimed subject-matter, for diagnosing oxidative stress-associated skin disease in a subject may comprise:
a) determining the quantity of RoxP in a sample from an area of the subject's skin suspected of being affected by an oxidative stress-associated skin disease;
b) determining the quantity of RoxP in a sample from a healthy area of the subject's skin; and
c) comparing the RoxP quantities determined in steps a) and b);
wherein a lower quantity of RoxP determined in step a) compared to the quantity of RoxP determined in step b) indicates that the subject has or is at risk of having an oxidative stress-associated skin disease.

Certain other embodiments, not falling within the scope of the claimed subject-matter, of the method for diagnosing oxidative stress-associated skin disease in a subject may comprise:
a) determining the amount of *C. acnes* type I, type II and type III strains in a sample from an area of the subject's skin suspected of being affected by an oxidative stress-associated skin disease;
b) determining the amount of *C. acnes* type I, type II and type III strains in a sample from a healthy area of the subject's skin; and
c) comparing the amounts of *C. acnes* type I, type II and type III strains determined in steps a) and b);
wherein a lower relative amount of *P. acnes* type I strains in the sample from an area of the subject's skin suspected of being affected by an oxidative stress-associated skin disease compared to the relative amount of *P. acnes* type I strains in the sample from a healthy area of the subject's skin indicates that the subject has or is at risk of having an oxidative stress-associated skin disease.

Also contemplated are companion diagnostic and personalised medicine approaches which employ the present diagnostic or benefit-evaluation methods or uses to select subjects as having or being at risk of having an oxidative stress-associated skin disease, or as ones that would benefit from administration of a live *C. acnes* strain secreting RoxP or from administration of RoxP. The so-selected subjects can undergo the prophylactic or therapeutic interventions disclosed herein comprising the administration of a live *C. acnes* strain secreting RoxP or administration of RoxP.

Hence, an aspect provides a composition comprising a live *C. acnes* strain secreting RoxP for use in a method of preventing or treating an oxidative stress-associated skin disease in a subject, wherein the subject has been selected as having or being at risk of having an oxidative stress-associated skin disease by the methods disclosed herein. A related aspect provides a method for preventing or treating an oxidative stress-associated skin disease in a subject in need thereof, the method comprising administering to the skin of the subject a prophylactically or therapeutically effective amount of a composition comprising a live *C. acnes* strain secreting RoxP, wherein the subject has been selected as having or being at risk of having an oxidative stress-associated skin disease by the methods disclosed herein. Another related aspect provides use of a composition comprising a live *C. acnes* strain secreting RoxP for the manufacture of a medicament for use in a method of preventing or treating an oxidative stress-associated skin disease in a subject, wherein the subject has been selected as having or being at risk of having an oxidative stress-associated skin disease by the methods disclosed herein. A further related aspect provides use of a composition comprising a live *C. acnes* strain secreting RoxP for preventing or treating an oxidative stress-associated skin disease in a subject, wherein the subject has been selected as having or being at risk of having an oxidative stress-associated skin disease by the methods disclosed herein.

Another aspect, not falling within the scope of the claimed subject-matter, provides a method comprising administering a live *C. acnes* strain secreting RoxP to a subject, wherein the subject has been selected as one that would benefit from administration of a live *C. acnes* strain secreting RoxP by the methods disclosed herein.

Another aspect provides a composition comprising RoxP for use in a method of preventing or treating an oxidative stress-associated skin disease in a subject, wherein the subject has been selected as having or being at risk of having an oxidative stress-associated skin disease by the methods disclosed herein, and wherein the oxidative stress-associated skin disease is selected from the group comprising actinic keratosis (AK) and basal cell carcinoma (BCC). A related aspect provides a method for preventing or treating an oxidative stress-associated skin disease in a subject in need thereof, the method comprising administering to the skin of the subject a prophylactically or therapeutically effective amount of a composition comprising RoxP, wherein the subject has been selected as having or being at risk of having an oxidative stress-associated skin disease by the methods disclosed herein, and wherein the oxidative stress-associated skin disease is selected from the group comprising actinic keratosis (AK) and basal cell carcinoma (BCC). Another related aspect provides use of a composition comprising RoxP for the manufacture of a medicament for use in a method of preventing or treating an oxidative stress-associated skin disease in a subject, wherein the subject has been selected as having or being at risk of having an oxidative stress-associated skin disease by the methods disclosed herein, and wherein the oxidative stress-associated skin disease is selected from the group comprising actinic keratosis (AK) and basal cell carcinoma (BCC). A further related aspect provides use of a composition comprising RoxP for preventing or treating an oxidative stress-associated skin disease in a subject, wherein the subject has been selected as having or being at risk of having an oxidative stress-associated skin disease by the methods disclosed herein, and wherein the oxidative stress-associated skin disease is selected from the group comprising actinic keratosis (AK) and basal cell carcinoma (BCC).

A further aspect, not falling within the scope of the claimed subject-matter, provides a method comprising administering a composition comprising RoxP thereof to a subject, wherein the subject has been selected as one that would benefit from administration of RoxP by the methods disclosed herein.

Hence, the present application also provides aspects and embodiments as set forth in the appended set of claims.

While the invention has been described in conjunction with specific embodiments thereof, it is evident that many alternatives, modifications, and variations will be apparent to those skilled in the art in light of the foregoing description.

The herein disclosed aspects and embodiments of the invention are further supported by the following non-limiting examples.

### EXAMPLES

### Materials and methods used in Examples 1 to 6

### Bacterial strains and cultivation

*Cutibacterium acnes* isolates, representative of type I, II and III strains, were grown at 37°C under anaerobic conditions in Wilkins-Chalgren anaerobe broth (WC) until reaching either exponential (1-3 days) or stationary phase (5-7 days). Further information about specific *C. acnes* strains and their isolation can be found in Homberg et al. 2009 Clin Microbiol Infect 15:787-95, and information about their respective RoxP homologs can be found in Allhorn et al. 2016 Sci Rep 6:36412. The isolates for example included *C. acnes* strain KPA171202, publically available from Leibniz Institute DSMZ-German Collection of Microorganisms and Cell Cultures under accession number DSM-16379. The complete genome of C. *acnes* KPA171202 has been annotated in Genbank under accession numbers NC_006085.1/AE017283.1. The isolates further included *C*. *acnes* subsp. *defendens* strain publically available from American Type Culture Collection (ATCC) under accession number ATCC 11828. The complete genome *of C. acnes* ATCC 11828 has been annotated in Genbank under accession numbers NC_017550.1/CP003084.1. The isolates further included *C. acnes* strain publically available from ATCC under accession number ATCC 6919. The complete genome of *C. acnes* ATCC 6919 has been annotated in Genbank under accession numbers NZ_CP023676.1/CP023676.1. The isolates further included *C. acnes* strain publically available from DSMZ under accession number DSM 1897. The isolates further included the following *C. acnes* strains the complete genomes of which have been annotated in Genbank under the respective accession numbers: SK137 (NC_014039.1/CP001977.1), 266 (NC_017534.1/CP002409.1), 6609 (NC_017535.1/CP002815.1), P.acn33 (NC_016516.1/CP003195.1), P.acn17 (NC_016512.1/CP003196.1), P.acn31 (NC_016511.1/CP003197.1), C1 (NC_018707.1/CP003877.1), HL096PA1 (NC_021085.1/CP003293.1), hdn-1 (NZ_CP006032.1/CP006032.1), KCOM 1861 (= ChDC B594) (NZ_CP012647.1/CP012647.1), PA_15_1_R1 (NZ_CP012355.1/CP012355.1), PA_21_1_L1 (NZ_CP012351.1/CP012351.1), PA_15_2_L1 (NZ_CP012352.1/CP012352.1), PA_12_1_L1 (NZ_CP012354.1/CP012354.1), KCOM 1315 (NZ_CP031442.1/CP031442.1), PA_30_2_L1 (NZ_CP012350.1/CP012350.1), PA_12_1_R1 (NZ_CP012353.1/CP012353.1), A1-14 (NZ_CP013693.1/CP013693.1). In total, 155 *C. acnes* isolates or genome sequences available from the above sources have been analysed.

### qPCR analysis of roxP gene expression

The RNA content of exponential and/or stationary phase *C. acnes* cultures was extracted using the RNeasy^{®} Mini Kit (Qiagen, Hilden, Germany) complemented with Bacteria Protect Reagent (Qiagen), following the manufacturer's instructions. In short, *C. acnes* cultures were mixed with an equal volume Bacteria Protect Reagent to ensure RNA stabilization prior to disruption of membrane structures. Cell lysis was subsequently achieved through addition of TE-buffer (10 mM Tris-HCl, 1 mM EDTA, pH 8.0) supplemented with 15 mg/ml lysozyme (Sigma-Aldrich, Saint Louis, MO) and proteinase K (Qiagen). Samples were thereafter treated with β-mercaptoethanol-supplemented buffer RLT (RNeasy Mini Kit), ethanol, and added to RNeasy spin columns according to manufacturer's protocol. Resulting RNA concentrations were measured using NanoDrop^{®} (Saveen Werner, Limhamn, Sweden) and samples stored at - 20°C until later use.

The relative abundance of *roxP* and *gapdh* transcripts was determined through quantitative real-time PCR (qPCR) using Power SYBR^{®} Green RNA-to-Ct 1-step Master Mix (ThermoFisher Scientific, Waltham, MA) mixed 1:1 (v/v) with 10 ng RNA and 100 nM primers in nuclease free water:
*roxP*:
   5'-GCATCTAGCCCTCTCACCAT-3' (SEQ ID NO: 13) and
   5' -CTGAGAGTCCGGTAGGTGGT-3' (SEQ ID NO: 14);
*gapdh*:
   5'-GCATCATGACTACCGTCCAC-3' (SEQ ID NO: 15) and
   5'-CGGTGGTCTCCTTAGAGGTC-3' (SEQ ID NO: 16).

Plates were run with reverse transcription for 30 min at 48°C, 10 min at 95°C, and 40 cycles of 15 seconds at 95°C alternating with 1 minute at 60°C, on an iCycler iQ^{®} (Bio-Rad, Hercules, CA). Results were interpreted via double delta Cq analysis, using the recorded values for *C. acnes* strain 266 as a reference. The experiment was run in biological and technological duplicates.

### Generation of a RoxP expressing yeast vector

A truncated version of *roxP* (amino acid residues 24-161, omitting the N-terminal signal peptide) from C. *acnes* strain KPA171202 was amplified (primers: 5'-GGCTGAAGCTGAATTCACACCCATCGATGAGAGCCAAC-3' (SEQ ID NO: 17) plus 5'-GATGATGATGGTCGACTCCTGCTGCGCCGTTGAGGGCGGGATCCACC-3' (SEQ ID NO: 18)) to generate a *roxP* fragment containing a C-terminal GAAG linker, and cloned into the EcoRI and SalI sites of the pPICZαA vector (ThermoFisher Scientific). Sequence verified linearized plasmids were used to transfect SuperMan5 *Pichia pastoris.*

### Expression of recombinant RoxP (rRoxP)

A generated SuperMan5 *Pichia pastoris* strain harbouring the formerly described vector, was aliquoted and stored at -80°C in YPD media supplemented with 50% (v/v) glycerol. For efficient expression of recombinant protein, thawed freeze cultures were grown in a Buffered Glycerol-complex medium (BM* (70%, v/v), 0.5 M potassium buffer pH 6.0 (10%, v/v), YNB (1.4%, w/v), >99% glycerol (1%, v/v), Biotin (4 × 10⁻⁷ %, w/v)) for 24 h prior to being resuspended in Buffered Methanol-complex medium (BM* (70%, v/v), 0.5 M potassium buffer pH 6.0 (10%, v/v), YNB (1.4%, w/v), 99.8% methanol (1.5%, v/v), Biotin (4 × 10⁻⁷%, w/v)). Cultures were subsequently maintained in a shaking incubator (30°C, 180 rpm) for 5 days with the continuous induction of *roxP* achieved through addition of methanol (1% v/v) every 24 h. *BM: Yeast extract (1.4%, w/v), Peptone (2.9%, w/v), CAS amino acids (1.4%, w/v).

### Purification of rRoxP

Ensuing a 10 min centrifugation at 800 × g and passage through a 0.22 µm filter, the yeast supernatant was loaded onto nickel columns (His Gravitrap^{™}, GE Healthcare, Chicago, IL) and eluted according to the manufacturer's instructions, using an imidazole buffer (20 mM sodium phosphate pH 7.4, 500 mM NaCl, 500 mM imidazole). Purified protein was immediately re-buffered in sodium phosphate (20 mM, pH 7.4) by means of dialysis (MWCO: 6-8 kDa, Novagen^{®}), after which the protein concentration was spectrophotometrically determined through NanoDrop^{®} (Saveen Werner) and sample purity via a 15% SDS-PAGE.

### Cryogelation

The method used for cryogelation has been previously described by Ertürk et al. (2013 J Mol Recognit 26:633-642). Briefly, the functional monomer N-Methacryloyl-1-histidine methyl ester (1 mM) and template RoxP (1 mM) were dissolved in deionized water for pre-complexation. 2-hydroxyethyl methacrylate (20 mM) was simultaneously prepared (1:1 v/v). Monomer solutions were then mixed with an equal volume cross-linking agent, N,N'-Methylenebisacrylamide (final concentration 90 mM), and the reaction mixture purged with nitric oxide. Polymerization was subsequently initialized by the addition of ammonium persulfate (4.4 mM) and N,N,N',N'-tetramethylene ethylenediamine (0.125%). The resulting solution was aliquoted into 4 × 5 ml plastic syringes (Ø = 1.3 cm) with closed lower outlets and immediately placed in 14°C for 24 h. After being thawed, columns were initially washed with H₂O and template protein later removed with an imidazole buffer (20 mM sodium phosphate, 500 mM NaCl, 500 mM imidazole, pH 7.4). Elution was discontinued when no RoxP could be detected in the flow through (spectrophotometrically determined at 280 nm).

### Purification of native RoxP

The main RoxP variant was purified from stationary phase *C. acnes* KPA171202 culture media, following a procedure of ammonium sulphate precipitation and ion-exchange chromatography that has been previously described (Allhorn et al. 2016 Sci Rep 6:36412). Purification of the less common variant from *C. acnes* AD24 culture media, required the use of a more sensitive technique. The ammonium sulphate (80%) protein fraction of stationary phase *C. acnes* AD24 was passed through a pre-equilibrated cryogel column using a peristaltic pump (1 mL/min). To enable absorption of native RoxP, the protein fraction was repeatedly passed through the gel under continuous stirring for 1 h. Bound RoxP was eluted using imidazole buffer (20 mM sodium phosphate, 500 mM NaCl, 500 mM imidazole, pH 7.4) and the resulting protein concentration and sample purity analysed through NanoDrop^{®} (Saveen Werner) and a 15% SDS-PAGE, respectively.

### ABTS radical cation assays

The reduction of pre-formed ABTS radical cations in the presence of RoxP was analysed using a spectrophotometric assay previously described by Re et al. (1999 Free Radic Biol Med 26:1231-1237). 2,2'-azinobis(3-ethylbenzothiazoline-6-sulfonic acid) diammonium salt (ABTS) (Sigma-Aldrich) was initially dissolved in ddH₂O to a concentration of 7 nM. Potassium persulfate (Merck KGaA, Darmstadt, Germany) was subsequently added to a final concentration of 2.45 mM and the resulting solution kept dark and incubated overnight at RT. Formed ABTS radicals were diluted with PBS (pH 7.4) until reaching an absorbance of approximately 0.7 at 734 nm. Antioxidants (1.7 µM) were added (1:9 v/v) and the absorbance spectrum 500 to 900 nm recorded after 30 sec.

### Cell culturing

Human keratinocytes (HaCat cells; disclosed in Boukamp et al. 1988 J Cell Biol 106:761-71 and available from Deutsches Krebsforschungszentrum, DKFZ, info@cell-lines-service.de; and also commercially available for example from Addexbio, San Diego, CA, catalogue number T0020001) were grown in SFM medium supplemented with L-glutamine, EGF (epidermal growth factor) and bovine pituitary extract (Gibson, Invitrogen) until 90% confluency in 96-well plates (Nunc A/S, Roskilde, Denmark). Human monocytes (THP-1 cells; disclosed in Tsuchiya et al. 1980 Int J Cancer 26:171-6; commercially available for example from Sigma-Aldrich, catalogue number 88081201; kindly provided by Dr Arne Egesten, Lund University) were cultured in RPMI 1640 medium supplemented with Glutamax-1 (Gibco^{®}, ThermoFisher Scientific), 100 µg/ml streptomycin, 100 µg/ml penicillin, and 10% v/v foetal calf serum (ThermoFisher Scientific). All cells were grown at 37°C in 5% CO₂ and 95% humidity.

### MTT assay

HaCat cells cultured to 90% confluency, or THP-1 cells (1.2 × 10⁶ cells/well) in a 96-well plate were treated with 2 mM paraquat (Sigma-Aldrich) and/or 5 µM RoxP for 24-48 h. HaCat cells were kept in their culture medium, while monocytes were transferred to a HEPES-HBSS (Hank's balanced salt solution) buffer at pH 7.4. Viability was measured by the addition of 0.5 mg/ml 3-(4,5-demethylthiazol-2-yl)-2,5-diphenyltetrazolium bromide (MTT) for 1-5 hours. The cells were washed with PBS followed by the addition of DMSO. The samples incubated for 30 minutes and absorbance at 550 nm was measured using a Wallac 1420 Multilabel Counter (Bio-Rad).

### Collection of skin swabs

Samples were collected between November 2016 and May 2017 at the Department of Dermatology at Lund University Hospital, Sweden. Patients (n = 54, aged between 50 - 70) presenting with clinically suspected actinic keratosis (AK) or superficial/nodular basal cell carcinoma (BCC) of the face, neck, upper chest or shoulder regions, were asked for participation.

**Table 1. Patient characteristics and selected baseline values. Patient groups were compared using a one-way ANOVA test. AK: Actinic keratosis. BCC: Basal cell carcinoma. * Statistically significant.**

| | | **Control** | **AK** | **BCC** | **P value** |
|---|---|---|---|---|---|
| Age | | | | | .058 |
| | Range | 51-70 | 53-69 | 50-70 | |
| | Mean ± SD | 61.56 ± 4.58 | 60.5 ± 5.52 | 64.61 ± 5.51 | |
| | Median | 61 | 60.5 | 66.5 | |

| Sex, n | | | | | .932 |
|---|---|---|---|---|---|
| | Male | 9 | 8 | 8 | |
| | Female | 9 | 10 | 10 | |

| Swab location | | | | | .**010*** |
|---|---|---|---|---|---|
| | Scalp | 0 | 1 | 0 | |
| | Forehead | 0 | 3 | 1 | |
| | Eyebrow | 0 | 4 | 0 | |
| | Temple | 0 | 1 | 2 | |
| | Nose | 0 | 0 | 5 | |
| | Cheek | 18 | 7 | 4 | |
| | Lip | 0 | 1 | 0 | |
| | Chest | 0 | 0 | 2 | |
| | Shoulder | 0 | 1 | 4 | |

Individuals were swabbed (Venturi Transystem, Copan, Italy) with buffer pre-soaked swabs for 30 sec at lesional regions (4 cm²) and for 30 sec at an anatomically identical healthy site (4 cm²), thus functioning as his/her own control. Swabs were gently shaken in their liquid storage solution, stored at 4°C overnight, and then prepared for analysis. Exclusion criteria included patients with: applied make up, newly washed face (<1h), diabetes, cognitive impairment, other active skin disorders, ulcerated tumors, immunosuppression, ongoing/newly ended antibiotic or corticosteroid treatment (systemic or topical, <4 weeks). The study was approved by the ethical committee in Malmö/Lund (2016/465), and in accordance with the Declaration of Helsinki. Written informed consent was obtained from all patients before enrolment.

### Analysis of RoxP concentration in skin swab samples using a capacitance biosensor

The abundance of RoxP in samples taken from either healthy volunteers or AK/BCC patients was analysed on a capacitive biosensor, applying a molecular imprinting technique previously described by Ertürk et al. (2018 PLoS ONE 13:e0193754). Accounting for any potential background, a calibration curve was initially obtained from RoxP spiked (0.14 - 0.71 mM) mock skin swabs (Venturi Transystem, Copan, Italy), diluted 1:100 (v/v) in sodium phosphate buffer (10 mM, pH 7.4). Cells and debris from patient swabs were subsequently removed through centrifugation (15 min, 4000 × g), supernatant diluted 1:100 (v/v) in sodium phosphate buffer, and eventually injected into the capacitive system in triplicates. The calibration curve was used for determination of RoxP concentrations in each sample prior to pooling and plotting as abundance in healthy skin, AK and BCC, respectively.

### Preparation of DNA from skin swabs

Cotton sticks with skin swabs from patients and healthy individuals were stored at 4°C overnight. Cellular material was collected from the liquid storage buffer through centrifugation (5000 g, 10 min), and DNA extracted using Qiagen DNeasy. Quantity and quality of DNA extractions were evaluated by NanoDrop^{®} (Saveen Werner).

### DNA sequencing and bioinformatics analysis

To determine the skin microbiome, the V1-V3 region of the 16S rRNA gene was amplified and amplicons were subjected to deep sequencing using Illumina technology, performed by Eurofins (Germany). To reduce the error rate of the reads, processing of raw reads from the Illumina platform was done using the Mothur software v1.39.1 and following the MiSeq SOP with some modifications. Initially, the paired-end reads were combined using the command "make.contig"(Kozich et al. 2013 Applied and environmental microbiology 79:5112-20). The combined reads were then trimmed off the primer sequence and sequences containing ambiguous bases or containing longer stretched of homopolymers than eight were discharged from further analysis. The reads were afterwards aligned against the Silva reference alignment v128 and the resulting alignment was filtered so that all reads only overlapped the same region. To further reduce the number of sequencing errors, a pre-clustering step implementing a pseudo-single linkage algorithm originally developed by Huse et al. (2010 Environmental microbiology 12(7):1889-98) with the "diff" variable set to four was performed. Chimeras were removed using the built-in version of the UCHIME algorithm in Mothur (Edgar et al. 2011 Bioinformatics 27:2194-200). Lastly, all singletons, duplicates, and triplicates were removed before final analysis to preclude inclusion of sequences from potential contamination or sequence errors that have not been detected in the previous steps. The resulting reads were then classified with the classify.seqs command using the Bayesian method and the taxonomic outline v14.51 from the Human Oral Microbiome Database (HOMD) (http://www.homd.org/) with a manual inclusion of relevant skin bacteria (Chen et al. 2010 Database : the journal of biological databases and curation baq013). The confidence cut-off was set to 80 %. Linear discriminant analysis (LDA) coupled with effect size measurement (LEfSe) to detect differentially abundant bacterial taxa (phyla, genera, species) between groups. The alpha values for the factorial Kruskal-Wallis and pairwise Wilcoxon tests were set to 0.05 and the LDA score threshold for discriminative features was set to 3.5. The online version of the LEfSe program was used (Segata et al. 2011 Genome Biology 12:R60).

To determine phylotypes of the C. *acnes* population in skin swab samples we used the SLST approach established previously (Scholz et al. 2014 PLoS ONE 9:e104199). The SLST amplicons were subjected to MiSeq sequencing, performed by Eurofins (Germany). All high-quality sequence read pairs were assigned to SLST alleles; all currently known SLST alleles are accessible on www.medbac.dk/slst/pacnes.

### Example 1: Expression of roxP is dependent on C. acnes phylotype

*roxP* expression rates were determined through quantitative real-time PCR (qPCR) in all major *C. acnes* phylotypes. *C. acnes* isolates identified as phylotype I displayed significantly higher expression levels of *roxP* compared to most phylotype II or III strains (**Fig. 1**). **Table 2** below presents the strain cohort utilised in the experiment underlying **Fig. 1**. Fold change represents the obtained values, normalised against gapdh and put in relation to the *roxP* expression noted from *C. acnes* strain 266.

**Table 2. Strain cohort utilised in the experiment underlying Fig. 1**

| Strain | Fold change | Phylotype/SLST | RoxP promoter variant according to **Fig. 8** |
|---|---|---|---|
| 266 | 1 | IA/A1 | PA12.1.L1-type |
| 12.1.L1 | 1,1604519 | IA/A1 | PA12.1.L1-type |
| 21.1.L1 | 1,527945494 | IB/H1 | KPA171202-type |
| 30.2.L1 | 1,54985506 | IA/D1 | PA15.1.R1-type |
| 15.1.R1 | 1,779504667 | IA/C1 | PA15.1.R1-type |
| 12.1.R1 | 1,790863582 | IA/A1 | PA12.1.L1-type |
| 09 - 323 | 0,003734342 | II/K8 | 09-09-type |
| 11 - 79 | 0,008892697 | II/K2 | 09-09-type |
| 09 - 09 | 0,025317342 | II/K1 | 09-09-type |
| 10 - 43 | 0,025606243 | II/K2 | 09-09-type |
| 09 - 23 | 1,947152474 | II/K1 | 09-109-type |
| PM H5 | 0,585279135 | III/L1 | PMH5-type |
| PM H7 | 0,839982175 | III/L1 | PMH5-type |

Sequencing of the respective promotor regions revealed the existence of 8 dominant sequences of the RoxP upstream region **(****Fig. 9****),** and further revealed revealed a base substitution in type II strains, affecting the -35 region of the predicted *roxP* promoter, potentially able to negatively influence transcription rates **(****Fig. 8****).** Comparison of 155 sequenced *C*. *acnes* genomes revealed that most phylotype I strains shared the same *roxP* upstream region, associated with high expression, while the *roxP* upstream region of most phylotype II strains was distinct **(****Fig. 9****).** - 35 sequences ACTTCGAT or ACTTCAAT appeared preferred for achieving comparatively higher RoxP expression. -10 sequence TGCTATACT appeared preferred for achieving comparatively higher RoxP expression, while -10 sequence TGCTACACT also achieved comparatively good RoxP expression.

Also aerobic/anaerobic growth capacities of the strains were investigated. Whereas an increased *roxP* expression, exhibited by most phylotype I strains, correlated with an ability to grow equally well in oxic and anoxic environments, low roxP expression resulted in reduced or delayed growth in oxygen-rich settings **(****Fig. 2****).**

### Example 2: RoxP homologs display comparable antioxidant activities

Although RoxP is highly conserved, with 99-100 % amino acid identity in the majority (approximately 93 %) of isolates, a subset of strains (approximately 7 %) classified as *C*. *acnes* phylotype II and III express a more distantly related protein, with approximately 83 % amino acid identity **(****Fig. 10****).** The reducing activity of different RoxP homologs was compared. The main variant could be successfully isolated from (type IB) *C*. *acnes* KPA171202 culture media, and the less common homolog from (type II) *C*. *acnes* AD24 culture media. The latter was purified using macroporous cryogels imprinted with recombinant RoxP **(****Fig. 11****),** after which the protein identity was verified through western blot analysis (data not shown). As demonstrated by a decreased absorbance at 734 nm, the two homologs were able to reduce the cationic ABTS⁺ substrate with equal efficiency and, in doing so, displayed highly similar antioxidant activities **(****Fig. 3****, A:** pre-formed ABTS+ radicals, **B**: pre-formed ABTS radicals plus NaCl). Although the variant more commonly expressed by phylotype I strains displayed a continuously higher activity, the difference was only minor and not statistically significant.

### Example 3: Antioxidant activity of RoxP is maintained in harsh conditions

RoxP displayed a concentration-dependent reducing activity **(****Fig. 4A****),** had a loss of activity in presence of CaCl₂ but withstood physiological concentrations of NaCl **(****Fig. 4B****).** The protein was furthermore able to endure temperatures of above 70°C without any significant loss of activity **(****Fig. 4C****),** as well as long-term storage at room temperature **(****Fig. 4D****).** These data show that RoxP remains active in physiological settings and exhibits remarkable stability under harsh conditions, attesting to its functionality on the skin and its usability as a biopharmaceutical agent.

### Example 4: RoxP can protect human cells from oxidative damage in vitro

To investigate the ability of RoxP to reduce free radicals in a biological system, we exposed human cells to oxidation (using paraquat) and investigated the ability of RoxP to rescue the cells from lethal oxidative stress. Even at a 1:400 ratio (RoxP:oxidizer) being added in a subsequent manner, RoxP completely inhibited the negative oxidative effect of paraquat **(****Fig. 5****).** The effect was more prominent in monocytes, while keratinocytes responded less well, both to oxidation and antioxidation **(**Fig. 5A-B). Notably, RoxP could also increase the overall viability of human cells in the absence of an added oxidizer.

### Example 5: RoxP is less abundant in skin disease associated with oxidative stress

RoxP protein abundances in actinic keratosis (AK) and basal cell carcinoma (BCC) was studied. Skin swabs (n = 54) were obtained from individuals with healthy skin, with the pre-cancerous condition AK and with developed BCC, at both disease-affected and non-affected regions. Samples were subsequently analysed using a highly sensitive capacitive biosensor, previously shown to detect concentrations of as little as 8.25 ng RoxP/cm² skin (Ertürk and Lood et al. 2018 J Vis Exp, doi: 10.3791/57208), and the recorded capacitance changes equilibrated to a pre-established calibration curve **(****Fig. 12****).** Results were analysed on a population **(****Fig. 6****)** and individual basis **(****Fig. 13****).** In the latter case, patients were excluded if accurate determination of RoxP concentration, in either affected or non-affected regions, were unsuccessful (data not shown). An overall higher RoxP abundance was observed among patients with BCC **(****Fig. 6****).** Individuals with AK showed a further RoxP concentration decline in diseased areas, while those with BCC displayed similar levels at both affected and non-affected sites **(****Fig. 13****).**

### Example 6: Dysbiosis in oxidative skin disease is characterized by diminished C. acnes prevalence

Bacterial composition was studied in AK and BCC at the genus, species and C. *acnes* phylotype level. Skin swabs, trailed by 16S rDNA amplicon deep sequencing, revealed markedly diminished *Propionibacterium* and elevated *Staphylococcus* abundances in diseased compared to healthy skin **(****Fig. 7A** and **Fig. 14****).** A drop in *C*. *acnes* prevalence during acute stages of oxidative disease (AK) seemingly proceeded the surge in staphylococcal species, particularly *S*. *aureus,* during later stages of disease (BCC) **(****Fig. 7A****).** Further characterisation of *C*. *acnes* populations through single locus sequence typing (SLST) revealed that the prevalence of specific *C*. *acnes* types was also shifted in AK- and, to a lesser extent, in BCC-affected skin (**Fig**. **7B**). Strains belonging to *C*. *acnes* type II could more commonly be isolated from diseased skin regions, while healthy skin areas displayed a closer-to-average predominance of type I strains.

### Materials and methods used in Examples 7 to 11

Synelvia SAS (Labège, France) was instructed to test the protective effect of RoxP-containing *C*. *acnes* supernatant (included as an active ingredient at 10% v/v in the medium) against cellular stressors in a reconstituted human epidermis (RHE) model. Relevant results of the analysis are set forth below.

### Active ingredient

The RoxP-containing *C*. *acnes* supernatant was prepared as follows. A H1 strain was grown in a 20L fermentor at 37°C for 3 days in a suitable medium. Then the supernatant was concentrated using Tangential flow filtrations. In a first step a 30kDa cut-off was used and in a second step a 3kDa cut-off was used. The final concentrate was analysed by gel filtration on a iProminence HPLC (Shimadzu, Kyoto, Japan) using TSKgel QC-PAK (Tosoh Bioscience, Griesheim, Germany). The concentrate was 80% pure RoxP and at a concentration of around 2mM (~34mg/ml). 5% v/v of pure glycerol was added to the product before it was stored at -80°C before using it in assays. This highly RoxP-enriched *C*. *acnes* supernatant was added as 10% v/v to the medium for experiments; the medium then contained about 0.16mM RoxP.

### Stressors and conditions

Ozone stress: Ozone production was done at 0.3 L/min by introducing a mixture of O₂ and O₃ (99/1% (v/v)) in the RHE (reconstituted human epidermis) culture medium (bubbling). This process produced about 1% of ozone which is equivalent to 4 ppm of ozone exposure.

The ozonolysis reaction induces several products called primary and secondary products according to Criegee rearrangement. The resulting primary ozonide decomposes into a primary carbonyl (an aldehyde or a ketone) and a carbonyl-O-oxide (often termed a Criegee intermediate). Secondary products react with the parent molecule under ozone to generate other degradation products. The ozonolysis reaction could also induce protein oxidation or DNA damage.

UV stress: RHEs were exposed to UV radiation (UVA + UVB) at 2 MED (Minimal Erythema Dose) using a Bio-Sun system (Vilber Lourmat, Collégien, France).

Photo-oxidation induces lipid and protein oxidation, DNA damage and generates specific oxidation/degradation products such as CPDs or 8-OHdG.

The following conditions have been tested.

Negative control 1: Unstressed RHEs without active ingredient. RHEs were produced during 10 days under 5% COz, 37°C in incubator and the medium was changed every two days until day 10. At day 10, RHEs and medium were recovered and stored at -20°C until analysis.

Negative control 2: Unstressed RHEs with active ingredient (RoxP-enriched *C*. *acnes* supernatant). RHEs were produced during 10 days under 5% COz, 37°C in incubator and the medium was changed every two days until day 8. At days 8, and 9, the active ingredient (RoxP-enriched *C*. *acnes* supernatant) was added in the medium at 10% v/v. At day 10, RHEs and medium were recovered and stored at -20°C until analysis.

Positive control 1: RHEs stressed with UV. RHEs were produced during 10 days under 5% COz, 37°C in incubator and the medium was changed every two days until day 8. At day 9, RHEs were introduced in the chamber and exposed at 2 MED (UVA: 9 J/cm² (365 nm) and UVB 300 mJ/cm² (312 nm)). After the stress, a new medium was added to the RHEs. At day 10, RHEs and medium were recovered and stored at -20°C until analysis.

Test 1: RHEs stressed with UV in contact with the active ingredient (RoxP-enriched *C*. *acnes* supernatant). RHEs were produced during 10 days under 5% COz, 37°C in incubator and the medium was changed every two days until day 8. At day 8, the active ingredient (RoxP-enriched *C*. *acnes* supernatant) was added in the medium at 10% v/v. At day 9, RHEs were introduced in the chamber and exposed at 2 MED (UVA: 9 J/cm2 (365 nm) and UVB 300 mJ/cm² (312 nm)) with the medium containing the active ingredient at 10% v/v. After the stress, a new medium containing the active ingredient at 10% v/v was added to RHE. At day 10, RHEs and medium were recovered and stored at -20°C until analysis.

Positive control 2: RHE stressed with ozone. RHEs were produced during 10 days under 5% COz, 37°C in incubator and the medium was changed every two days until day 8. At day 9, RHE were stressed with ozone (in the reaction chamber). The reaction medium was cooled at 5°C during the reaction with ozone (about 1 minute). After the stress, a new medium was added to RHE. At day 10, RHE and medium were recovered and stored at -20°C until analysis.

Test 2: RHEs stressed with ozone in contact with the active ingredient (RoxP-enriched *C. acnes* supernatant). RHEs were produced during 10 days under 5% COz, 37°C in incubator and the medium was changed every two days until day 8. At day 8, the active ingredient (RoxP-enriched *C. acnes* supernatant) was added in the medium at 10% v/v. At day 9, RHE were stressed with ozone (in the reaction chamber). The reaction medium was cooled at 5°C during the reaction with ozone (about 1 minute). After the stress, a new medium containing the active ingredient was added to RHE. At day 10, RHE and medium were recovered and stored at -20°C until analysis.

Each condition was performed in triplicate.

Active ingredient (C. *acnes* supernatant) was sterile filtered before use in cell culture assays.

### Morphology evaluation

Morphology was evaluated using Hemalun eosin coloration.

### Efficacy evaluation

DNA damage evaluation - CPDs (immunofluorescence analysis): To evaluate the inducible photo-protection afforded by the active, we evaluated DNA damage using CPDs immunostaining. UVB radiation (290-320 nm), the most energetic mutagenic and carcinogenic component of solar radiation, is directly absorbed by DNA, giving rise to dimeric photoproducts between adjacent pyrimidine bases. Two types of these bulky modifications are produced, namely cyclobutane pyrimidine dimers (CPDs) and pyrimidine (6-4) pyrimidone photoproducts (Cadet et al. 2005 Mutat Res 571:3-17). Both of these products involve dimerization of adjacent pyrimidines on the same DNA strand. Relevance of the CPD formation is further described in Sproul et al. 2014 Photochem Photobiol 90:145-154).

DNA damage evaluation - 8-OHdG (immunofluorescence analysis): In nuclear and mitochondrial DNA, 8-hydroxy-2-deoxyguanosine (8-OHdG) or 8-oxo-7,8-dihydro-2-deoxyguanosine (8-oxodG) is one of the predominant forms of free radical-induced oxidative lesions, and has therefore been widely used as a biomarker for oxidative stress and carcinogenesis. Relevance of this biomarker is for example described in Valavanidis et al. 2009 J Environ Sci Health C Environ Carcinog Ecotoxicol Rev 27: 120-39 and Nu et al. 1999 Br J Dermatol 140:226-31.

System detoxification evaluation (GPx analysis by colorimetric kit): Glutathione peroxidase (GPx) represents a selenium-containing antioxidant enzyme family with peroxidase activity whose main biological role is to protect the organism from oxidative damage. GPx effectively reduces HzOz and lipid peroxides to water and lipid alcohols, respectively, and in turn oxidizes glutathione to glutathione disulphide (Arthur JR. 2000 Cell Mol Life Sci 57:1825-1835). The importance of glutathione peroxidase for protection from oxidative stress is also illustrated by the fact that inactivation of glutathione peroxidase activity contributes to UV-induced squamous cell carcinoma formation (Walshe et al. 2007 Cancer Res 67:4751-8). Cutaneous squamous cell carcinomas (CSCC) are a common malignancy of keratinocytes that arise in sites of the skin exposed to excessive UV radiation.

Apoptosis evaluation (Caspase-3 by immunofluorescence analysis): Caspases (cysteinyl aspartate-specific proteases) are a family of important signaling molecules with various tasks depending on the subtype and organ involved. The caspase 3 has been implicated as an "effector" caspase associated with the initiation of the "death cascade" and is therefore an important marker of the cell's entry point into the apoptotic signaling pathway. Caspase-3 is activated by the upstream caspase-8 and caspase-9, and since it serves as a convergence point for different signalling pathways, it is well suited as a read-out in an apoptosis assay (Nicholson et al. 1995 Nature 376:37-43; Shi 2002 Molecular Cell 9:459-470).

### Example 7: Morphology evaluation of the effect of RoxP on UV stressed reconstructed human epidermis (RHEs)

Negative control 1 (Unstressed RHEs without *C. acnes* supernatant): The RHEs were fully differentiated with four layers (basal, spinous, granular and cornified layers). The basal layer is the proliferative compartment, we could observe aligned columnar cells with elliptical nucleus rich in chromatin; basal cells are attached to the polycarbonate membrane. The four strata of the spinous layer showed polygonal cells in the lower layers that flattened in the upper layers. The granular cell layer was made of 2-3 strata of flattened granular cells which contained visible keratohyalin granules. See **Fig. 15A**.

### Negative control 2 (Unstressed RHEs with C. acnes supernatant at 10% v/v)

The RHEs treated with the active ingredient at 10% v/v in the medium appeared thinner with an abnormal basal layer and with a defective differentiation process. Indeed, basal keratinocytes were not well aligned and did not shown a cubic shape. Furthermore, a decrease of the number of keratohyalin granules and a parakeratosis (retention of nuclei in the stratum corneum) were observed. Finally, compared to unstressed RHEs, the number of apoptotic cells (orange cells) in RHEs treated with the active ingredient was increased. This particular phenotype was indicative of some toxicity of the active ingredient at a concentration of 10% v/v in the medium. See **Fig. 15B****.**

Positive control 1 (RHEs stressed with UV): RHE stressed with UVA/UVB showed typical phenotype with sunburn cells. Indeed, the first evidence of UV-induced keratinocyte apoptosis was the appearance of sunburn cells in the epidermis. Sunburn cells characteristic are round shape, loss of connection with surrounding keratinocytes combined with a typical chromatin condensation in the nucleus. Apoptosis is a major protective response of skin to the induction of DNA damage. It leads to a preventive cell death to avoid accumulation of mutations in cells dividing with damage in their genome, and thus blocks tumorigenesis initiation. The most sunburn cells appeared in the basal and spinous layers but some were distinguished in granular layer. The whole basal layer cells seemed to be in apoptosis leading to an absence of proliferative cells and the death of the epidermis. Decrease of keratohyalin granules could also be noted. Evidence for the induction of apoptosis was also provided by the *in situ* detection of the caspase-3 using an antibody directed against the cleaved and active form of the enzyme (see below). See **Fig. 15C****.**

Test 1 (RHEs stressed with UV, with C. *acnes* supernatant at 10% v/v): RHEs treated with active ingredient and stressed with UVA/UVB showed a minor phenotype in the spinous layer but the nucleus in the basal layer were absent or abnormal. While the whole basal keratinocytes were in apoptosis, the number of sunburn cells in the spinous layers was decreased. Hence, under UVs stress, the number of classical sunburn cells observed in basal and spinous layers was decreased in presence of active ingredient. See **Fig. 15D****.**

### Example 8: DNA damage / CPDs evaluation of the effect of RoxP on UV stressed RHEs

Tissue was processed to detect the cyclobutane pyrimidine dimers (CPDs) level from paraffin sections using a red fluorescent detection. Nuclei were stained in blue with DAPI. Dashed lines correspond to the membrane insert in RHE model and to the dermal epidermal junction in human skin. Scale bar in **Fig. 16** = 50 µm.

No dimer formation was observed in unstressed RHEs with **(****Fig. 16B****)** or without **(****Fig. 16A****)** active ingredient. Under UV conditions, dimeric photoproducts (CPDs) were formed in the nucleated layers of the RHEs **(****Fig. 16C**, **D**). The active ingredient appeared to decrease DNA damage induced by UV **(****Fig. 16D**, **E**).

### Example 9: DNA damage / 8-OHdG evaluation of the effect of RoxP on UV stressed RHEs

Tissue was processed to detect the 8-hydroxy-2-deoxyguanosine (8-OHdG) level from paraffin sections using a red fluorescent detection. Nuclei were stained in blue with DAPI. Dashed lines correspond to the membrane insert in RHE model and to the dermal epidermal junction in human skin. Scale bar in **Fig. 17** = 50 µm.

8-OHdG was detected in unstressed RHEs without active ingredient **(****Fig. 17A**, **E**). The active ingredient slightly increased the production of 8-OHdG **(****Fig. 17B**, **E**) that confirmed the limited toxicity of the active ingredient at the chosen concentration already observed in morphology evaluation.

Under UV, the whole of nucleated layers showed DNA damages with a strong formation of 8-OHdG **(****Fig. 17C-E**). The active ingredient seemed to have a protective effect with a decrease of 8-OHdG detection **(****Fig. 17D-E**).

### Example 10: System detoxification evaluation (GPx analysis)

Glutathione peroxidase activity was determined using a colorimetric kit from BioAssay Systems (Hayward, CA) (EGPX-100). The results are set forth in **Table 3** and **Fig. 18****.**

**Table 3. Measurement of glutathione peroxidase activity.**

| **Conditions** | **GPX activity in the medium (U/L) Mean ± SEM** | **Evolution vs negative control 1 (%)** |
|---|---|---|
| **Negative control 1** | | |
| Unstressed RHEs without active ingredient | 50.3 ± 1.9 | / |
| **Negative control 2** | 44.3 ± 1.2 | -12 |
| Unstressed RHEs with active ingredient | | |
| **Positive control 1** | 0 | -100 |
| RHEs stressed with UVs | | |
| **Test 1** | 40.0± 1.5 | -20 |
| RHEs stressed with UVs in contact with the active ingredient | | |
| **Positive control 2** | 0.33 ± 0.3 | -99 |
| RHEs stressed with ozone | | |
| **Test 2** | 32.7 ± 0.7 | -35 |
| RHEs stressed with ozone in contact with the active ingredient | | |

For both UV and ozone stress, the active ingredient inhibited the effect of the stress on GPX activity.

### Example 11: Apoptosis evaluation (active caspase-3)

Tissue was processed to detect the active caspase-3 level from paraffin sections using a red fluorescent detection. Nuclei were stained in blue with DAPI. Dashed lines correspond to the membrane insert in RHE model and to the dermal epidermal junction in human skin. Scale bar in **Fig. 19** = 50 µm.

Only few cells showed an immunostaining of active caspase-3 in unstressed RHEs without active ingredient **(****Fig. 19A****, E bar #1).** In presence of active ingredient, caspase-3 was slightly activated indicating that the active ingredient induced apoptosis in RHEs **(****Fig. 19B****, E bar #2).** Caspase-3 was strongly activated under UV stress, from basal layer to spinous layer showing apoptotic cells **(****Fig. 19C****, E bar #3).** The active ingredient reduced caspase-3 activation in the basal layer **(****Fig. 19D****, E bar #4).**

### Example 12: RoxP peptides with antioxidant activity

Two peptides derived from RoxP were tested for antioxidant activity in an ABTS radical cation assay essentially as described in previous experiments, and in an oxygen radical absorbance capacity (ORAC) assay.

The peptides (denoted "RoxP-Peptide 1" and "RoxP-Peptide 2" herein) correspond to amino acid positions 66-83 and 128-140 of the native RoxP protein shown in SEQ ID NO: 1, and reproduced below with the amino acid positions corresponding to RoxP-Peptide 1 and RoxP-Peptide 2 indicated in **bold** and ***bold* + *italics*,** respectively:

MFVQIAASLAAASSIALGIPGAATPIDESQLPVGPQVSVTDSAQHTGPFAASSPLTITVKP
GAPCVRADGYQESMVTRVLDDKGHQVWTGTFDESKLIGGTGLGTATFHVGSPAAAF
NFHGSERTTYRTLSYCAYPHYVNGTRERLSQVSVKTFMVDPALN (SEQ ID NO: 1)

The sequences of the peptides are set forth below:
RoxP-Peptide 1: VRADGYQESMVTRVLDDK (SEQ ID NO: 20),
RoxP-Peptide 2: RTLSYCAYPHYVN (SEQ ID NO: 21).

**Fig. 20A and B** demonstrate that both peptides displayed antioxidant activity in the ABTS assay, which increased with the concentration of the peptides.

ORAC assays measure the oxidative degradation of a fluorescent molecule, such as fluorescein or beta-phycoerythrin, after being mixed with free radical generators, such as azo-initiator compounds, such as AAPH (2,2'-azobis(2-methylpropionamidine) dihydrochloride). Azo-initiators typically produce peroxyl radicals upon thermal decomposition, which cause oxidation and thereby quenching of the fluorescence of the fluorescent molecule. The addition of an antioxidant prevents the oxidative degeneration of the fluorescent molecule, and the degree of this protection can be quantified by a fluorimeter. Advantageously, peroxyl free radical is commonly found in the body, which makes ORAC assays particularly informative of antioxidant properties of test compounds *in vivo.*

In the present example, the ORAC assay (Zenbio, ordering number AOX-2) was performed as follows:
The dilution of the RoxP sample were prepared in the provided Assay buffer. Then all solutions and machines were preheated to 37°C and 150µl working Flourescein solution was added to a black 96 well plate with clear bottom. Then 25µl of the samples was added to the fluorescein containing wells and the plate was placed in an incubator at 37°C for 10 minutes in the dark. Then 25µl of freshly prepared AAPH solution (2,2'-azobis-2-methyl-propanimidamide, dihydrochloride) was added and the plate was put in a plate reader. The read out was done in fluorescent mode with excitation at 485 nm and emission between 528 and 538nm.**Fig. 21A and B** demonstrate that both peptides displayed antioxidant activity in the ORAC assay, which increased with the concentration of the peptides.

### Example 13: RoxP inhibits generation of hydroxyl radicals (•OH)

Deoxyribose assay is a well-established method to measure generation of hydroxyl radicals in biological systems and for evaluating the ability of antioxidants to reduce •OH radical generation. Such assays are described for example in Gutteridge & Halliwell (Biochem J. 1988, vol. 253(3), 932-933) and references therein. In particular, deoxyribose incubated with H₂0₂ and Fe2⁺ (or Fe3⁺ plus a reductant) is degraded into products that can react to form thiobarbituric acid-malondialdehyde (classic TBA-MDA assay) chromogen. This reaction is driven by •OH radicals. A reduction in prevalence of •OH radicals will result in fewer formed complexes and lower absorbance.

A reaction mixture containing 5 mM 2-deoxyribose, 100 µM FeCl₃, 100 µM EDTA, 1 mM HzOz, and 7.6 µg/ml ascorbic acid in buffer A (20 mM KH₂PO₄-KOH, pH 7,4), including or not 2 mg/ml RoxP, in triplicate, was incubated at 37°C for 1h (total volume 1 ml). After incubation, 1% w/v TBA (thiobarbituric acid) in 50 mM NaOH, and 2-8% v/v TCA (trichloroacetic acid) was added, followed by incubation at 95°C for 15 minutes, and reading of absorbance at 532 nm.

Addition of RoxP reduced absorbance at 532 nm in the TBA-MDA assay, indicating its ability to decrease generation of •OH radicals **(see** **Fig. 22****).**

### Example 14: RoxP decreases lipid hydroperoxide levels in irradiated plasma

Lipid peroxidation, the oxidative degradation of lipids, results from the action of free radicals on lipids. Peroxidation of lipids in cell membranes may result in cell damage.

Hydroperoxides react with Fe2⁺ ions to produce Fe3⁺ ions which are detected using thiocyanate ion as chromogen. Lower abundance of Fe3⁺ ions results in lowered absorbance, indicating lower level of lipid hydroperoxides in irradiated plasma.

Blood was collected in EDTA tubes. Samples containing 500 µl blood + 150 µl RoxP (300 µg RoxP), or 500 µl blood + 150 µl PBS, were subjected to irradiation:
UVA - irradiation 3 × 5000 µJ/cm²; tubes were rotated between irradiation steps; or
UVB - irradiation 1 × 9000 µJ/cm², 2 × 5000 µJ/cm².

Controls having composition identical to the samples, but not irradiated, were included in the experiment.

The blood samples were centrifuged at 1000 × g, plasma was collected and frozen at -80 °C. Lipid hydroperoxides were quantified using lipid hydroperoxide assay kit (Cayman Chemical, item no. Item No. 705002).

Addition of RoxP to plasma (both irradiated and non-irradiated) resulted in reduced lipid hydroperoxide levels **(see** **Fig. 23****).**

### Example 15: RoxP reduces Fe3⁺

Phenantroline chelates specifically Fe2⁺ and generates absorbance at 550 nm. Hence, the more Fe2⁺ is present in a sample, the higher the absorbance at 550 nm.

A reaction mixture containing 0.7 mM ammonium iron (II) sulfate hexahydrate (from 1.4 mM working solution in PBS) or 0.5 mM ammonium iron (III) sulfate dodecahydrate (from 1.0 mM working solution in PBS); and further containing 10 µg or 20 µg RoxP (from 2 mg/ml RoxP working solution in PBS) or equivalent volume of PBS as control; and further containing 0.75 mM phenatroline (from 5 mM working solution in PBS); total volume 200 µl; was incubated for 15 minutes at room temperature. Absorbance was read at 550 nm.

Addition of RoxP reduced Fe3⁺ to Fe2⁺ and generated high absorbance, while it did not affect the reaction when only Fe2⁺ was present (see **Fig. 24****).**

These results were corroborated using the ferrozine assay, which confirmed reduction of Fe³⁺ to Fe2⁺ by RoxP (see **Fig. 25****).** The reagents used in the ferrozine assay included 100 µl of 50 mM iron ammonium dodecahydrate, 100 µl 2 mM ferrozine in H₂O, and RoxP (50 µl) resulting in various RoxP concentrations indicated in the graph, diluted from a stock solution of 2 mg/ml RoxP in PBS. Incubation was Incubation 17 hours.

### Example 16: RoxP interaction with coproporphyrin III

1 mg/ml RoxP (100 µl) was mixed with 50 µg Coproporphyrin (10 µl) and total volume was adjusted to 290 µl with PBS. Absorbance was measured at different wavelengths. The absorbance curves indicate binding of coproporphyrin III by RoxP (see **Fig. 26****).**

### Example 17: Effects of FMN/FDN on RoxP in reaction with ABTS

The effect of flavin mononucleotide (FMN)/ flavin adenine dinucleotide (FDN)
on RoxP activity in ABTS assay was measured. The reaction components included ABTS (oxidized according to the protocol), 20 x in PBS, pre-heated at 37° C, 89 ul added; RoxP, 2 mg/ml, 10 µl; FMN/FDN 1 mM, 1 µl or 3 µl ("1FMN", "1FDN" or "3FMN", "3FDN" in the graph, respectively); total volume was adjusted to 100 µl with PBS. Results showed an increased reduction of ABTS radical by RoxP when FMN/FDN was added, especially FDN. (see **Fig. 27****).**

### Example 18: RoxP inhibits the generation of radicals formed through porphyrins

The sebum of normal humans contains high amounts of porphyrins which are implicated in the generation of reactive oxygen species (ROS) on the skin. The amount of porphyrins can be easily visualised by UV light or flourescence photography.

The Singlet Oxygen Sensor Green dye was dissolved in Methanol and added in PBS buffer to reach a final concentration of 0,4µM. This solution was distributed in a clear 96 well plate. To the wells 1µM Coproporphyrin III, or 1µM Protoporphyrin IX was added. Then varying amounts of RoxP were added. Each condition was set up as 4 replicas. Two of the replicas were covered by aluminium foil during the exposure, while two wells were exposed for 10 min to a UV lamp. After exposure, the flouresence of the Singlet Oxygen Sensor Green dye was read out indicating the amount of singlet oxygen generated.

RoxP efficiently inhibited the generation of radicals formed through the porphyrins (see **Fig. 32****)** and can thus be a preferential way to reduce damage caused by UV induced singlet oxygen generation.

### Example 19: RoxP showed antioxidative potential when administered to human subjects in an UVA/UVB-irradiation study

The objective of this study was to assess the UV-protection potential of three compositions containing RoxP in 7 healthy subjects by measuring the antioxidative effect and skin erythema. Oxidative stress was induced by exposition to UVA- and UVB-light to the skin on the upper back. The anti-oxidative activity of the test products was evaluated by measurement of sebum lipid oxidization levels (concentration of squalene monohydroperoxide) of swab-samples from the skin surface. The potential was assessed after two applications of RoxP in comparison to water, an untreated control and one hydrophilic standard antioxidant (H1) after induction of a light sunburn (1.25 MED). Specific sampling kits (Synelvia) were used to obtain lipid samples for analysis. Additionally, the UV-protection potential of the products was evaluated by measuring the produced erythema (Chromameter).

The tested products were as follows: untreated ("UT"); demineralized water ("Aqua"); Antioxidant H1, a natural hydrophilic catechol 1 % in water (positive control) ("H1"); 0.3 mM ("EC-AA-001"), 1.2 mM ("YE-LU-001") or 4.0 mM ("CA-FT-001") full-length RoxP protein in an aqueous solution. The samples were provided as solutions and were stored at room temperature. For all reference the Aqua control was used as the samples were applied as an aqueous solution.

The study was designed as randomized, double blind for analysis of sebum lipids, with intra-individual comparisons, and placebo controlled.

Erythema was evaluated (Chromameter) at t1 = baseline before irradiation, t2 = 30 to 50 minutes after irradiation, and t3 = 16 to 24 hours after irradiation. Squalene monohydroperoxide (SQOOH) was quantified at 25 to 45 minutes after irradiation.

The general test area was upper back. Treatment areas had a size of 4.5 cm × 4.5 cm. Irradiation areas had a size of 4 cm × 4 cm. The round swabbing area had a diameter of approx. 2.6 cm. Numbers of test areas were assigned starting on the left upper side of the back towards the right upper side of the back building a row (area 1 to 4). A second row started under the first row on the left side (area 5 and 6). Two areas were always irradiated together (e.g. Area 1 and 3, 2 and 4, 5 and 6). See Fig. 28. Treatments were balanced assigned to the test areas according to a 6 × 6 orthogonal Latin Squares with randomly permuted blocks of fixed size.

45 µl (approx. 2 mg/cm²) of the respective test solution were applied with a pipette. After applying the test product to the test area, they were quickly spread by gently rubbing (soft touch with light pressure) with a non-saturated finger-cot, first with rotating followed by crosswise movements for a final uniform coating on the skin.

Suitable inclusion and exclusion criteria were applied. Subjects included both males and females, at least 18 years of age, with healthy skin in the test area, uniform skin color and no erythema or dark pigmentation in the test area, and ITA>28 in the test area. Subjects with, e.g., active skin disease at the test area, regular recent use of tanning beds, moles, tattoos, scars, irritated skin, hairs, etc. at the test area that could influence the investigation, recent use of medication with known photo-toxic and/or photo-sensitizing potential, recent use of any topical medication at the test area, and/or intake of dietary supplements with anti-oxidative effects, were excluded from the study. Subjects were instructed to avoid factors that could influence the investigation throughout the duration of the study.

On **Day 1,** before instrumental measurements the subjects laid down on their belly for at least 15 minutes in the waiting area at ambient temperature to allow the skin to dry in case of sweating. The skin type (ITA°) of the subjects was classified by use of colorimetric measurements of the skin. After that 6 spots of approx. 1 cm × 1 cm each were irradiated on the back (separate to the treatment areas) to detect the minimal erythemal dose (MED). The UV-B dose was increased from spot to spot by an increment of 25 %.

Then, the study products were applied according to the randomization scheme by a technician. To avoid absorption of the test products by clothes, the subjects stayed undressed at room temperature for approximately 15 minutes.

On **Day 2,** subjects returned to the Study Site 20 ± 4 hours after irradiation. Before instrumental measurements the subjects laid down on their belly for at least 15 minutes in the waiting area at ambient temperature to allow the skin to dry in case of sweating. The reading of the MED was performed and the irradiation time for 1 MED was determined. The correct dose for the irradiation of test areas was calculated by multiplying the irradiation dose of 1 MED with the factor for irradiation (1.25).

Chromameter measurements were performed on all test areas (Baseline) to assess erythema. The study products were applied according to the randomization scheme. To avoid absorption of the test products by clothes, the subjects stayed undressed at room temperature for approximately 15 minutes. 45 mins ± 5 mins after application, every treatment area was irradiated with 1.25 MED. 25 to 45 mins after irradiation, swabbing was performed on all treatment areas according to the Swab method and analyzed as described by Synelvia (Appendix 2). 5 to 10 minutes after swabbing, Chromameter measurements were repeated.

On **Day 3** subjects returned to the Study Site 20 ± 4 hours after irradiation. Before instrumental measurements the subjects laid down on their belly for at least 15 minutes in the waiting area at ambient temperature to allow the skin to dry in case of sweating. Chromameter measurements were repeated on all test areas.

The following instrumental measurement(s) were performed:
- CHROMAMETER CR 400 (Minolta, Device D-Langenhagen, Germany): Skin color can objectively be quantified by reflectance measurements with a tri-stimulus Chromameter. The measuring principle is based on the reflection of a Xenon flash that diffusely lightens the skin on an area of 8 mm in diameter. The vertically reflected light from the skin is then detected by high-sensitive silica photodiodes that provide a color analysis of the detected light. The Chromameter defines the measured color in the L*a*b* color coordinate system. The L*-value defines the brightness. The color is defined by the parameters a* (red-green axis; negative a*-value green, positive a*-value red) and b* (blue-yellow axis, negative b*-value blue, positive b*-value yellow). The a* value correlates well with visual assessments of skin redness (erythema). a* is a measure for erythema. An increase in the a*-value corresponds to an increase in the degree of skin redness. Parameter: a* Skin redness. 3 measurements per test area and assessment time for assessments or erythema.

The following investigational method(s) were performed:
- UV-Irradiation (Solar Simulator UVASPOT 1000, Dr. Hönle AG, Gräfelfing, Germany).
- Irradiations were performed with the UVA Spot 1000. It produces a large, homogenous field of irradiation (approximately 20 cm × 20 cm in 50 cm distance from radiation source). The spectrum is defined by specific filters. The "H2" filter was used (UVB and UVA radiation). The sun simulator was used to irradiate the test areas with the requested irradiation intensity on the test area. Multiple spots may have been irradiated at the same time. The sun stimulator irradiates an area of approx. 20 cm × 20 cm. 1 irradiation per test area.
- Swab Sampling, Storage and Shipment. Swab sampling was performed on each test area by a trained technician. Cotton Swabs were wetted with the sampling buffer and a first swab is taken by applying pressure for 45 seconds during regular movements of the swab over the whole test area. The swab was cut with scissors, so it fits into the provided Eppendorf tube containing a buffer. The procedure was repeated on the same test area with a second buffer wetted swab. The second swab was stored in the same Eppendorf tube as the first swab. Eppendorf tubes containing two swabs per test area were stored directly on ice after sampling and were put into a -20 °C freezer within 2 hours after sampling and stored at -20 °C until shipment. The shipment of samples was performed on dry ice with an appropriate courier service after the end of the study (on the next working day). 1 swab per test area.
- Swab Sampling Analysis. The analysis of the swab samples for the contents of the sebum lipid peroxidation product squalene monohydroperoxyde (SQOOH) was done by liquid chromatography-mass spectrometry (LC/MS).

The Investigator decided which protocol deviations occurring in the study were assumed to be minor and major before data were analyzed. Valid subjects were defined as enrolled subjects who had finished the study without major deviations from the protocol and who had not withdrawn their consent. No replacement of missing data was performed and the affected assessments were regarded as lost for analysis. Demographic variables (age, gender) were given for the analysis population. No unblinding was necessary for analysis of data, because statistical analysis was done using the treatment codes. Derandomization would have been done by merging data to the randomization list using an unique identifier variable. Raw data (squalene content (ng/mg) and Chromameter a*-values) were listed by assessment time and treatment. For Chromameter values, differences to Baseline (before irradiation) were calculated and listed by assessment time and treatment. N, mean, standard deviation, median, minimum and maximum were given for raw data and differences to Baseline. The mean values of raw data and differences to Baseline over subjects are presented in bar charts by treatment and assessment time with standard deviation.

7 subjects enrolled in the study and finished the study without major deviations. Age: 55 ± 9.8 years (mean ± standard deviation); gender: 1 male (14 %), 6 female (86 %). No adverse reactions were observed in the study.

Results of Chromameter Measurements: Skin color a* (erythema) was measured with a Chromameter before (Day 2, Baseline) and 30 to 50 minutes after (Day 2) and 16 to 24 hours after irradiation (Day 3). An increase of a*-values corresponds to an increase of skin redness. **Fig. 29** shows skin color a* (erythema) by Chromameter - bar chart with means and 95 % confidence intervals of raw data (n = 7). **Fig. 30** shows skin color a* (erythema) by Chromameter - bar chart with means and 95 % confidence intervals of differences to baseline (n = 7).

Results of Analysis of the Sebum Lipid Peroxidation Product Squalene Monohydroperoxyde: Samples for the analysis of the sebum lipid peroxidation product Squalene monohydroperoxyde (SQOOH) were taken on the upper back. **Fig. 31** shows SQOOH content (ng/mg) - bar chart with means and 95 % confidence intervals of raw data (n = 5, data from subjects #4 and #5 were excluded due to implausible results likely due to mixing up of samples).

In conclusion, the assessment of **SQOOH** showed high amounts of SQOOH on the untreated, irradiated area and even higher amounts on the irradiated area treated with water. The higher amount of SQOOH on the irradiated area treated with water might be explained by the effect of water to make the skin more transparent and therefore more light might be absorbed by the skin leading to higher oxidation levels. The irradiated areas treated with the positive control "H1" showed very low values for SQOOH, as expected. The three test products resulted in decreasing values in SQOOH: the test product EC-AA-001 showed the lowest anti-oxidative efficacy, followed by YE-LU-001 and the test product CA-FT-001 showed the highest tendency for an anti-oxidative efficacy. This difference in product efficacy matched to the concentrations of antioxidants. The difference between the CA-FT-001 test product and the aqua control was statistically significant with a p-value <0.05.

The irradiation with 1.25 MED resulted in a mean increase of **skin color a*** over all subjects during 24 hours, thus resulted in a slight erythema. No statistically significant differences between the tested products and the controls were observed; however a clear trend for the reduction of erythema was visible in all tested antioxidants and the effect size was equal among all antioxidants. While the water control increased by a median of 6 units the RoxP treated fields only increased by a median of 3.4 units which matches with the increase of the H1 control of median of 3.5 units. Due to the high standard deviation a statistical significance could not be observed at this sample size. Especially in the measurement 30-50min after UV irradiation the RoxP treated fields showed less erythrema than the water control and equal or less erythrema than the positive control.

The present study thus showed, in cosmetically and therapeutically relevant settings, i.e., on the skin of actual human subjects, that RoxP showed a meaningful and even statistically significant antioxidative effect after UV irradiation of the skin, and that the antioxidative effect increased with increasing concentration of administered RoxP.

### Example 20: Compositions

The present example sets forth illustrative compositions (e.g., lotions, gels, or creams) embodying the principles of the present invention, which can be suitably employed in cosmetic or pharmaceutical settings.

| **Body milk composition** | % by weight |
|---|---|
| Magnesium chloride | 3 |
| Calcium ascorbate | 3 |
| Glyceryl stearate | 1 |
| Sinnowax AO^{®} (BASF, Ludwigshafen am Rhein, Germany) (cetearyl alcohol (and) Ceteareth-30) or similar fatty alcohols | 3 |
| Cetyl alcohol | 1 |
| Dimethicone (DC 200 Fluid ^{®}, sold by Dow Coming, Midland, MI) | 1 |
| Liquid petrolatum | 6 |
| Isopropyl myristate (Estol IMP 1514 ^{®},sold by Uniqema, Goole, United Kingdom) | 3 |
| Active (e.g., RoxP preparation / C. *acnes* RoxP-enriched supernatant) | 0,005-0,3 |
| Vitamin C and E (stabilised forms) | 0,05 |
| Glycerol | 20 |
| Preservative | 0,3 |
| Water | fill up to 100% |

| **Lotion for the body / scalp composition** | % by weight |
|---|---|
| Active (e.g., RoxP preparation / *C. acnes* RoxP-enriched supernatant) | 0,005-0,3 |
| Antioxidant | 0,05 |
| Isopropanol | 40 |
| Preservative | 0,3 |
| Water | fill up to 100% |

| **Milk for the care of the body / scalp composition** | % by weight |
|---|---|
| Active (e.g., RoxP preparation / *C. acnes* RoxP-enriched supernatant) | 0.001-1 |
| Glyceryl stearate | 1 |
| Sinnowax AO^{®} (BASF) or similar fatty alcohols | 3 |
| Cetyl alcohol | 1 |
| Dimethicone (DC 200 Fluid ^{®} sold by Dow Coming) | 1 |
| Liquid petrolatum | 6 |
| Isopropyl myristate | 3 |
| Antioxidants | 0,05 |
| Preservative | 0,3 |
| Water | fill up to 100% |

| **Gel for the care of the body / scalp composition** | % by weight or Molar |
|---|---|
| Active (e.g., RoxP preparation / *C. acnes* RoxP-enriched supernatant) | 2µM-2mM RoxP |
| Hydroxypropylcellulose | 1 |
| Vitamin E or derivative | 2,5 |
| Antioxidant | 0,05 |
| Isopropanol | 40 |
| Preservative | 0,3 |
| Water | fill up to 100% |

| **Gel for the care of the hair composition** | % by weight or Molar |
|---|---|
| Active (e.g., RoxP preparation / *C. acnes* RoxP-enriched supernatant) | 2µM-2mM RoxP |
| Copper citrate | 2 |
| Antioxidant | 0,05 |
| Vitamin C | 2,5 |
| Isopropanol | 40 |
| Preservative | 0,3 |
| Water | fill up to 100% |

| **Lotion for the face composition** | % by weight or Molar |
|---|---|
| Active (e.g., RoxP preparation / *C. acnes* RoxP-enriched supernatant) | 2µM-2mM |
| Anti-inflammatory | 0,05 |
| Antioxidant | 0,05 |
| Isopropanol | 40 |
| Preservative | 0,3 |
| Water | fill up to 100% |

| **Topical composition** | Amount |
|---|---|
| Active (e.g., RoxP preparation / C. *acnes* RoxP-enriched supernatant) | 5mg |
| Poloxamer 188 | 10g |
| methyl paraoxybenzoate | 200mg |
| disodium hydrogen phosphate | 340.23mg |
| sodium chloride | 832.77mg |
| phosphoric acid | To adjust pH |
| purified water | fill up to 100 ml |
| Total | 100ml |

| **Formulations for reconstitution of *C. acnes* lyophilised bacteria** | | | |
|---|---|---|---|
| Ingredients (w/v) | Formulation 1 | Formulation 2 | Formulation 3 |
| Carageenan | 0,2% | - | - |
| Citrate | 0,17% | 0,17% | 0,17% |
| Glycerol | - | 5% | 1% |
| Hyaluronate | - | 1% | - |
| Polyacrylic acid | - | - | 0,2% |
| Citric acid | 0,09% | 0,09% | 0,09% |
| Water | Fill up to 100% | Fill up to 100% | Fill up to 100% |

## Claims

1. A cosmetic use of a composition comprising a live *Cutibacterium acnes* strain secreting RoxP (radical oxygenase of *Propionibacterium acnes*) for preventing or reducing skin ageing in a subject.

2. A composition comprising a live *C. acnes* strain secreting RoxP for use in a method of preventing or treating an oxidative stress-associated skin disease, wherein the oxidative stress-associated skin disease is selected from the group comprising actinic keratosis (AK), and basal cell carcinoma (BCC).

3. The use according to claim 1 or the composition for use according to claim 2, wherein:
- the *C. acnes* strain endogenously secretes RoxP;
- the *C. acnes* strain is not genetically engineered;
- the composition comprises two or more *C. acnes* strains secreting RoxP;
- the *C. acnes* strain secretes, or the two or more *C. acnes* strains individually or collectively secrete, at least 1 µg RoxP per mL of medium when measured in stationary phase;
- the endogenous RoxP promoter of the *C. acnes* strain, or of at least one of the two or more *C. acnes* strains, comprises -10 box having a sequence selected from TGCTATACT or TGCTACACT, preferably TGCTATACT, and/or -35 box having a sequence selected from ACTTCGAT or ACTTCAAT;
- the *C. acnes* strain is, or at least one of the two or more *C. acnes* strains each independently is, type I, II or III, preferably type IA, IB or III; and/or
- the *C. acnes* strain is, or at least one of the two or more *C. acnes* strains each independently is, SLST type A, C, G, H, L or K.

4. The use according to any one of claims 1 or 3 or the composition for use according to any one of claims 2 to 3, wherein
- the *C. acnes* strain is, or the two or more *C. acnes* strains individually or collectively are, present in the composition at an amount of at least 100 colony forming units (CFU) per gram of the composition; and/or
- the composition is configured for topical administration to the skin, such as wherein the composition is a gel, cream, ointment, lotion, drops, spray including aerosol sprays, foam, or powder, optionally wherein the composition is comprised by a mask, pad, patch, a two-chamber device, or make-up.

5. A cosmetic use of a composition comprising RoxP for preventing or reducing skin ageing in a subject.

6. A composition comprising RoxP for use in a method of preventing or treating an oxidative stress-associated skin disease, wherein the oxidative stress-associated skin disease is selected from the group comprising actinic keratosis (AK) and basal cell carcinoma (BCC).

7. The use according to claim 5 or the composition for use according to claim 6, wherein:
- the composition comprises supernatant or a RoxP-enriched fraction of supernatant of a cultured live C. *acnes* strain secreting RoxP, preferably endogenously secreting RoxP;
- the composition comprises at least 1×10⁻⁹ M RoxP; and/or
- the composition is configured for topical administration to the skin, such as wherein the composition is a gel, cream, ointment, lotion, drops, spray including aerosol sprays, foam, or powder, optionally wherein the composition is comprised by a mask, pad, patch, a two-chamber device, or make-up.

## Patentansprüche

1. Kosmetische Verwendung einer Zusammensetzung, die einen *Cutibacterium* acnes-Lebendstamm umfasst, der RoxP (Radikal-Oxygenase von *Propionibacterium acnes*) sezerniert, zur Vorbeugung oder Verringerung der Hautalterung bei einem Individuum.

2. Zusammensetzung, umfassend einen *C. acnes-*Lebendstamm, der RoxP sezerniert, zur Verwendung bei einem Verfahren zur Vorbeugung oder Behandlung einer Hautkrankheit in Zusammenhang mit oxidativem Stress, wobei die Hautkrankheit in Zusammenhang mit oxidativem Stress aus der aktinische Keratose (AK) und Basalzellkarzinom (BCC) umfassenden Gruppe ausgewählt ist.

3. Verwendung nach Anspruch 1 oder Zusammensetzung zur Verwendung nach Anspruch 2, wobei:
- der C. acnes-Stamm endogen RoxP sezerniert;
- der C. acnes-Stamm nicht gentechnisch konstruiert ist;
- die Zusammensetzung zwei oder mehr RoxP sezernierende *C.* acnes-Stämme umfasst;
- der *C.* acnes-Stamm bzw. die zwei oder mehr C. *acnes-*Stämme einzeln oder gemeinsam wenigstens 1 ug RoxP pro ml Medium bei Messung in der stationären Phase sezerniert bzw. sezernieren;
- der endogene RoxP-Promotor des *C.* acnes-Stamms oder wenigstens eines der zwei oder mehr *C.* acnes-Stämme eine
- 10-Box mit einer Sequenz, die aus TGCTATACT oder TGCTACACT ausgewählt ist, bevorzugt TGCTATACT, und/oder
- 35-Box mit einer Sequenz, die aus ACTTCGAT oder ACTTCAAT ausgewählt ist, umfasst;
- der *C.* acnes-Stamm bzw. wenigstens einer der zwei oder mehr *C.* acnes-Stämme jeweils unabhängig Typ I, II oder III, bevorzugt Typ IA, IB oder III ist; und/oder
- der *C.* acnes-Stamm bzw. wenigstens einer der zwei oder mehr *C.* acnes-Stämme jeweils unabhängig SLST-Typ A, C, G, H, L oder K ist.

4. Verwendung nach einem der Ansprüche 1 oder 3 oder Zusammensetzung zur Verwendung nach einem der Ansprüche 2 bis 3, wobei
- der *C.* acnes-Stamm bzw. die zwei oder mehr *C. acnes-*Stämme einzeln oder gemeinsam in der Zusammensetzung in einer Menge von wenigstens 100 koloniebildenden Einheiten (CFU) pro Gramm der Zusammensetzung vorliegt bzw. vorliegen; und/oder
- die Zusammensetzung für die topische Verabreichung an die Haut ausgelegt ist, wie wobei es sich bei der Zusammensetzung um ein Gel, eine Creme, eine Salbe, eine Lotion, Tropfen, ein Spray einschließlich Aerosolsprays, Schaum oder Pulver handelt, gegebenenfalls wobei die Zusammensetzung in einer Maske, einem Kissen, einem Pflaster, einer Zwei-Kammer-Vorrichtung oder Make-up enthalten ist.

5. Kosmetische Verwendung einer RoxP umfassenden Zusammensetzung zur Vorbeugung oder Verringerung der Hautalterung bei einem Individuum.

6. Zusammensetzung, umfassend RoxP, zur Verwendung bei einem Verfahren zur Vorbeugung oder Behandlung einer Hautkrankheit in Zusammenhang mit oxidativem Stress, wobei die Hautkrankheit in Zusammenhang mit oxidativem Stress aus der aktinische Keratose (AK) und Basalzellkarzinom (BCC) umfassenden Gruppe ausgewählt ist.

7. Verwendung nach Anspruch 5 oder Zusammensetzung zur Verwendung nach Anspruch 6, wobei:
- die Zusammensetzung Überstand oder eine mit RoxP angereicherte Fraktion von Überstand eines kultivierten C. acnes-Lebendstamms, der RoxP sezerniert, bevorzugt endogen sezerniert, umfasst;
- die Zusammensetzung wenigstens 1×10⁻⁹ M RoxP umfasst; und/oder
- die Zusammensetzung für die topische Verabreichung an die Haut ausgelegt ist, wie wobei es sich bei der Zusammensetzung um ein Gel, eine Creme, eine Salbe, eine Lotion, Tropfen, ein Spray einschließlich Aerosolsprays, Schaum oder Pulver handelt, gegebenenfalls wobei die Zusammensetzung in einer Maske, einem Kissen, einem Pflaster, einer Zwei-Kammer-Vorrichtung oder Make-up enthalten ist.

## Revendications

1. Utilisation cosmétique d'une composition comprenant une souche de *Cutibacterium acnes* vivante sécrétant RoxP (radical oxygénase de *Propionibacterium acnes*) pour la prévention ou la réduction du vieillissement de la peau chez un sujet.

2. Composition comprenant une souche de *C. acnes* vivante sécrétant RoxP pour une utilisation dans un procédé de prévention ou de traitement d'une maladie de la peau associée au stress oxydant, la maladie de la peau associée au stress oxydant étant choisie dans le groupe comprenant une kératose actinique (AK), et un carcinome de cellules basales (BCC).

3. Utilisation selon la revendication 1 ou composition pour une utilisation selon la revendication 2 :
- la souche de *C. acnes* sécrétant RoxP de manière endogène ;
- la souche de *C. acnes* n'étant pas génétiquement modifiée ;
- la composition comprenant deux souches de C. *acnes* ou plus sécrétant RoxP ;
- la souche de *C. acnes* sécrétant, ou les deux souches de *C. acnes* ou plus sécrétant individuellement ou collectivement, au moins 1 µg de RoxP par mL de milieu lorsqu'elle est mesurée dans une phase stationnaire ;
- le promoteur de RoxP endogène de la souche de *C. acnés,* ou d'au moins une des deux souches de *C. acnes* ou plus, comprenant -10 box ayant une séquence choisie parmi TGCTATACT ou TGCTACACT, préférablement TGCTATACT, et/ou
- 35 box ayant une séquence choisie parmi ACTTCGAT ou ACTTCAAT ;
- la souche de *C. acnes* étant, ou au moins une des deux souches de *C. acnes* ou plus chacune étant indépendamment, du type I, II ou III, préférablement du type IA, IB ou III ; et/ou
- la souche de *C. acnes* étant, ou au moins une des deux souches de *C. acnes* ou plus chacune étant indépendamment, SLST du type A, C, G, H, L ou K.

4. Utilisation selon l'une quelconque des revendications 1 ou 3 ou composition pour une utilisation selon l'une quelconque des revendications 2 à 3,
- la souche de *C. acnes* étant, ou les deux souches de *C. acnes* ou plus étant indépendamment ou collectivement, présente(s) dans la composition à une quantité d'au moins 100 unités formant colonie (UFC) par gramme de la composition ; et/ou
- la composition étant configurée pour une administration topique sur la peau, de sorte que la composition étant un gel, une crème, un onguent, une lotion, des gouttes, un spray y compris des sprays d'aérosol, une mousse, ou une poudre, éventuellement la composition étant comprise par un masque, un tampon, un patch, un dispositif à deux compartiments ou un maquillage.

5. Utilisation cosmétique d'une composition comprenant RoxP pour la prévention ou la réduction du vieillissement de la peau chez un sujet.

6. Composition comprenant RoxP pour une utilisation dans un procédé de prévention ou de traitement d'une maladie de la peau associée au stress oxydant, la maladie de la peau associée au stress oxydant étant choisie dans le groupe comprenant une kératose actinique (AK), et un carcinome de cellules basales (BCC).

7. Utilisation selon la revendication 5 ou composition pour une utilisation selon la revendication 6 :
- la composition comprenant un surnageant ou une fraction enrichie en RoxP de surnageant d'une souche de *C. acnes* vivante cultivée sécrétant RoxP, préférablement sécrétant RoxP de manière endogène ;
- la composition comprenant au moins 1×10⁻⁹ M de RoxP ; et/ou
- la composition étant configurée pour une administration topique sur la peau, de sorte que la composition étant un gel, une crème, un onguent, une lotion, des gouttes, un spray y compris des sprays d'aérosol, une mousse, ou une poudre, éventuellement la composition étant comprise par un masque, un tampon, un patch, un dispositif à deux compartiments ou un maquillage.
